# EUROPEAN PATENT APPLICATION

(11) **EP 1 332 760 A1**
(43) Date of publication of application: **06.08.2003**
(21) Application number: 02075456.0
(22) Date of filing: 04.02.2002
(51) Int. Cl.: A61K 38/02, A61K 39/35, C07K 7/08, C07K 14/415, C07K 16/16, C12N 5/08, C12Q 1/37, C12Q 1/68, G01N 33/02

(54) **Novel epitopes for celiac disease and autoimmune diseases, methods for detecting those and novel non-antigenic food compounds**

(71) Applicant: Academisch Ziekenhuis Leiden, 2333 ZA Leiden (NL)
(72) Inventor: The designation of the inventor has not yet been filed
(74) Representative: Prins, Adrianus Willem

(57) **Abstract**

The invention describes the patterns of deamidation in gluten, and it is found that this is highly dependent on the spacing between the glutamine and proline residues. This knowledge can be used to predict novel T cell stimulatory gluten peptides. Several newly defined peptides and epitopes are provided. Also, the finding can explain the formation of neo-epitopes in autoimmune diseases such as RA (rheumatoid arthritis), MS (multiple sclerosis), SLE (systemic lupus erythomatosus), SS (Sjogren syndrome) and DB (diabetes). Several neo-epitopes and the peptides that are substrate for deamidation are provided.

Further, the inventions provides for methods for detecting these peptides and epitopes and methods for making food more suitable for celiac disease patients.

SEQUENCE LISTING

<110> Academisch Ziekenhuis Leiden

<120> Novel epitopes for celiac disease and autoimmune
      diseases, methods for detecting those and novel
      non-antigenic food compounds

<130> P58975EP00

<140> 02075456.0
<141> 2002-02-04

<160> 337

<170> PatentIn Ver. 2.1

<210> 1
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

<400> 1
Gln Gln Pro Tyr Pro Gln Gln Pro Gln Gln Pro Phe Pro Gln
  1               5                  10


<210> 2
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

<400> 2
Gln Gln Pro Phe Pro Gln Gln Pro Gln Gln Pro Phe Pro Gln
  1               5                  10


<210> 3
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

P58975ep

<400> 3
Pro Phe Pro Gln Thr Gln Gln Pro Gln Gln Pro Phe Pro Gln
1               5                   10


<210> 4
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

<400> 4
Pro Phe Pro Gln Leu Gln Gln Pro Gln Gln Pro Phe Pro Gln
1               5                   10


<210> 5
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

<400> 5
Gln Phe Pro Gln Thr Gln Gln Pro Gln Gln Pro Phe Pro Gln
1               5                   10


<210> 6
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

<400> 6
Gln Leu Pro Phe Pro Gln Gln Pro Gln Gln Pro Phe Pro Gln
1               5                   10


<210> 7
<211> 14
<212> PRT
<213> Artificial Sequence

P58975ep
<220>
<223> Description of Artificial Sequence: gluten-derived
      peptide which is able to act as substrate for
      tissue transglutaminase

<220>
<221> SITE
<222> (1)..(14)

<400> 7
Pro Phe Pro Gln Pro Gln Gln Pro Gln Gln Pro Phe Pro Gln
1               5                   10


<210> 8
<211> 14
<212> PRT
<213> Artificial Sequence

<220>
<223> Description of Artificial Sequence: gluten-derived
      peptide which is able to act as substrate for
      tissue transglutaminase

<220>
<221> SITE
<222> (1)..(14)

<400> 8
Pro Phe Pro Gln Ser Gln Gln Pro Gln Gln Pro Phe Pro Gln
1               5                   10


<210> 9
<211> 14
<212> PRT
<213> Artificial Sequence

<220>
<223> Description of Artificial Sequence: gluten-derived
      epitope

<220>
<221> SITE
<222> (1)..(14)

<400> 9
Gln Gln Pro Tyr Pro Glu Gln Pro Gln Gln Pro Phe Pro Gln
1               5                   10


<210> 10
<211> 14
<212> PRT
<213> Artificial Sequence

<220>
<223> Description of Artificial Sequence: gluten-derived
      epitope

<220>
<221> SITE
<222> (1)..(14)

<400> 10
Gln Gln Pro Phe Pro Glu Gln Pro Gln Gln Pro Phe Pro Gln
1               5                   10

P58975ep

<210> 11
<211> 14
<212> PRT
<213> Artificial Sequence

<220>
<223> Description of Artificial Sequence: gluten-derived
      epitope

<220>
<221> SITE
<222> (1)..(14)

<400> 11
Pro Phe Pro Gln Thr Glu Gln Pro Gln Gln Pro Phe Pro Gln
  1               5                   10


<210> 12
<211> 14
<212> PRT
<213> Artificial Sequence

<220>
<223> Description of Artificial Sequence: gluten-derived
      epitope

<220>
<221> SITE
<222> (1)..(14)

<400> 12
Pro Phe Pro Gln Leu Glu Gln Pro Gln Gln Pro Phe Pro Gln
  1               5                   10


<210> 13
<211> 14
<212> PRT
<213> Artificial Sequence

<220>
<223> Description of Artificial Sequence: gluten-derived
      epitope

<220>
<221> SITE
<222> (1)..(14)

<400> 13
Gln Phe Pro Gln Thr Glu Gln Pro Gln Gln Pro Phe Pro Gln
  1               5                   10


<210> 14
<211> 14
<212> PRT
<213> Artificial Sequence

<220>
<223> Description of Artificial Sequence: gluten-derived
      epitope

<220>
<221> SITE
<222> (1)..(14)

P58975ep
<400> 14
Gln Leu Pro Phe Pro Glu Gln Pro Gln Gln Pro Phe Pro Gln
1               5                   10


<210> 15
<211> 14
<212> PRT
<213> Artificial Sequence

<220>
<223> Description of Artificial Sequence: gluten-derived
      epitope

<220>
<221> SITE
<222> (1)..(14)

<400> 15
Pro Phe Pro Gln Pro Glu Gln Pro Gln Gln Pro Phe Pro Gln
1               5                   10


<210> 16
<211> 14
<212> PRT
<213> Artificial Sequence

<220>
<223> Description of Artificial Sequence: gluten-derived
      epitope

<220>
<221> SITE
<222> (1)..(14)

<400> 16
Pro Phe Pro Gln Ser Glu Gln Pro Gln Gln Pro Phe Pro Gln
1               5                   10


<210> 17
<211> 14
<212> PRT
<213> Artificial Sequence

<220>
<223> Description of Artificial Sequence: gluten-derived
      epitope

<220>
<221> SITE
<222> (1)..(14)

<400> 17
Gln Gln Pro Tyr Pro Glu Gln Pro Glu Gln Pro Phe Pro Gln
1               5                   10


<210> 18
<211> 14
<212> PRT
<213> Artificial Sequence

<220>
<223> Description of Artificial Sequence: gluten-derived
      epitope

P58975ep
<220>
<221> SITE
<222> (1)..(14)

<400> 18
Gln Gln Pro Phe Pro Glu Gln Pro Glu Gln Pro Phe Pro Gln
1               5                   10


<210> 19
<211> 14
<212> PRT
<213> Artificial Sequence

<220>
<223> Description of Artificial Sequence: gluten-derived
      epitope

<220>
<221> SITE
<222> (1)..(14)

<400> 19
Pro Phe Pro Gln Thr Glu Gln Pro Glu Gln Pro Phe Pro Gln
1               5                   10


<210> 20
<211> 14
<212> PRT
<213> Artificial Sequence

<220>
<223> Description of Artificial Sequence: gluten-derived
      epitope

<220>
<221> SITE
<222> (1)..(14)

<400> 20
Pro Phe Pro Gln Leu Glu Gln Pro Glu Gln Pro Phe Pro Gln
1               5                   10


<210> 21
<211> 14
<212> PRT
<213> Artificial Sequence

<220>
<223> Description of Artificial Sequence: gluten-derived
      epitope

<220>
<221> SITE
<222> (1)..(14)

<400> 21
Gln Phe Pro Gln Thr Glu Gln Pro Glu Gln Pro Phe Pro Gln
1               5                   10


<210> 22
<211> 14
<212> PRT
<213> Artificial Sequence

P58975ep
<220>
<223> Description of Artificial Sequence: gluten-derived
      epitope

<220>
<221> SITE
<222> (1)..(14)

<400> 22
Gln Leu Pro Phe Pro Glu Gln Pro Glu Gln Pro Phe Pro Gln
  1               5                   10


<210> 23
<211> 14
<212> PRT
<213> Artificial Sequence

<220>
<223> Description of Artificial Sequence: gluten-derived
      epitope

<220>
<221> SITE
<222> (1)..(14)

<400> 23
Pro Phe Pro Gln Pro Glu Gln Pro Glu Gln Pro Phe Pro Gln
  1               5                   10


<210> 24
<211> 14
<212> PRT
<213> Artificial Sequence

<220>
<223> Description of Artificial Sequence: gluten-derived
      epitope

<220>
<221> SITE
<222> (1)..(14)

<400> 24
Pro Phe Pro Gln Ser Glu Gln Pro Glu Gln Pro Phe Pro Gln
  1               5                   10


<210> 25
<211> 14
<212> PRT
<213> Artificial Sequence

<220>
<223> Description of Artificial Sequence: gluten-derived
      epitope

<220>
<221> SITE
<222> (1)..(14)

<400> 25
Gln Gln Pro Tyr Pro Gln Gln Pro Glu Gln Pro Phe Pro Gln
  1               5                   10


<210> 26

P58975ep
<211> 14
<212> PRT
<213> Artificial Sequence

<220>
<223> Description of Artificial Sequence: gluten-derived
      epitope

<220>
<221> SITE
<222> (1)..(14)

<400> 26
Gln Gln Pro Phe Pro Gln Gln Pro Glu Gln Pro Phe Pro Gln
1               5                   10


<210> 27
<211> 14
<212> PRT
<213> Artificial Sequence

<220>
<223> Description of Artificial Sequence: gluten-derived
      epitope

<220>
<221> SITE
<222> (1)..(14)

<400> 27
Pro Phe Pro Gln Thr Gln Gln Pro Glu Gln Pro Phe Pro Gln
1               5                   10


<210> 28
<211> 14
<212> PRT
<213> Artificial Sequence

<220>
<223> Description of Artificial Sequence: gluten-derived
      epitope

<220>
<221> SITE
<222> (1)..(14)

<400> 28
Pro Phe Pro Gln Leu Gln Gln Pro Glu Gln Pro Phe Pro Gln
1               5                   10


<210> 29
<211> 14
<212> PRT
<213> Artificial Sequence

<220>
<223> Description of Artificial Sequence: gluten-derived
      epitope

<220>
<221> SITE
<222> (1)..(14)

<400> 29
Gln Phe Pro Gln Thr Gln Gln Pro Glu Gln Pro Phe Pro Gln

P58975ep
1               5                   10

<210> 30
<211> 14
<212> PRT
<213> Artificial Sequence

<220>
<223> Description of Artificial Sequence: gluten-derived
      epitope

<220>
<221> SITE
<222> (1)..(14)

<400> 30
Gln Leu Pro Phe Pro Gln Gln Pro Glu Gln Pro Phe Pro Gln
1               5                   10

<210> 31
<211> 14
<212> PRT
<213> Artificial Sequence

<220>
<223> Description of Artificial Sequence: gluten-derived
      epitope

<220>
<221> SITE
<222> (1)..(14)

<400> 31
Pro Phe Pro Gln Pro Gln Gln Pro Glu Gln Pro Phe Pro Gln
1               5                   10

<210> 32
<211> 14
<212> PRT
<213> Artificial Sequence

<220>
<223> Description of Artificial Sequence: gluten-derived
      epitope

<220>
<221> SITE
<222> (1)..(14)

<400> 32
Pro Phe Pro Gln Ser Gln Gln Pro Glu Gln Pro Phe Pro Gln
1               5                   10

<210> 33
<211> 10
<212> PRT
<213> Artificial Sequence

<220>
<223> Description of Artificial Sequence: consensus
      sequence for gluten-derived peptide or epitope

<220>
<221> SITE

P58975ep
<222> (1)..(10)
<223> /note="X on position 1, 2, 3, 5 and 8 can be any
      amino acid and X on position 10 is F, Y, M, W, I
      or L"

<400> 33
Xaa Xaa Xaa Gln Xaa Pro Gln Xaa Pro Xaa
1               5                   10


<210> 34
<211> 10
<212> PRT
<213> Artificial Sequence

<220>
<223> Description of Artificial Sequence: consensus
      sequence for gluten-derived peptide or epitope

<220>
<221> SITE
<222> (1)..(10)
<223> /note="X on position 1 2, 3, 5, 8 and 10 can be
      any amino acid and X on position 9 is F, Y, M, W,
      I or L"

<400> 34
Xaa Xaa Xaa Gln Xaa Pro Gln Xaa Xaa Xaa
1               5                   10


<210> 35
<211> 3
<212> PRT
<213> Artificial Sequence

<220>
<223> Description of Artificial Sequence: consensus
      sequence for gluten-derived peptide or epitope

<220>
<221> SITE
<222> (1)..(3)
<223> /note="X on position 2 can be any amino acid"

<400> 35
Gln Xaa Pro
1


<210> 36
<211> 4
<212> PRT
<213> Artificial Sequence

<220>
<223> Description of Artificial Sequence: consensus
      sequence for gluten-derived peptide or epitope

<220>
<221> SITE
<222> (1)..(4)
<223> /note="X on position 2 can be any amino acid and X
      on position 4 is F, Y, W, M, L, I or V"

<400> 36
Gln Xaa Pro Xaa
1

P58975ep

<210> 37
<211> 4
<212> PRT
<213> Artificial Sequence

<220>
<223> Description of Artificial Sequence: consensus
      sequence for gluten-derived peptide or epitope

<220>
<221> SITE
<222> (1)..(4)
<223> /note="X on position 2 and 3 can any amino acid
      and X on position 4 is F, Y, W , M, L, I or V"

<400> 37
Gln Xaa Xaa Xaa
  1


<210> 38
<211> 16
<212> PRT
<213> Artificial Sequence

<220>
<223> Description of Artificial Sequence: gliadin
      peptide part which is able to stimulate T-cells

<220>
<221> SITE
<222> (1)..(16)

<400> 38
Pro Ser Gly Gln Gly Ser Phe Gln Pro Ser Gln Gln Asn Pro Gln Ala
  1               5                  10                  15


<210> 39
<211> 4
<212> PRT
<213> Artificial Sequence

<220>
<223> Description of Artificial Sequence: gluten-derived
      sequence wherein no or very little deamidation of
      the Q-residue was observed

<220>
<221> SITE
<222> (1)..(4)
<223> /note="Wherein X can be any amino acid"

<400> 39
Gln Xaa Xaa Pro
  1


<210> 40
<211> 8
<212> PRT
<213> Artificial Sequence

<220>
<223> Description of Artificial Sequence: amino acid
      sequence derivable from protein auto-antigen

P58975ep
containing the tTG substrate motif

<220>
<221> SITE
<222> (1)..(8)

<400> 40
Phe Ser Trp Gly Ala Glu Gly Gln
1               5


<210> 41
<211> 8
<212> PRT
<213> Artificial Sequence

<220>
<223> Description of Artificial Sequence: amino acid
      sequence derivable from protein auto-antigen
      containing the tTG substrate motif

<220>
<221> SITE
<222> (1)..(8)

<400> 41
Ser Trp Gly Ala Glu Gly Gln Arg
1               5


<210> 42
<211> 8
<212> PRT
<213> Artificial Sequence

<220>
<223> Description of Artificial Sequence: amino acid
      sequence derivable from protein auto-antigen
      containing the tTG substrate motif

<220>
<221> SITE
<222> (1)..(8)

<400> 42
Trp Gly Ala Glu Gly Gln Arg Pro
1               5


<210> 43
<211> 8
<212> PRT
<213> Artificial Sequence

<220>
<223> Description of Artificial Sequence: amino acid
      sequence derivable from protein auto-antigen
      containing the tTG substrate motif

<220>
<221> SITE
<222> (1)..(8)

<400> 43
Gly Ala Glu Gly Gln Arg Pro Gly
1               5

P58975ep
<210> 44
<211> 8
<212> PRT
<213> Artificial Sequence

<220>
<223> Description of Artificial Sequence: amino acid
      sequence derivable from protein auto-antigen
      containing the tTG substrate motif

<220>
<221> SITE
<222> (1)..(8)

<400> 44
Ala Glu Gly Gln Arg Pro Gly Phe
  1               5


<210> 45
<211> 8
<212> PRT
<213> Artificial Sequence

<220>
<223> Description of Artificial Sequence: amino acid
      sequence derivable from protein auto-antigen
      containing the tTG substrate motif

<220>
<221> SITE
<222> (1)..(8)

<400> 45
Glu Gly Gln Arg Pro Gly Phe Gly
  1               5


<210> 46
<211> 8
<212> PRT
<213> Artificial Sequence

<220>
<223> Description of Artificial Sequence: amino acid
      sequence derivable from protein auto-antigen
      containing the tTG substrate motif

<220>
<221> SITE
<222> (1)..(8)

<400> 46
Gly Gln Arg Pro Gly Phe Gly Tyr
  1               5


<210> 47
<211> 8
<212> PRT
<213> Artificial Sequence

<220>
<223> Description of Artificial Sequence: amino acid
      sequence derivable from protein auto-antigen
      containing the tTG substrate motif

<220>

P58975ep
<221> SITE
<222> (1)..(8)

<400> 47
Gln Arg Pro Gly Phe Gly Tyr Gly
1               5


<210> 48
<211> 4
<212> PRT
<213> Artificial Sequence

<220>
<223> Description of Artificial Sequence: gluten
      sequence for which tTG has defined specificity

<220>
<221> SITE
<222> (1)..(4)

<400> 48
Gln Leu Pro Phe
1


<210> 49
<211> 4
<212> PRT
<213> Artificial Sequence

<220>
<223> Description of Artificial Sequence: gluten
      sequence for which tTG has defined specificity

<220>
<221> SITE
<222> (1)..(4)

<400> 49
Gln Xaa Xaa Phe
1


<210> 50
<211> 13
<212> PRT
<213> Artificial Sequence

<220>
<223> Description of Artificial Sequence: part of
      gliadin peptide

<220>
<221> SITE
<222> (1)..(13)

<400> 50
Pro Ser Gly Gln Gly Ser Phe Gln Pro Ser Gln Gln Asn
1               5                   10


<210> 51
<211> 4
<212> PRT
<213> Artificial Sequence

<220>

P58975ep
<223> Description of Artificial Sequence: part of
      gliadin peptide

<220>
<221> SITE
<222> (1)..(4)

<400> 51
Gln Gly Ser Phe
  1


<210> 52
<211> 4
<212> PRT
<213> Artificial Sequence

<220>
<223> Description of Artificial Sequence: part of
      gliadin peptide

<220>
<221> SITE
<222> (1)..(4)

<400> 52
Gln Asn Pro Gln
  1


<210> 53
<211> 15
<212> PRT
<213> Artificial Sequence

<220>
<223> Description of Artificial Sequence: gluten-derived
      peptide

<220>
<221> SITE
<222> (1)..(15)

<400> 53
Leu Gln Gln Pro Gln Gln Pro Gln Phe Gln Pro Gln Gln Gln Phe
  1               5                   10                  15


<210> 54
<211> 4
<212> PRT
<213> Artificial Sequence

<220>
<223> Description of Artificial Sequence: gluten-derived
      sequence allowing deamidation at Q7

<220>
<221> SITE
<222> (1)..(4)

<400> 54
Gln Xaa Pro Phe
  1


<210> 55
<211> 10

P58975ep
<212> PRT
<213> Artificial Sequence

<220>
<223> Description of Artificial Sequence: variant of
      algorithm 2

<220>
<221> SITE
<222> (1)..(10)
<223> /note="Wherein X on position 10 can be Y, F, W, I
      or L"

<400> 55
Xaa Xaa Xaa Xaa Xaa Pro Gln Xaa Pro Xaa
  1               5                   10


<210> 56
<211> 12
<212> PRT
<213> Artificial Sequence

<220>
<223> Description of Artificial Sequence: alpha-gliadin
      peptide which is immunodominant in adult celiac
      disease patients

<220>
<221> SITE
<222> (1)..(12)

<400> 56
Gln Leu Gln Pro Phe Pro Gln Pro Gln Leu Pro Tyr
  1               5                   10


<210> 57
<211> 9
<212> PRT
<213> Artificial Sequence

<220>
<223> Description of Artificial Sequence: part of gluten
      sequence

<220>
<221> SITE
<222> (1)..(9)

<400> 57
Pro Phe Ser Gln Gln Gln Gln Pro Val
  1               5


<210> 58
<211> 10
<212> PRT
<213> Artificial Sequence

<220>
<223> Description of Artificial Sequence: sequence which
      is homologous to gluten sequence

<220>
<221> SITE
<222> (1)..(10)

P58975ep
<400> 58
Pro Phe Ser Gln Gln Gln Gln Leu Val Leu
1               5                   10


<210> 59
<211> 22
<212> PRT
<213> Artificial Sequence

<220>
<223> Description of Artificial Sequence: gluten peptide

<220>
<221> SITE
<222> (1)..(22)

<400> 59
Val Pro Val Pro Gln Leu Gln Pro Gln Asn Pro Ser Gln Gln Gln Pro
1               5                   10                  15

Gln Glu Gln Val Pro Leu
            20


<210> 60
<211> 18
<212> PRT
<213> Artificial Sequence

<220>
<223> Description of Artificial Sequence: gluten peptide

<220>
<221> SITE
<222> (1)..(18)

<400> 60
Pro Leu Val Gln Gln Gln Gln Phe Leu Gly Gln Gln Gln Pro Phe Pro
1               5                   10                  15

Pro Gln


<210> 61
<211> 12
<212> PRT
<213> Artificial Sequence

<220>
<223> Description of Artificial Sequence: gluten peptide

<220>
<221> SITE
<222> (1)..(12)

<400> 61
Tyr Tyr Pro Thr Ser Pro Gln Gln Ser Gly Gln Gly
1               5                   10


<210> 62
<211> 12
<212> PRT
<213> Artificial Sequence

<220>
Page 17

P58975ep
<223> Description of Artificial Sequence: gluten peptide

<220>
<221> SITE
<222> (1)..(12)

<400> 62
Gln Gly Gln Gln Gly Tyr Tyr Pro Thr Ser Pro Gln
1               5                   10


<210> 63
<211> 12
<212> PRT
<213> Artificial Sequence

<220>
<223> Description of Artificial Sequence: gluten peptide

<220>
<221> SITE
<222> (1)..(12)

<400> 63
Ser Ser Gln Val Ser Phe Gln Pro Ser Gln Leu Asn
1               5                   10


<210> 64
<211> 15
<212> PRT
<213> Artificial Sequence

<220>
<223> Description of Artificial Sequence: gluten peptide

<220>
<221> SITE
<222> (1)..(15)

<400> 64
Phe Pro Gln Thr Gln Gln Pro Gln Gln Leu Phe Pro Gln Ser Gln
1               5                   10                  15


<210> 65
<211> 15
<212> PRT
<213> Artificial Sequence

<220>
<223> Description of Artificial Sequence: gluten peptide

<220>
<221> SITE
<222> (1)..(15)

<400> 65
Gly Gln Gln Gly Tyr Tyr Pro Thr Ser Val Gln Gln Ser Gly Gln
1               5                   10                  15


<210> 66
<211> 15
<212> PRT
<213> Artificial Sequence

<220>

P58975ep
<223> Description of Artificial Sequence: gluten peptide

<220>
<221> SITE
<222> (1)..(15)

<400> 66
Gly Ser Val Gln Pro Gln Gln Gln Leu Pro Gln Phe Glu Ile Arg
1               5                   10                  15


<210> 67
<211> 18
<212> PRT
<213> Artificial Sequence

<220>
<223> Description of Artificial Sequence: gluten peptide

<220>
<221> SITE
<222> (1)..(18)

<400> 67
Phe Pro Gln His Cys Asn Tyr Gln Gln Gln Pro Gln Thr Phe Pro Gln
1               5                   10                  15

Pro Phe



<210> 68
<211> 15
<212> PRT
<213> Artificial Sequence

<220>
<223> Description of Artificial Sequence: gluten peptide

<220>
<221> SITE
<222> (1)..(15)

<400> 68
Gln Gln Pro Ile Gln Pro Gln Gln Phe Pro Gln Gln Gln Phe Phe
1               5                   10                  15


<210> 69
<211> 14
<212> PRT
<213> Artificial Sequence

<220>
<223> Description of Artificial Sequence: gluten peptide

<220>
<221> SITE
<222> (1)..(14)

<400> 69
Gln Ser Gly Gln Tyr Gln Gln Gln Pro Gln Met Gln Thr Thr
1               5                   10


<210> 70
<211> 13
<212> PRT

P58975ep
<213> Artificial Sequence

<220>
<223> Description of Artificial Sequence: gluten peptide

<220>
<221> SITE
<222> (1)..(13)

<400> 70
Glu Ser Gln Gln Ser Gln Gln Asp Glu Pro Gln Pro Phe
1               5                   10


<210> 71
<211> 13
<212> PRT
<213> Artificial Sequence

<220>
<223> Description of Artificial Sequence: gluten peptide

<220>
<221> SITE
<222> (1)..(13)

<400> 71
Phe Pro Gln Pro Glu Asp Gln Gln Ser Gln Gln Ser Glu
1               5                   10


<210> 72
<211> 19
<212> PRT
<213> Artificial Sequence

<220>
<223> Description of Artificial Sequence: gluten peptide

<220>
<221> SITE
<222> (1)..(19)

<400> 72
Leu Gln Gln Val Gln Gln Gly Pro Gln Gln Gln Pro Phe Pro Gln Pro
1               5                   10                  15

Gln Pro Phe


<210> 73
<211> 17
<212> PRT
<213> Artificial Sequence

<220>
<223> Description of Artificial Sequence: gluten peptide

<220>
<221> SITE
<222> (1)..(17)

<400> 73
Gln Val Gln Trp Pro Gln Gln Gln Pro Phe Pro Gln Pro Gln Gln Pro
1               5                   10                  15

Phe

P58975ep

<210> 74
<211> 17
<212> PRT
<213> Artificial Sequence

<220>
<223> Description of Artificial Sequence: gluten peptide

<220>
<221> SITE
<222> (1)..(17)

<400> 74
Pro Leu Leu Pro Gln Gln Pro Phe Pro Ser Gln Gln Glu Gln Pro Gln
1               5                   10                  15

Phe

<210> 75
<211> 14
<212> PRT
<213> Artificial Sequence

<220>
<223> Description of Artificial Sequence: gluten peptide

<220>
<221> SITE
<222> (1)..(14)

<400> 75
Trp Gln Gln Gln Pro Pro Phe Ser Gln Glu Gln Pro Ile Leu
1               5                   10

<210> 76
<211> 20
<212> PRT
<213> Artificial Sequence

<220>
<223> Description of Artificial Sequence: gluten peptide

<220>
<221> SITE
<222> (1)..(20)

<400> 76
Gln Ser Asn Leu Pro Gln Pro Ala Gln Gln Pro Phe Gln Pro Gln Val
1               5                   10                  15

Pro Gln Gln Pro
            20

<210> 77
<211> 17
<212> PRT
<213> Artificial Sequence

<220>
<223> Description of Artificial Sequence: gluten peptide

P58975ep
<220>
<221> SITE
<222> (1)..(17)

<400> 77
Trp Phe Gln Pro Ser Gln Leu Asn Pro Gln Ala Gln Gln Asp Gln Pro
1               5                   10                  15

Gln



<210> 78
<211> 13
<212> PRT
<213> Artificial Sequence

<220>
<223> Description of Artificial Sequence: gluten peptide

<220>
<221> SITE
<222> (1)..(13)

<400> 78
Val Gln Gln Gln Ile Pro Val Val Gln Pro Ser Ile Leu
1               5                   10


<210> 79
<211> 14
<212> PRT
<213> Artificial Sequence

<220>
<223> Description of Artificial Sequence: DQ2 epitope
      selection algorithm 1

<220>
<221> SITE
<222> (1)..(14)
<223> /note="Wherein X on position 11 can be F, Y, W, I
      or L"

<400> 79
Xaa Xaa Xaa Xaa Xaa Gln Xaa Pro Gln Xaa Xaa Xaa Xaa Xaa
1               5                   10


<210> 80
<211> 14
<212> PRT
<213> Artificial Sequence

<220>
<223> Description of Artificial Sequence: DQ2 epitope
      selection algorithm 2

<220>
<221> SITE
<222> (1)..(14)
<223> /note="Wherein X on position 12 can be F, Y, W, I
      or L"

<400> 80
Xaa Xaa Xaa Xaa Xaa Gln Xaa Pro Gln Xaa Pro Xaa Xaa Xaa
1               5                   10

P58975ep

<210> 81
<211> 7
<212> PRT
<213> Artificial Sequence

<220>
<223> Description of Artificial Sequence: DQ2 epitope
      selection algorithm 2

<220>
<221> SITE
<222> (1)..(7)
<223> /note="Wherein X on position 7 can be Y, F, W, I
      or L"

<400> 81
Gln Xaa Pro Gln Xaa Pro Xaa
  1               5


<210> 82
<211> 6
<212> PRT
<213> Artificial Sequence

<220>
<223> Description of Artificial Sequence: DQ2 epitope
      selection algorithm 1

<220>
<221> SITE
<222> (1)..(6)
<223> /note="Wherein X on position 6 can be Y, F, W, I
      or L"

<400> 82
Gln Xaa Pro Gln Xaa Xaa
  1               5


<210> 83
<211> 6
<212> PRT
<213> Artificial Sequence

<220>
<223> Description of Artificial Sequence: DQ2 epitope
      selection algorithm 1-Q4

<220>
<221> SITE
<222> (1)..(6)
<223> /note="Wherein X on position 6 can be Y, F, W, I
      or L"

<400> 83
Xaa Xaa Pro Gln Xaa Xaa
  1               5


<210> 84
<211> 6
<212> PRT
<213> Artificial Sequence

<220>
<223> Description of Artificial Sequence: DQ2 epitope

P58975ep
selection algorithm 1-Q7

<220>
<221> SITE
<222> (1)..(6)
<223> /note="Wherein X on position 6 can be Y, F, W, I
or L"

<400> 84
Gln Xaa Pro Xaa Xaa Xaa
1               5


<210> 85
<211> 7
<212> PRT
<213> Artificial Sequence

<220>
<223> Description of Artificial Sequence: DQ2 epitope
selection algorithm 2

<220>
<221> SITE
<222> (1)..(7)
<223> /note="Wherein X on position 7 can be Y, F, W, I
or L"

<400> 85
Gln Xaa Pro Gln Xaa Pro Xaa
1               5


<210> 86
<211> 7
<212> PRT
<213> Artificial Sequence

<220>
<223> Description of Artificial Sequence: DQ2 epitope
selection algorithm 2-Q4

<220>
<221> SITE
<222> (1)..(7)
<223> /note="Wherein X on position 7 can be Y, F, W, I
or L"

<400> 86
Xaa Xaa Pro Gln Xaa Pro Xaa
1               5


<210> 87
<211> 7
<212> PRT
<213> Artificial Sequence

<220>
<223> Description of Artificial Sequence: DQ2 epitope
selection algorithm 2-Q7

<220>
<221> SITE
<222> (1)..(7)
<223> /note="Wherein X on position 7 can be Y, F, W, I
or L"

P58975ep
<400> 87
Gln Xaa Pro Xaa Xaa Pro Xaa
1               5


<210> 88
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 88
Ser Arg Phe Ser Trp Gly Ala Glu Gly Gln Arg Pro Gly Phe Gly Tyr
1               5                   10                  15

Gly Gly Arg



<210> 89
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 89
His Lys Gly Phe Lys Gly Val Asp Ala Gln Gly Thr Leu Ser Lys Ile
1               5                   10                  15

Phe Lys Leu



<210> 90
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 90
Leu Gln Val Ser Ser Ser Tyr Ala Gly Gln Phe Arg Val Ile Gly Pro
1               5                   10                  15

Arg His Pro

P58975ep
<210> 91
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 91
Arg Asn Gly Lys Asp Gln Asp Gly Asp Gln Ala Pro Glu Tyr Arg Gly
1               5                   10                  15

Arg Thr Glu



<210> 92
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 92
Leu Ala Val Leu Pro Val Leu Leu Leu Gln Ile Thr Val Gly Leu Val
1               5                   10                  15

Phe Leu Cys



<210> 93
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 93
Thr Val Gly Leu Val Phe Leu Cys Leu Gln Tyr Arg Leu Arg Gly Lys
1               5                   10                  15

Leu Arg Ala



<210> 94
<211> 19
<212> PRT
<213> Artificial Sequence

P58975ep
<220>
<223> Description of Artificial Sequence: peptide
      derived from multiple sclerosis autoantigen

<220>
<221> SITE
<222> (1)..(19)

<400> 94
Tyr Leu Ile Asn Val Ile His Ala Phe Gln Tyr Val Ile Tyr Gly Thr
1               5                   10                  15

Ala Ser Phe



<210> 95
<211> 19
<212> PRT
<213> Artificial Sequence

<220>
<223> Description of Artificial Sequence: peptide
      derived from multiple sclerosis autoantigen

<220>
<221> SITE
<222> (1)..(19)

<400> 95
Leu Ser Ile Cys Lys Thr Ala Glu Phe Gln Met Thr Phe His Leu Phe
1               5                   10                  15

Ile Ala Ala



<210> 96
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 96
Tyr Tyr Phe Thr Phe Gln Val Leu Ser Gln Trp Glu Ile Cys Leu Ser
1               5                   10                  15

Ile Val Ser



<210> 97
<211> 19
<212> PRT
<213> Artificial Sequence

<220>
<223> Description of Artificial Sequence: peptide
      derived from rheumatoid arthritis autoantigen

<220>

P58975ep
<221> SITE
<222> (1)..(19)

<400> 97
Met Val Leu Gln Leu Gln Gln Gly Asp Gln Val Trp Val Glu Lys Asp
1               5                   10                  15

Pro Lys Lys



<210> 98
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 98
Leu Leu Leu Gly Leu Ile Asp Ile Ser Gln Ala Gln Leu Ser Cys Thr
1               5                   10                  15

Gly Pro Pro



<210> 99
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 99
Asn Leu Met Arg Gly Arg Glu Arg Ala Gln Lys Val Val Thr Phe Cys
1               5                   10                  15

Asp Tyr Ala



<210> 100
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 100
Glu Glu Gly Arg Tyr Lys Gln Lys Phe Gln Ser Val Phe Thr Val Thr

P58975ep
1               5                   10                  15

Arg Gln Thr



<210> 101
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 101
Asp Phe Pro Ile Ala Lys Gly Glu Arg Gln Ser Pro Val Asp Ile Asp
1               5                   10                  15

Thr His Thr



<210> 102
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 102
Pro Leu Asp Gly Thr Tyr Arg Leu Ile Gln Phe His Phe His Trp Gly
1               5                   10                  15

Ser Leu Asp



<210> 103
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 103
Lys Tyr Gly Asp Phe Gly Lys Ala Val Gln Gln Pro Asp Gly Leu Ala
1               5                   10                  15

Val Leu Gly

P58975ep

<210> 104
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 104
Lys Val Gly Ser Ala Lys Pro Gly Leu Gln Lys Val Val Asp Val Leu
1               5                   10                  15

Asp Ser Ile



<210> 105
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 105
Lys Lys Ala Val Ser Arg Ser Ala Glu Gln Gln Pro Ser Glu Lys Ser
1               5                   10                  15

Thr Glu Pro



<210> 106
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 106
Lys Ser Thr Glu Pro Lys Thr Lys Pro Gln Asp Met Ile Ser Ala Gly
1               5                   10                  15

Gly Glu Ser



<210> 107
<211> 19
<212> PRT
<213> Artificial Sequence

P58975ep

<220>
<223> Description of Artificial Sequence: peptide
      derived from rheumatoid arthritis autoantigen

<220>
<221> SITE
<222> (1)..(19)

<400> 107
Ala Ala Ala Ile Ser Glu Val Val Ser Gln Thr Pro Ala Ser Thr Thr
  1               5                  10                  15

Gln Ala Gly



<210> 108
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 108
Glu Asp Ser Lys Lys Pro Ala Asp Asp Gln Asp Pro Ile Asp Ala Leu
  1               5                  10                  15

Ser Gly Asp



<210> 109
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 109
Val Gly Pro Lys Gly Pro Pro Gly Pro Gln Gly Pro Ala Gly Glu Gln
  1               5                  10                  15

Gly Pro Arg



<210> 110
<211> 19
<212> PRT
<213> Artificial Sequence

<220>
<223> Description of Artificial Sequence: peptide
      derived from rheumatoid arthritis autoantigen

P58975ep
<220>
<221> SITE
<222> (1)..(19)

<400> 110
Pro Gly Pro Gln Gly Pro Ala Gly Glu Gln Gly Pro Arg Gly Asp Arg
1               5                   10                  15

Gly Asp Lys



<210> 111
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 111
Gly Phe Asp Glu Lys Ala Gly Gly Ala Gln Leu Gly Val Met Gln Gly
1               5                   10                  15

Pro Met Gly



<210> 112
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 112
Ala Gly Gly Ala Gln Leu Gly Val Met Gln Gly Pro Met Gly Pro Met
1               5                   10                  15

Gly Pro Arg



<210> 113
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 113

P58975ep
Thr Gly Ala Arg Gly Pro Glu Gly Ala Gln Gly Pro Arg Gly Glu Pro
1               5                   10                  15

Gly Thr Pro

<210> 114
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 114
Pro Gly Ile Ala Gly Phe Lys Gly Glu Gln Gly Pro Lys Gly Glu Pro
1               5                   10                  15

Gly Pro Ala

<210> 115
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 115
Ala Pro Gly Asn Arg Gly Phe Pro Gly Gln Asp Gly Leu Ala Gly Pro
1               5                   10                  15

Lys Gly Ala

<210> 116
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 116
Thr Gly Arg Pro Gly Asp Ala Gly Pro Gln Gly Lys Val Gly Pro Ser
1               5                   10                  15

Gly Ala Pro

P58975ep

<210> 117
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 117
Arg Gly Ser Pro Gly Ala Gln Gly Leu Gln Gly Pro Arg Gly Leu Pro
1               5                   10                  15

Gly Thr Pro

<210> 118
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 118
Lys Gly Asp Ala Gly Ala Pro Gly Pro Gln Gly Pro Ser Gly Ala Pro
1               5                   10                  15

Gly Pro Gln

<210> 119
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 119
Gln Gly Pro Ser Gly Ala Pro Gly Pro Gln Gly Pro Thr Gly Val Thr
1               5                   10                  15

Gly Pro Lys

<210> 120
<211> 19
<212> PRT

P58975ep
<213> Artificial Sequence

<220>
<223> Description of Artificial Sequence: peptide
      derived from rheumatoid arthritis autoantigen

<220>
<221> SITE
<222> (1)..(19)

<400> 120
Pro Gly Arg Ala Gly Glu Pro Gly Leu Gln Gly Pro Ala Gly Pro Pro
  1               5                  10                  15

Gly Glu Lys



<210> 121
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 121
Pro Pro Gly Pro Gln Gly Leu Ala Gly Gln Arg Gly Ile Val Gly Leu
  1               5                  10                  15

Pro Gly Gln



<210> 122
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 122
Gln Gly Asp Arg Gly Glu Ala Gly Ala Gln Gly Pro Met Gly Pro Ser
  1               5                  10                  15

Gly Pro Ala



<210> 123
<211> 19
<212> PRT
<213> Artificial Sequence

<220>
<223> Description of Artificial Sequence: peptide
      derived from rheumatoid arthritis autoantigen

P58975ep

<220>
<221> SITE
<222> (1)..(19)

<400> 123
Ser Gly Pro Ala Gly Ala Arg Gly Ile Gln Gly Pro Gln Gly Pro Arg
1               5                   10                  15

Gly Asp Lys



<210> 124
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 124
Ala Gly Ala Arg Gly Ile Gln Gly Pro Gln Gly Pro Arg Gly Asp Lys
1               5                   10                  15

Gly Glu Ala



<210> 125
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 125
Asn Leu Ala Pro Asn Thr Ala Asn Val Gln Met Thr Phe Leu Arg Leu
1               5                   10                  15

Leu Ser Thr



<210> 126
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

P58975ep
<400> 126
Ser Arg His Ser Thr Ser Gln Glu Gly Gln Asp Thr Ile His Gly His
1               5                   10                  15

Arg Gly Ser

<210> 127
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 127
Ala Ser Arg Asn His His Gly Ser Ala Gln Glu Gln Leu Arg Asp Gly
1               5                   10                  15

Ser Arg His

<210> 128
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 128
Ser Arg His Ser Ala Ser Gln Asp Gly Gln Asp Thr Ile Arg Gly His
1               5                   10                  15

Pro Gly Ser

<210> 129
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 129
Gly Arg Thr Trp Asn Asp Pro Ser Val Gln Gln Asp Ile Lys Phe Leu
1               5                   10                  15

Pro Phe Lys

P58975ep

<210> 130
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 130
Val Glu Lys Lys Thr Lys Pro Tyr Ile Gln Val Asp Ile Gly Gly Gly
1               5                   10                  15

Gln Thr Lys

<210> 131
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 131
Thr His Leu Gly Gly Glu Asp Phe Asp Gln Arg Val Met Glu His Phe
1               5                   10                  15

Ile Lys Leu

<210> 132
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 132
Ala Lys Arg Ala Leu Ser Ser Gln His Gln Ala Arg Ile Glu Ile Glu
1               5                   10                  15

Ser Phe Tyr

<210> 133
<211> 19

P58975ep
<212> PRT
<213> Artificial Sequence

<220>
<223> Description of Artificial Sequence: peptide
      derived from rheumatoid arthritis autoantigen

<220>
<221> SITE
<222> (1)..(19)

<400> 133
Leu Phe Arg Ser Thr Met Lys Pro Val Gln Lys Val Leu Glu Asp Ser
1               5                   10                  15

Asp Leu Lys



<210> 134
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 134
Gly Gly Ser Thr Arg Ile Pro Lys Ile Gln Gln Leu Val Lys Glu Phe
1               5                   10                  15

Phe Asn Gly



<210> 135
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 135
Glu Ala Val Ala Tyr Gly Ala Ala Val Gln Ala Gly Val Leu Ser Gly
1               5                   10                  15

Asp Gln Asp



<210> 136
<211> 19
<212> PRT
<213> Artificial Sequence

<220>
<223> Description of Artificial Sequence: peptide

P58975ep
derived from rheumatoid arthritis autoantigen

<220>
<221> SITE
<222> (1)..(19)

<400> 136
Ile Pro Pro Ala Pro Arg Gly Val Pro Gln Ile Glu Val Thr Phe Glu
1               5                   10                  15

Ile Asp Val



<210> 137
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 137
Lys Asn Lys Ile Thr Ile Thr Asn Asp Gln Asn Arg Leu Thr Pro Glu
1               5                   10                  15

Glu Ile Glu



<210> 138
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 138
Thr Gln Glu Gln Asp Val Asp Leu Val Gln Lys Tyr Leu Glu Lys Tyr
1               5                   10                  15

Tyr Asn Leu



<210> 139
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

P58975ep

<400> 139
Pro Val Val Glu Lys Leu Lys Gln Met Gln Glu Phe Phe Gly Leu Lys
1               5                   10                  15

Val Thr Gly



<210> 140
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 140
Pro Arg Cys Gly Val Pro Asp Val Ala Gln Phe Val Leu Thr Glu Gly
1               5                   10                  15

Asn Pro Arg



<210> 141
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 141
Leu Thr Glu Gly Asn Pro Arg Trp Glu Gln Thr His Leu Thr Tyr Arg
1               5                   10                  15

Ile Glu Asn



<210> 142
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 142
Leu Thr Phe Thr Lys Val Ser Glu Gly Gln Ala Asp Ile Met Ile Ser
1               5                   10                  15

P58975ep
Phe Val Arg

<210> 143
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 143
Thr Phe Ser Gly Asp Val Gln Leu Ala Gln Asp Asp Ile Asp Gly Ile
1               5                   10                  15

Gln Ala Ile

<210> 144
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 144
Leu Ala Gln Asp Asp Ile Asp Gly Ile Gln Ala Ile Tyr Gly Arg Ser
1               5                   10                  15

Gln Asn Pro

<210> 145
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 145
Gly Ile Gln Ala Ile Tyr Gly Arg Ser Gln Asn Pro Val Gln Pro Ile
1               5                   10                  15

Gly Pro Gln

<210> 146

P58975ep
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 146
Gln Asn Pro Val Gln Pro Ile Gly Pro Gln Thr Pro Lys Ala Cys Asp
1               5                   10                  15

Ser Lys Leu



<210> 147
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 147
Leu Asn Phe Ile Ser Val Phe Trp Pro Gln Leu Pro Asn Gly Leu Glu
1               5                   10                  15

Ala Ala Tyr



<210> 148
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 148
Gly Asn Lys Tyr Trp Ala Val Gln Gly Gln Asn Val Leu His Gly Tyr
1               5                   10                  15

Pro Lys Asp



<210> 149
<211> 19
<212> PRT
<213> Artificial Sequence

<220>

P58975ep
<223> Description of Artificial Sequence: peptide
      derived from rheumatoid arthritis autoantigen

<220>
<221> SITE
<222> (1)..(19)

<400> 149
Phe Phe Tyr Phe Phe His Gly Thr Arg Gln Tyr Lys Phe Asp Pro Lys
1               5                   10                  15

Thr Lys Arg



<210> 150
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 150
Gly Glu Asp Thr Ser Met Asn Leu Val Gln Lys Tyr Leu Glu Asn Tyr
1               5                   10                  15

Tyr Asp Leu



<210> 151
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 151
Pro Val Val Lys Lys Ile Arg Glu Met Gln Lys Phe Leu Gly Leu Glu
1               5                   10                  15

Val Thr Gly



<210> 152
<211> 19
<212> PRT
<213> Artificial Sequence

<220>
<223> Description of Artificial Sequence: peptide
      derived from rheumatoid arthritis autoantigen

<220>
<221> SITE

P58975ep
<222> (1)..(19)

<400> 152
Thr Asp Leu Thr Arg Phe Arg Leu Ser Gln Asp Asp Ile Asn Gly Ile
1               5                   10                  15

Gln Ser Leu



<210> 153
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 153
Leu Ser Gln Asp Asp Ile Asn Gly Ile Gln Ser Leu Tyr Gly Pro Pro
1               5                   10                  15

Pro Asp Ser



<210> 154
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 154
Phe Phe Tyr Phe Phe Thr Gly Ser Ser Gln Leu Glu Phe Asp Pro Asn
1               5                   10                  15

Ala Lys Lys



<210> 155
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 155
Glu Val Ala Pro Val Asp Tyr Leu Ser Gln Tyr Gly Tyr Leu Gln Lys
1               5                   10                  15

P58975ep

Pro Leu Glu

<210> 156
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 156
Asp Tyr Leu Ser Gln Tyr Gly Tyr Leu Gln Lys Pro Leu Glu Gly Ser
  1               5                  10                  15

Asn Asn Phe

<210> 157
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 157
Arg Cys Gly Leu Glu Asp Pro Phe Asn Gln Lys Thr Leu Lys Tyr Leu
  1               5                  10                  15

Leu Leu Gly

<210> 158
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 158
Asp Ile Arg Leu Ser Phe His Gly Arg Gln Ser Ser Tyr Cys Ser Asn
  1               5                  10                  15

Thr Phe Asp

P58975ep
<210> 159
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 159
Leu Gly Leu Gly His Ser Arg Tyr Ser Gln Ala Leu Met Ala Pro Val
1               5                   10                  15

Tyr Glu Gly



<210> 160
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 160
Leu His Pro Asp Asp Val Ala Gly Ile Gln Ala Leu Tyr Gly Lys Lys
1               5                   10                  15

Ser Pro Val



<210> 161
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 161
Asp Ala Ala Leu Tyr Trp Pro Leu Asn Gln Lys Val Phe Leu Phe Lys
1               5                   10                  15

Gly Ser Gly



<210> 162
<211> 19
<212> PRT
<213> Artificial Sequence

P58975ep
<220>
<223> Description of Artificial Sequence: peptide
      derived from rheumatoid arthritis autoantigen

<220>
<221> SITE
<222> (1)..(19)

<400> 162
Gly Lys Val Tyr Trp Arg Leu Asn Gln Gln Leu Arg Val Glu Lys Gly
1               5                   10                  15

Tyr Pro Arg



<210> 163
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 163
Asn Tyr Asn Asp Phe Gly Asn Tyr Asn Gln Gln Pro Ser Asn Tyr Gly
1               5                   10                  15

Pro Met Lys



<210> 164
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 164
Leu Pro Ala His Leu Asp Glu Glu Leu Gln Ala Thr Leu His Asp Phe
1               5                   10                  15

Arg His Gln



<210> 165
<211> 19
<212> PRT
<213> Artificial Sequence

<220>
<223> Description of Artificial Sequence: peptide
      derived from rheumatoid arthritis autoantigen

<220>

P58975ep
<221> SITE
<222> (1)..(19)

<400> 165
Leu Gln Thr Arg Gly Ala Leu Ser Leu Gln Gly Ser Ile Met Thr Val
1               5                   10                  15

Gly Glu Lys



<210> 166
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 166
Lys Met Ala Lys Met Ile Asp Glu Arg Gln Gln Glu Leu Thr His Gln
1               5                   10                  15

Glu His Arg



<210> 167
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 167
Ser Ile Ala Lys Lys Ile Asp Ala Ala Gln Asn Trp Leu Ala Asp Pro
1               5                   10                  15

Asn Gly Gly



<210> 168
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 168
His Leu Glu Gly Lys Ile Glu Gln Ala Gln Arg Trp Ile Asp Asn Pro

P58975ep
1               5                   10                  15

Thr Val Asp



<210> 169
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 169
Pro Thr Val Asp Asp Arg Gly Val Gly Gln Ala Ala Ile Arg Gly Leu
1               5                   10                  15

Val Ala Glu



<210> 170
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 170
Ala Asn Val Met Met Gly Pro Tyr Arg Gln Asp Leu Leu Ala Lys Cys
1               5                   10                  15

Asp Arg Val



<210> 171
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 171
Gln Ala Arg Ala Leu Ala Ser Gln Leu Gln Asp Ser Leu Lys Asp Leu
1               5                   10                  15

Lys Ala Arg

P58975ep

<210> 172
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 172
Arg Ile Leu Leu Arg Asn Pro Gly Asn Gln Ala Ala Tyr Glu His Phe
1               5                   10                  15

Glu Thr Met

<210> 173
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 173
Lys Val Ala Met Ala Asn Ile Gln Pro Gln Met Leu Val Ala Gly Ala
1               5                   10                  15

Thr Ser Ile

<210> 174
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 174
Ala Gly Asn Ile Ser Asp Pro Gly Leu Gln Lys Ser Phe Leu Asp Ser
1               5                   10                  15

Gly Tyr Arg

<210> 175
<211> 19
<212> PRT
<213> Artificial Sequence

P58975ep

<220>
<223> Description of Artificial Sequence: peptide
      derived from rheumatoid arthritis autoantigen

<220>
<221> SITE
<222> (1)..(19)

<400> 175
Ala Lys Val Arg Glu Ala Phe Gln Pro Gln Glu Pro Asp Phe Pro Pro
1               5                   10                  15

Pro Pro Pro

<210> 176
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 176
Phe Pro Pro Pro Pro Pro Asp Leu Glu Gln Leu Arg Leu Thr Asp Glu
1               5                   10                  15

Leu Ala Pro

<210> 177
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 177
Cys Glu Arg Ile Pro Thr Ile Ser Thr Gln Leu Lys Ile Leu Ser Thr
1               5                   10                  15

Val Lys Ala

<210> 178
<211> 19
<212> PRT
<213> Artificial Sequence

<220>
<223> Description of Artificial Sequence: peptide
      derived from rheumatoid arthritis autoantigen

P58975ep
<220>
<221> SITE
<222> (1)..(19)

<400> 178
Ala Thr Glu Met Leu Val His Asn Ala Gln Asn Leu Met Gln Ser Val
1               5                   10                  15

Lys Glu Thr



<210> 179
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 179
Tyr Phe Thr Asn Trp Ser Gln Asp Arg Gln Glu Pro Gly Lys Phe Thr
1               5                   10                  15

Pro Glu Asn



<210> 180
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 180
Leu Ile His Glu Leu Ala Glu Ala Phe Gln Lys Asp Phe Thr Lys Ser
1               5                   10                  15

Thr Lys Glu



<210> 181
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 181

P58975ep
Ala Lys Ile Thr Arg Leu Gln Asp Gln Gln Val Pro Tyr Ala Val Lys
1               5                   10                  15

Gly Asn Gln



<210> 182
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 182
Asp Asp Phe Thr Gly Lys Ser Cys Asn Gln Gly Pro Tyr Pro Leu Val
1               5                   10                  15

Gln Ala Val



<210> 183
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 183
Ile Lys Glu Ala Gln Pro Gly Lys Lys Gln Leu Leu Leu Ser Ala Ala
1               5                   10                  15

Leu Ser Ala



<210> 184
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 184
Ala Thr Val His Arg Thr Leu Gly Gln Gln Val Pro Tyr Ala Thr Lys
1               5                   10                  15

Gly Asn Gln

P58975ep

<210> 185
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 185
Gly Asn Gln Trp Val Gly Tyr Asp Asp Gln Glu Ser Val Lys Ser Lys
1               5                   10                  15

Val Gln Tyr

<210> 186
<211> 19
<212> PRT
<213> Artificial Sequence

<220>
<223> Description of Artificial Sequence: peptide
      derived from rheumatoid arthritis autoantigen

<220>
<221> SITE
<222> (1)..(19)

<400> 186
Val Trp Ala Leu Asp Leu Asp Asp Phe Gln Gly Ser Phe Cys Gly Gln
1               5                   10                  15

Asp Leu Arg

<210> 187
<211> 19
<212> PRT
<213> Artificial Sequence

<220>
<223> Description of Artificial Sequence: peptide
      derived from diabetes autoantigen

<220>
<221> SITE
<222> (1)..(19)

<400> 187
Cys Gly Trp Leu Leu Gly Ala Glu Ala Gln Glu Pro Gly Ala Pro Ala
1               5                   10                  15

Ala Gly Met

<210> 188
<211> 19
<212> PRT

P58975ep
<213> Artificial Sequence

<220>
<223> Description of Artificial Sequence: peptide
      derived from diabetes autoantigen

<220>
<221> SITE
<222> (1)..(19)

<400> 188
Ala Glu Cys Phe Pro Ala Cys Asn Pro Gln Asn Gly Phe Cys Glu Asp
1               5                   10                  15

Asp Asn Val



<210> 189
<211> 19
<212> PRT
<213> Artificial Sequence

<220>
<223> Description of Artificial Sequence: peptide
      derived from diabetes autoantigen

<220>
<221> SITE
<222> (1)..(19)

<400> 189
Asn Val Cys Arg Cys Gln Pro Gly Trp Gln Gly Pro Leu Cys Asp Gln
1               5                   10                  15

Cys Val Thr



<210> 190
<211> 19
<212> PRT
<213> Artificial Sequence

<220>
<223> Description of Artificial Sequence: peptide
      derived from diabetes autoantigen

<220>
<221> SITE
<222> (1)..(19)

<400> 190
Asn Gly Gly Thr Cys Leu Gln His Thr Gln Val Ser Tyr Glu Cys Leu
1               5                   10                  15

Cys Lys Pro



<210> 191
<211> 19
<212> PRT
<213> Artificial Sequence

<220>
<223> Description of Artificial Sequence: peptide
      derived from diabetes autoantigen

P58975ep

<220>
<221> SITE
<222> (1)..(19)

<400> 191
Thr Pro Gly Val His Glu Leu Pro Val Gln Gln Pro Glu His Arg Ile
1               5                   10                  15

Leu Lys Val



<210> 192
<211> 19
<212> PRT
<213> Artificial Sequence

<220>
<223> Description of Artificial Sequence: peptide
      derived from diabetes autoantigen

<220>
<221> SITE
<222> (1)..(19)

<400> 192
Ala Arg Lys Ala Ala Cys Ala Cys Asp Gln Lys Pro Cys Ser Cys Ser
1               5                   10                  15

Lys Val Asp



<210> 193
<211> 19
<212> PRT
<213> Artificial Sequence

<220>
<223> Description of Artificial Sequence: peptide
      derived from diabetes autoantigen

<220>
<221> SITE
<222> (1)..(19)

<400> 193
Gly Glu Arg Pro Thr Leu Ala Phe Leu Gln Asp Val Met Asn Ile Leu
1               5                   10                  15

Leu Gln Tyr



<210> 194
<211> 19
<212> PRT
<213> Artificial Sequence

<220>
<223> Description of Artificial Sequence: peptide
      derived from diabetes autoantigen

<220>
<221> SITE
<222> (1)..(19)

P58975ep
<400> 194
Leu Gln Asp Val Met Asn Ile Leu Leu Gln Tyr Val Val Lys Ser Phe
1               5                   10                  15

Asp Arg Ser



<210> 195
<211> 19
<212> PRT
<213> Artificial Sequence

<220>
<223> Description of Artificial Sequence: peptide
      derived from diabetes autoantigen

<220>
<221> SITE
<222> (1)..(19)

<400> 195
Asn Leu Glu Glu Ile Leu Met His Cys Gln Thr Thr Leu Lys Tyr Ala
1               5                   10                  15

Ile Lys Thr



<210> 196
<211> 19
<212> PRT
<213> Artificial Sequence

<220>
<223> Description of Artificial Sequence: peptide
      derived from diabetes autoantigen

<220>
<221> SITE
<222> (1)..(19)

<400> 196
Leu Glu Arg Arg Ile Leu Glu Ala Lys Gln Lys Gly Phe Val Pro Phe
1               5                   10                  15

Leu Val Ser



<210> 197
<211> 19
<212> PRT
<213> Artificial Sequence

<220>
<223> Description of Artificial Sequence: peptide
      derived from diabetes autoantigen

<220>
<221> SITE
<222> (1)..(19)

<400> 197
Ile Gly Ser Glu Asp Gly Glu Pro Pro Gln Gln Arg Val Thr Gly Thr
1               5                   10                  15

Leu Val Leu

P58975ep

<210> 198
<211> 19
<212> PRT
<213> Artificial Sequence

<220>
<223> Description of Artificial Sequence: peptide
      derived from diabetes autoantigen

<220>
<221> SITE
<222> (1)..(19)

<400> 198
Val Phe Ser Ala Val Leu Gly Ser Leu Gln Phe Gly Tyr Asn Ile Gly
1               5                   10                  15

Val Ile Asn

<210> 199
<211> 19
<212> PRT
<213> Artificial Sequence

<220>
<223> Description of Artificial Sequence: peptide
      derived from diabetes autoantigen

<220>
<221> SITE
<222> (1)..(19)

<400> 199
Tyr Asn Ile Gly Val Ile Asn Ala Pro Gln Lys Val Ile Glu Gln Ser
1               5                   10                  15

Tyr Asn Glu

<210> 200
<211> 19
<212> PRT
<213> Artificial Sequence

<220>
<223> Description of Artificial Sequence: peptide
      derived from diabetes autoantigen

<220>
<221> SITE
<222> (1)..(19)

<400> 200
Leu Arg Gly Ala Leu Gly Thr Leu Asn Gln Leu Ala Ile Val Ile Gly
1               5                   10                  15

Ile Leu Ile

<210> 201
<211> 19

P58975ep
<212> PRT
<213> Artificial Sequence

<220>
<223> Description of Artificial Sequence: peptide
      derived from diabetes autoantigen

<220>
<221> SITE
<222> (1)..(19)

<400> 201
Gly Leu Thr Val Leu Pro Ala Leu Leu Gln Leu Val Leu Leu Pro Phe
1               5                   10                  15

Cys Pro Glu



<210> 202
<211> 19
<212> PRT
<213> Artificial Sequence

<220>
<223> Description of Artificial Sequence: peptide
      derived from diabetes autoantigen

<220>
<221> SITE
<222> (1)..(19)

<400> 202
Trp Phe Ile Val Ala Glu Leu Phe Ser Gln Gly Pro Arg Pro Ala Ala
1               5                   10                  15

Met Ala Val



<210> 203
<211> 19
<212> PRT
<213> Artificial Sequence

<220>
<223> Description of Artificial Sequence: peptide
      derived from diabetes autoantigen

<220>
<221> SITE
<222> (1)..(19)

<400> 203
Thr Ser Lys Gly Gln Lys Cys Glu Phe Gln Asp Ala Tyr Val Leu Leu
1               5                   10                  15

Ser Glu Lys



<210> 204
<211> 19
<212> PRT
<213> Artificial Sequence

<220>
<223> Description of Artificial Sequence: peptide

P58975ep
derived from diabetes autoantigen

<220>
<221> SITE
<222> (1)..(19)

<400> 204
Leu Ser Glu Lys Lys Ile Ser Ser Ile Gln Ser Ile Val Pro Ala Leu
1               5                   10                  15

Glu Ile Ala



<210> 205
<211> 19
<212> PRT
<213> Artificial Sequence

<220>
<223> Description of Artificial Sequence: peptide
      derived from diabetes autoantigen

<220>
<221> SITE
<222> (1)..(19)

<400> 205
Asp Lys Ala Gln Ile Glu Lys Arg Ile Gln Glu Ile Ile Glu Gln Leu
1               5                   10                  15

Asp Val Thr



<210> 206
<211> 19
<212> PRT
<213> Artificial Sequence

<220>
<223> Description of Artificial Sequence: peptide
      derived from diabetes autoantigen

<220>
<221> SITE
<222> (1)..(19)

<400> 206
Glu Lys Arg Ile Gln Glu Ile Ile Glu Gln Leu Asp Val Thr Thr Ser
1               5                   10                  15

Glu Tyr Glu



<210> 207
<211> 19
<212> PRT
<213> Artificial Sequence

<220>
<223> Description of Artificial Sequence: peptide
      derived from diabetes autoantigen

<220>
<221> SITE
<222> (1)..(19)

P58975ep

<400> 207
Leu Cys Ser His Leu Glu Val Cys Ile Gln Asp Gly Leu Phe Gly Gln
1               5                   10                  15

Cys Gln Val



<210> 208
<211> 19
<212> PRT
<213> Artificial Sequence

<220>
<223> Description of Artificial Sequence: peptide
      derived from diabetes autoantigen

<220>
<221> SITE
<222> (1)..(19)

<400> 208
Val Cys Ile Gln Asp Gly Leu Phe Gly Gln Cys Gln Val Gly Val Gly
1               5                   10                  15

Gln Ala Arg



<210> 209
<211> 19
<212> PRT
<213> Artificial Sequence

<220>
<223> Description of Artificial Sequence: peptide
      derived from diabetes autoantigen

<220>
<221> SITE
<222> (1)..(19)

<400> 209
Ile Gln Asp Gly Leu Phe Gly Gln Cys Gln Val Gly Val Gly Gln Ala
1               5                   10                  15

Arg Pro Leu



<210> 210
<211> 19
<212> PRT
<213> Artificial Sequence

<220>
<223> Description of Artificial Sequence: peptide
      derived from diabetes autoantigen

<220>
<221> SITE
<222> (1)..(19)

<400> 210
Val Thr Ser Pro Val Leu Gln Arg Leu Gln Gly Val Leu Arg Gln Leu
1               5                   10                  15

P58975ep
Met Ser Gln

<210> 211
<211> 19
<212> PRT
<213> Artificial Sequence

<220>
<223> Description of Artificial Sequence: peptide
      derived from diabetes autoantigen

<220>
<221> SITE
<222> (1)..(19)

<400> 211
Gly Leu Ser Trp His Asp Asp Leu Thr Gln Tyr Val Ile Ser Gln Glu
  1               5                  10                  15

Met Glu Arg

<210> 212
<211> 19
<212> PRT
<213> Artificial Sequence

<220>
<223> Description of Artificial Sequence: peptide
      derived from diabetes autoantigen

<220>
<221> SITE
<222> (1)..(19)

<400> 212
Ile Pro Thr Gly Ser Ala Pro Ala Ala Gln His Arg Leu Pro Gln Pro
  1               5                  10                  15

Pro Val Gly

<210> 213
<211> 19
<212> PRT
<213> Artificial Sequence

<220>
<223> Description of Artificial Sequence: peptide
      derived from diabetes autoantigen

<220>
<221> SITE
<222> (1)..(19)

<400> 213
Gly Ala Ser Ser Ser Leu Ser Pro Leu Gln Ala Glu Leu Leu Pro Pro
  1               5                  10                  15

Leu Leu Glu

<210> 214

P58975ep
<211> 19
<212> PRT
<213> Artificial Sequence

<220>
<223> Description of Artificial Sequence: peptide
      derived from diabetes autoantigen

<220>
<221> SITE
<222> (1)..(19)

<400> 214
Glu Glu Tyr Gly Tyr Ile Val Thr Asp Gln Lys Pro Leu Ser Leu Ala
  1               5                  10                  15

Ala Gly Val



<210> 215
<211> 19
<212> PRT
<213> Artificial Sequence

<220>
<223> Description of Artificial Sequence: peptide
      derived from diabetes autoantigen

<220>
<221> SITE
<222> (1)..(19)

<400> 215
Asn Leu Ser Leu Ala Asp Val Thr Gln Gln Ala Gly Leu Val Lys Ser
  1               5                  10                  15

Glu Leu Glu



<210> 216
<211> 19
<212> PRT
<213> Artificial Sequence

<220>
<223> Description of Artificial Sequence: peptide
      derived from diabetes autoantigen

<220>
<221> SITE
<222> (1)..(19)

<400> 216
Gly Leu Val Lys Ser Glu Leu Glu Ala Gln Thr Gly Leu Gln Ile Leu
  1               5                  10                  15

Gln Thr Gly



<210> 217
<211> 19
<212> PRT
<213> Artificial Sequence

<220>

P58975ep
<223> Description of Artificial Sequence: peptide
      derived from diabetes autoantigen

<220>
<221> SITE
<222> (1)..(19)

<400> 217
Glu Ala Gln Thr Gly Leu Gln Ile Leu Gln Thr Gly Val Gly Gln Arg
  1               5                  10                  15

Glu Glu Ala



<210> 218
<211> 19
<212> PRT
<213> Artificial Sequence

<220>
<223> Description of Artificial Sequence: peptide
      derived from diabetes autoantigen

<220>
<221> SITE
<222> (1)..(19)

<400> 218
Thr Pro Ser Trp Cys Glu Glu Pro Ala Gln Ala Asn Met Asp Ile Ser
  1               5                  10                  15

Thr Gly His



<210> 219
<211> 19
<212> PRT
<213> Artificial Sequence

<220>
<223> Description of Artificial Sequence: peptide
      derived from diabetes autoantigen

<220>
<221> SITE
<222> (1)..(19)

<400> 219
Pro Arg Met Pro Ala Tyr Ile Ala Thr Gln Gly Pro Leu Ser His Thr
  1               5                  10                  15

Ile Ala Asp



<210> 220
<211> 19
<212> PRT
<213> Artificial Sequence

<220>
<223> Description of Artificial Sequence: peptide
      derived from diabetes autoantigen

<220>
<221> SITE

P58975ep
<222> (1)..(19)

<400> 220
Leu Ser His Thr Ile Ala Asp Phe Trp Gln Met Val Trp Glu Ser Gly
1               5                   10                  15

Cys Thr Val



<210> 221
<211> 19
<212> PRT
<213> Artificial Sequence

<220>
<223> Description of Artificial Sequence: peptide
      derived from diabetes autoantigen

<220>
<221> SITE
<222> (1)..(19)

<400> 221
Gln Thr Gln Glu Thr Arg Thr Leu Thr Gln Phe His Phe Leu Ser Trp
1               5                   10                  15

Pro Ala Glu



<210> 222
<211> 19
<212> PRT
<213> Artificial Sequence

<220>
<223> Description of Artificial Sequence: peptide
      derived from diabetes autoantigen

<220>
<221> SITE
<222> (1)..(19)

<400> 222
Ala Ala Thr Leu Glu His Val Arg Asp Gln Arg Pro Gly Leu Val Arg
1               5                   10                  15

Ser Lys Asp



<210> 223
<211> 19
<212> PRT
<213> Artificial Sequence

<220>
<223> Description of Artificial Sequence: peptide
      derived from diabetes autoantigen

<220>
<221> SITE
<222> (1)..(19)

<400> 223
Arg Pro Gly Leu Val Arg Ser Lys Asp Gln Phe Glu Phe Ala Leu Thr
1               5                   10                  15

P58975ep

Ala Val Ala

<210> 224
<211> 19
<212> PRT
<213> Artificial Sequence

<220>
<223> Description of Artificial Sequence: peptide
      derived from diabetes autoantigen

<220>
<221> SITE
<222> (1)..(19)

<400> 224
His Lys Cys Ser Tyr Pro Trp Asp Leu Gln Asp Arg Tyr Ala Gln Asp
1               5                   10                  15

Lys Ser Val

<210> 225
<211> 19
<212> PRT
<213> Artificial Sequence

<220>
<223> Description of Artificial Sequence: peptide
      derived from diabetes autoantigen

<220>
<221> SITE
<222> (1)..(19)

<400> 225
Gln Asp Lys Ser Val Val Asn Lys Met Gln Gln Arg Tyr Trp Glu Thr
1               5                   10                  15

Lys Gln Ala

<210> 226
<211> 19
<212> PRT
<213> Artificial Sequence

<220>
<223> Description of Artificial Sequence: peptide
      derived from diabetes autoantigen

<220>
<221> SITE
<222> (1)..(19)

<400> 226
Asp Lys Ser Val Val Asn Lys Met Gln Gln Arg Tyr Trp Glu Thr Lys
1               5                   10                  15

Gln Ala Phe

P58975ep
<210> 227
<211> 19
<212> PRT
<213> Artificial Sequence

<220>
<223> Description of Artificial Sequence: peptide
      derived from diabetes autoantigen

<220>
<221> SITE
<222> (1)..(19)

<400> 227
Met Gln Gln Arg Tyr Trp Glu Thr Lys Gln Ala Phe Ile Lys Ala Thr
1               5                   10                  15

Gly Lys Lys



<210> 228
<211> 19
<212> PRT
<213> Artificial Sequence

<220>
<223> Description of Artificial Sequence: peptide
      derived from diabetes autoantigen

<220>
<221> SITE
<222> (1)..(19)

<400> 228
Asp Leu Ser Lys Ala Ile Val Leu Tyr Gln Lys Arg Ile Cys Phe Leu
1               5                   10                  15

Ser Gln Glu



<210> 229
<211> 19
<212> PRT
<213> Artificial Sequence

<220>
<223> Description of Artificial Sequence: peptide
      derived from diabetes autoantigen

<220>
<221> SITE
<222> (1)..(19)

<400> 229
Thr Gly Lys Ala Leu Cys Phe Ser Ser Gln Gln Arg Leu Ala Leu Arg
1               5                   10                  15

Asn Pro Leu



<210> 230
<211> 19
<212> PRT
<213> Artificial Sequence

P58975ep
<220>
<223> Description of Artificial Sequence: peptide
      derived from diabetes autoantigen

<220>
<221> SITE
<222> (1)..(19)

<400> 230
Lys Gln Met Glu Lys Phe Arg Lys Val Gln Thr Gln Val Arg Leu Ala
1               5                   10                  15

Lys Lys Asn



<210> 231
<211> 19
<212> PRT
<213> Artificial Sequence

<220>
<223> Description of Artificial Sequence: peptide
      derived from diabetes autoantigen

<220>
<221> SITE
<222> (1)..(19)

<400> 231
Met Glu Lys Phe Arg Lys Val Gln Thr Gln Val Arg Leu Ala Lys Lys
1               5                   10                  15

Asn Phe Asp



<210> 232
<211> 19
<212> PRT
<213> Artificial Sequence

<220>
<223> Description of Artificial Sequence: peptide
      derived from diabetes autoantigen

<220>
<221> SITE
<222> (1)..(19)

<400> 232
Leu Leu Ser His Met Leu Ala Thr Tyr Gln Thr Thr Leu Leu His Phe
1               5                   10                  15

Trp Glu Lys



<210> 233
<211> 19
<212> PRT
<213> Artificial Sequence

<220>
<223> Description of Artificial Sequence: peptide
      derived from diabetes autoantigen

<220>

P58975ep
<221> SITE
<222> (1)..(19)

<400> 233
Tyr Glu Phe Thr Thr Leu Lys Ser Leu Gln Asp Pro Met Lys Lys Leu
1               5                   10                  15

Val Glu Lys



<210> 234
<211> 19
<212> PRT
<213> Artificial Sequence

<220>
<223> Description of Artificial Sequence: peptide
      derived from diabetes autoantigen

<220>
<221> SITE
<222> (1)..(19)

<400> 234
Gln Gln Glu Ser Thr Asp Ala Ala Val Gln Glu Pro Ser Gln Leu Ile
1               5                   10                  15

Ser Leu Glu



<210> 235
<211> 19
<212> PRT
<213> Artificial Sequence

<220>
<223> Description of Artificial Sequence: peptide
      derived from diabetes autoantigen

<220>
<221> SITE
<222> (1)..(19)

<400> 235
Leu Leu Asp Gln Asn Met Lys Asp Leu Gln Ala Ser Leu Gln Glu Pro
1               5                   10                  15

Ala Lys Ala



<210> 236
<211> 19
<212> PRT
<213> Artificial Sequence

<220>
<223> Description of Artificial Sequence: peptide
      derived from diabetes autoantigen

<220>
<221> SITE
<222> (1)..(19)

<400> 236
Asn Met Lys Asp Leu Gln Ala Ser Leu Gln Glu Pro Ala Lys Ala Ala

P58975ep
1               5                   10                  15

Ser Asp Leu



<210> 237
<211> 19
<212> PRT
<213> Artificial Sequence

<220>
<223> Description of Artificial Sequence: peptide
      derived from diabetes autoantigen

<220>
<221> SITE
<222> (1)..(19)

<400> 237
Gly Ala Val Arg Thr Glu Asn Asn Ile Gln Arg His Phe Cys Thr Ser
1               5                   10                  15

Arg Ser Ile



<210> 238
<211> 19
<212> PRT
<213> Artificial Sequence

<220>
<223> Description of Artificial Sequence: peptide
      derived from diabetes autoantigen

<220>
<221> SITE
<222> (1)..(19)

<400> 238
Pro Ser Gly Arg Ala Ser Thr Arg Pro Gln His Gln Ile Gln Phe Asp
1               5                   10                  15

Glu Asp Met



<210> 239
<211> 19
<212> PRT
<213> Artificial Sequence

<220>
<223> Description of Artificial Sequence: peptide
      derived from diabetes autoantigen

<220>
<221> SITE
<222> (1)..(19)

<400> 239
Gly Arg Ala Ser Thr Arg Pro Gln His Gln Ile Gln Phe Asp Glu Asp
1               5                   10                  15

Met Asp Ser

P58975ep

<210> 240
<211> 19
<212> PRT
<213> Artificial Sequence

<220>
<223> Description of Artificial Sequence: peptide
      derived from diabetes autoantigen

<220>
<221> SITE
<222> (1)..(19)

<400> 240
Asp Lys Tyr Leu Glu Asp Phe Pro Lys Gln Gly Pro Ile Arg Leu Phe
1               5                   10                  15

Met Glu Leu



<210> 241
<211> 19
<212> PRT
<213> Artificial Sequence

<220>
<223> Description of Artificial Sequence: peptide
      derived from diabetes autoantigen

<220>
<221> SITE
<222> (1)..(19)

<400> 241
Arg Glu Ala Glu Asp Leu Gln Val Gly Gln Val Glu Leu Gly Gly Gly
1               5                   10                  15

Pro Gly Ala



<210> 242
<211> 19
<212> PRT
<213> Artificial Sequence

<220>
<223> Description of Artificial Sequence: peptide
      derived from diabetes autoantigen

<220>
<221> SITE
<222> (1)..(19)

<400> 242
Asn Cys Ser Val Ile Glu Gly His Leu Gln Ile Leu Leu Met Phe Lys
1               5                   10                  15

Thr Arg Pro



<210> 243
<211> 19
<212> PRT
<213> Artificial Sequence

P58975ep

<220>
<223> Description of Artificial Sequence: peptide
      derived from diabetes autoantigen

<220>
<221> SITE
<222> (1)..(19)

<400> 243
Lys Asn Ser Arg Arg Gln Gly Cys His Gln Tyr Val Ile His Asn Asn
1               5                   10                  15

Lys Cys Ile



<210> 244
<211> 19
<212> PRT
<213> Artificial Sequence

<220>
<223> Description of Artificial Sequence: peptide
      derived from diabetes autoantigen

<220>
<221> SITE
<222> (1)..(19)

<400> 244
Trp Ser Lys His Asn Leu Thr Thr Thr Gln Gly Lys Leu Phe Phe His
1               5                   10                  15

Tyr Asn Pro



<210> 245
<211> 19
<212> PRT
<213> Artificial Sequence

<220>
<223> Description of Artificial Sequence: peptide
      derived from diabetes autoantigen

<220>
<221> SITE
<222> (1)..(19)

<400> 245
Leu Met Arg Gly Leu Lys Pro Trp Thr Gln Tyr Ala Ile Phe Val Lys
1               5                   10                  15

Thr Leu Val



<210> 246
<211> 19
<212> PRT
<213> Artificial Sequence

<220>
<223> Description of Artificial Sequence: peptide
      derived from diabetes autoantigen

P58975ep
<220>
<221> SITE
<222> (1)..(19)

<400> 246
Pro Ile Ser Val Ser Asn Ser Ser Ser Gln Ile Ile Leu Lys Trp Lys
1               5                   10                  15

Pro Pro Ser



<210> 247
<211> 19
<212> PRT
<213> Artificial Sequence

<220>
<223> Description of Artificial Sequence: peptide
      derived from diabetes autoantigen

<220>
<221> SITE
<222> (1)..(19)

<400> 247
Glu Ser Glu Asp Ser Gln Lys His Asn Gln Ser Glu Tyr Glu Asp Ser
1               5                   10                  15

Ala Gly Glu



<210> 248
<211> 19
<212> PRT
<213> Artificial Sequence

<220>
<223> Description of Artificial Sequence: peptide
      derived from diabetes autoantigen

<220>
<221> SITE
<222> (1)..(19)

<400> 248
Glu Asn Asn Val Val His Leu Met Trp Gln Glu Pro Lys Glu Pro Asn
1               5                   10                  15

Gly Leu Ile



<210> 249
<211> 19
<212> PRT
<213> Artificial Sequence

<220>
<223> Description of Artificial Sequence: peptide
      derived from diabetes autoantigen

<220>
<221> SITE
<222> (1)..(19)

<400> 249

P58975ep
Lys Ile Thr Leu Leu Arg Glu Leu Gly Gln Gly Ser Phe Gly Met Val
1               5                   10                  15

Tyr Glu Gly

<210> 250
<211> 19
<212> PRT
<213> Artificial Sequence

<220>
<223> Description of Artificial Sequence: peptide
      derived from diabetes autoantigen

<220>
<221> SITE
<222> (1)..(19)

<400> 250
Asn Pro Gly Arg Pro Pro Pro Thr Leu Gln Glu Met Ile Gln Met Ala
1               5                   10                  15

Ala Glu Ile

<210> 251
<211> 19
<212> PRT
<213> Artificial Sequence

<220>
<223> Description of Artificial Sequence: peptide
      derived from diabetes autoantigen

<220>
<221> SITE
<222> (1)..(19)

<400> 251
Ala Ile Ala Ala Asp Ser Glu Ala Glu Gln Asp Ser Trp Tyr Gln Ala
1               5                   10                  15

Leu Leu Gln

<210> 252
<211> 19
<212> PRT
<213> Artificial Sequence

<220>
<223> Description of Artificial Sequence: peptide
      derived from diabetes autoantigen

<220>
<221> SITE
<222> (1)..(19)

<400> 252
Ser Glu Ala Glu Gln Asp Ser Trp Tyr Gln Ala Leu Leu Gln Leu His
1               5                   10                  15

Asn Arg Ala

P58975ep

<210> 253
<211> 19
<212> PRT
<213> Artificial Sequence

<220>
<223> Description of Artificial Sequence: peptide
      derived from diabetes autoantigen

<220>
<221> SITE
<222> (1)..(19)

<400> 253
Pro Gly Pro Ala Phe Lys Glu Val Trp Gln Val Ile Leu Lys Pro Lys
1               5                   10                  15

Gly Leu Gly

<210> 254
<211> 19
<212> PRT
<213> Artificial Sequence

<220>
<223> Description of Artificial Sequence: peptide
      derived from diabetes autoantigen

<220>
<221> SITE
<222> (1)..(19)

<400> 254
His His Leu Asn Asn Pro Pro Pro Ser Gln Val Gly Leu Thr Arg Arg
1               5                   10                  15

Ser Arg Thr

<210> 255
<211> 19
<212> PRT
<213> Artificial Sequence

<220>
<223> Description of Artificial Sequence: peptide
      derived from diabetes autoantigen

<220>
<221> SITE
<222> (1)..(19)

<400> 255
Thr Ile Thr His Gln Lys Thr Pro Ser Gln Ser Ser Val Ala Ser Ile
1               5                   10                  15

Glu Glu Tyr

<210> 256
<211> 19
<212> PRT

P58975ep
<213> Artificial Sequence

<220>
<223> Description of Artificial Sequence: peptide
      derived from diabetes autoantigen

<220>
<221> SITE
<222> (1)..(19)

<400> 256
Met Ser Pro Lys Ser Val Ser Ala Pro Gln Gln Ile Ile Asn Pro Ile
  1               5                  10                  15

Arg Arg His



<210> 257
<211> 19
<212> PRT
<213> Artificial Sequence

<220>
<223> Description of Artificial Sequence: peptide
      derived from diabetes autoantigen

<220>
<221> SITE
<222> (1)..(19)

<400> 257
Ser Leu Pro Arg Ser Phe Lys His Thr Gln Arg Pro Gly Glu Pro Glu
  1               5                  10                  15

Glu Gly Ala



<210> 258
<211> 19
<212> PRT
<213> Artificial Sequence

<220>
<223> Description of Artificial Sequence: peptide
      derived from diabetes autoantigen

<220>
<221> SITE
<222> (1)..(19)

<400> 258
Pro Arg Ala Arg Glu Gln Gln Gln Gln Gln Gln Pro Leu Leu His Pro
  1               5                  10                  15

Pro Glu Pro



<210> 259
<211> 19
<212> PRT
<213> Artificial Sequence

<220>
<223> Description of Artificial Sequence: peptide
      derived from diabetes autoantigen

P58975ep

<220>
<221> SITE
<222> (1)..(19)

<400> 259
Tyr Val Asn Ile Glu Phe Gly Ser Asp Gln Ser Gly Tyr Leu Ser Gly
1               5                   10                  15

Pro Val Ala



<210> 260
<211> 19
<212> PRT
<213> Artificial Sequence

<220>
<223> Description of Artificial Sequence: peptide
      derived from diabetes autoantigen

<220>
<221> SITE
<222> (1)..(19)

<400> 260
Met Thr Met Gln Met Ser Cys Pro Arg Gln Ser Tyr Val Asp Thr Ser
1               5                   10                  15

Pro Ala Ala



<210> 261
<211> 19
<212> PRT
<213> Artificial Sequence

<220>
<223> Description of Artificial Sequence: peptide
      derived from diabetes autoantigen

<220>
<221> SITE
<222> (1)..(19)

<400> 261
Ala His Ser Ser Leu Leu Gly Gly Pro Gln Gly Pro Gly Gly Met Ser
1               5                   10                  15

Ala Phe Thr



<210> 262
<211> 19
<212> PRT
<213> Artificial Sequence

<220>
<223> Description of Artificial Sequence: peptide
      derived from diabetes autoantigen

<220>
<221> SITE
<222> (1)..(19)

P58975ep
<400> 262
Asp Leu Asp Leu Val Lys Asp Phe Lys Gln Cys Pro Gln Glu Cys Thr
1               5                   10                  15

Pro Glu Pro

<210> 263
<211> 19
<212> PRT
<213> Artificial Sequence

<220>
<223> Description of Artificial Sequence: peptide
      derived from diabetes autoantigen

<220>
<221> SITE
<222> (1)..(19)

<400> 263
Arg Ala Ser Gln Asp Ser Ala Asp Pro Gln Ala Pro Ala Gln Gly Asn
1               5                   10                  15

Phe Arg Gly

<210> 264
<211> 19
<212> PRT
<213> Artificial Sequence

<220>
<223> Description of Artificial Sequence: peptide
      derived from diabetes autoantigen

<220>
<221> SITE
<222> (1)..(19)

<400> 264
Asp Ser Ala Asp Pro Gln Ala Pro Ala Gln Gly Asn Phe Arg Gly Ser
1               5                   10                  15

Trp Asp Cys

<210> 265
<211> 19
<212> PRT
<213> Artificial Sequence

<220>
<223> Description of Artificial Sequence: peptide
      derived from diabetes autoantigen

<220>
<221> SITE
<222> (1)..(19)

<400> 265
Gly Val Ser Glu Ala Ala Ser Gly Ser Gln Glu Lys Leu Asp Phe Asn
1               5                   10                  15

Arg Asn Leu

P58975ep

<210> 266
<211> 19
<212> PRT
<213> Artificial Sequence

<220>
<223> Description of Artificial Sequence: peptide
      derived from diabetes autoantigen

<220>
<221> SITE
<222> (1)..(19)

<400> 266
Met Glu Ala Lys Asp Val Lys Gly Thr Gln Glu Ser Leu Ala Glu Lys
1               5                   10                  15

Glu Leu Gln

<210> 267
<211> 19
<212> PRT
<213> Artificial Sequence

<220>
<223> Description of Artificial Sequence: peptide
      derived from diabetes autoantigen

<220>
<221> SITE
<222> (1)..(19)

<400> 267
Gln Glu Ser Leu Ala Glu Lys Glu Leu Gln Leu Leu Val Met Ile His
1               5                   10                  15

Gln Leu Ser

<210> 268
<211> 19
<212> PRT
<213> Artificial Sequence

<220>
<223> Description of Artificial Sequence: peptide
      derived from diabetes autoantigen

<220>
<221> SITE
<222> (1)..(19)

<400> 268
Asp Gln Leu Leu Thr Ala His Ser Glu Gln Lys Asn Met Ala Ala Met
1               5                   10                  15

Leu Phe Glu

<210> 269
<211> 19

P58975ep
<212> PRT
<213> Artificial Sequence

<220>
<223> Description of Artificial Sequence: peptide
      derived from diabetes autoantigen

<220>
<221> SITE
<222> (1)..(19)

<400> 269
Met Ala Ala Met Leu Phe Glu Lys Gln Gln Gln Gln Met Glu Leu Ala
1               5                   10                  15

Arg Gln Gln



<210> 270
<211> 19
<212> PRT
<213> Artificial Sequence

<220>
<223> Description of Artificial Sequence: peptide
      derived from diabetes autoantigen

<220>
<221> SITE
<222> (1)..(19)

<400> 270
Ala Met Leu Phe Glu Lys Gln Gln Gln Gln Met Glu Leu Ala Arg Gln
1               5                   10                  15

Gln Gln Glu



<210> 271
<211> 19
<212> PRT
<213> Artificial Sequence

<220>
<223> Description of Artificial Sequence: peptide
      derived from diabetes autoantigen

<220>
<221> SITE
<222> (1)..(19)

<400> 271
Gln Gln Met Glu Leu Ala Arg Gln Gln Gln Glu Gln Ile Ala Lys Gln
1               5                   10                  15

Gln Gln Gln



<210> 272
<211> 19
<212> PRT
<213> Artificial Sequence

<220>
<223> Description of Artificial Sequence: peptide

P58975ep
derived from diabetes autoantigen

<220>
<221> SITE
<222> (1)..(19)

<400> 272
Gln Gln Gln Glu Gln Ile Ala Lys Gln Gln Gln Gln Leu Ile Gln Gln
1               5                   10                  15

Gln His Lys



<210> 273
<211> 19
<212> PRT
<213> Artificial Sequence

<220>
<223> Description of Artificial Sequence: peptide
      derived from diabetes autoantigen

<220>
<221> SITE
<222> (1)..(19)

<400> 273
Gln Gln Glu Gln Ile Ala Lys Gln Gln Gln Gln Leu Ile Gln Gln Gln
1               5                   10                  15

His Lys Ile



<210> 274
<211> 19
<212> PRT
<213> Artificial Sequence

<220>
<223> Description of Artificial Sequence: peptide
      derived from diabetes autoantigen

<220>
<221> SITE
<222> (1)..(19)

<400> 274
Lys Gln Gln Gln Gln Leu Ile Gln Gln Gln His Lys Ile Asn Leu Leu
1               5                   10                  15

Gln Gln Gln



<210> 275
<211> 19
<212> PRT
<213> Artificial Sequence

<220>
<223> Description of Artificial Sequence: peptide
      derived from diabetes autoantigen

<220>
<221> SITE
<222> (1)..(19)

P58975ep

<400> 275
Gln Gln Gln His Lys Ile Asn Leu Leu Gln Gln Gln Ile Gln Gln Val
1               5                   10                  15

Asn Met Pro

<210> 276
<211> 19
<212> PRT
<213> Artificial Sequence

<220>
<223> Description of Artificial Sequence: peptide
      derived from diabetes autoantigen

<220>
<221> SITE
<222> (1)..(19)

<400> 276
Ile Asn Leu Leu Gln Gln Gln Ile Gln Gln Val Asn Met Pro Tyr Val
1               5                   10                  15

Met Ile Pro

<210> 277
<211> 19
<212> PRT
<213> Artificial Sequence

<220>
<223> Description of Artificial Sequence: peptide
      derived from diabetes autoantigen

<220>
<221> SITE
<222> (1)..(19)

<400> 277
Gln Pro Leu Pro Val Thr Pro Asp Ser Gln Leu Ala Leu Pro Ile Gln
1               5                   10                  15

Pro Ile Pro

<210> 278
<211> 19
<212> PRT
<213> Artificial Sequence

<220>
<223> Description of Artificial Sequence: peptide
      derived from diabetes autoantigen

<220>
<221> SITE
<222> (1)..(19)

<400> 278
Gly Ala Met Ala Thr His His Pro Leu Gln Glu Pro Ser Gln Pro Leu
1               5                   10                  15

P58975ep
Asn Leu Thr

<210> 279
<211> 19
<212> PRT
<213> Artificial Sequence

<220>
<223> Description of Artificial Sequence: peptide
      derived from diabetes autoantigen

<220>
<221> SITE
<222> (1)..(19)

<400> 279
Gln Glu Lys Gln Pro Tyr Tyr Glu Glu Gln Ala Arg Leu Ser Arg Gln
1               5                   10                  15

His Leu Glu

<210> 280
<211> 19
<212> PRT
<213> Artificial Sequence

<220>
<223> Description of Artificial Sequence: peptide
      derived from diabetes autoantigen

<220>
<221> SITE
<222> (1)..(19)

<400> 280
Met Arg Thr Arg Arg Gln Asp Ala Arg Gln Ser Tyr Val Ile Pro Pro
1               5                   10                  15

Gln Ala Gly

<210> 281
<211> 19
<212> PRT
<213> Artificial Sequence

<220>
<223> Description of Artificial Sequence: peptide
      derived from diabetes autoantigen

<220>
<221> SITE
<222> (1)..(19)

<400> 281
Ser Tyr Val Ile Pro Pro Gln Ala Gly Gln Val Gln Met Ser Ser Ser
1               5                   10                  15

Asp Val Leu

<210> 282

P58975ep
<211> 19
<212> PRT
<213> Artificial Sequence

<220>
<223> Description of Artificial Sequence: peptide
      derived from diabetes autoantigen

<220>
<221> SITE
<222> (1)..(19)

<400> 282
Ser Thr Ser Ala Phe Arg Ala Tyr Gly Gln Gly Thr Leu Tyr Asp Ser
  1               5                  10                  15

Pro Leu Leu



<210> 283
<211> 19
<212> PRT
<213> Artificial Sequence

<220>
<223> Description of Artificial Sequence: peptide
      derived from diabetes autoantigen

<220>
<221> SITE
<222> (1)..(19)

<400> 283
Gly Thr Leu Tyr Asp Ser Pro Leu Leu Gln Val Ser Ile His Leu Gly
  1               5                  10                  15

Tyr Gly Ile



<210> 284
<211> 19
<212> PRT
<213> Artificial Sequence

<220>
<223> Description of Artificial Sequence: peptide
      derived from diabetes autoantigen

<220>
<221> SITE
<222> (1)..(19)

<400> 284
Ile Thr Arg Ile Ala Leu Tyr Phe Val Gln Lys Gly Leu Ala Val Pro
  1               5                  10                  15

Cys Cys Phe



<210> 285
<211> 19
<212> PRT
<213> Artificial Sequence

<220>

P58975ep
<223> Description of Artificial Sequence: peptide
      derived from diabetes autoantigen

<220>
<221> SITE
<222> (1)..(19)

<400> 285
Leu Ser Leu Ser Val Leu Val Ser Leu Gln Gly Pro Leu Phe Leu Ser
  1               5                  10                  15

Tyr Leu Gly



<210> 286
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 286
Ser Ser His Gln His Ser Arg Arg Arg Gln Gly Trp Leu Lys Glu Ile
  1               5                  10                  15

Arg Lys Leu



<210> 287
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 287
Arg Gly Val Asp Phe Asn Trp Gln Ala Gln Ala Leu Leu Ala Leu Gln
  1               5                  10                  15

Glu Ala Ala



<210> 288
<211> 19
<212> PRT
<213> Artificial Sequence

<220>
<223> Description of Artificial Sequence: peptide
      derived from systemic lupus erythematosus and
      Sjogren syndrome autoantigen

P58975ep

<220>
<221> SITE
<222> (1)..(19)

<400> 288
Pro Ser Val Ala Glu Gly Tyr Ala Ser Gln Asp Val Phe Ser Ala Thr
1               5                   10                  15

Glu Thr Ser



<210> 289
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 289
Gly Lys Ser Ala Lys Pro Arg Ala Gly Gln Ala Gly Leu Pro Cys Asp
1               5                   10                  15

Tyr Thr Ala



<210> 290
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 290
Leu Asp Thr Ser Gly Leu Arg His Val Gln Leu Ala Phe Phe Pro Pro
1               5                   10                  15

Gly Thr Val



<210> 291
<211> 19
<212> PRT
<213> Artificial Sequence

<220>
<223> Description of Artificial Sequence: peptide
      derived from systemic lupus erythematosus and
      Sjogren syndrome autoantigen

<220>

P58975ep
<221> SITE
<222> (1)..(19)

<400> 291
Val Gln Gln Val Lys Gly His Tyr Arg Gln Ala Met Leu Leu Lys Ala
1               5                   10                  15

Met Ala Ala



<210> 292
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 292
Leu Lys Ala Met Ala Ala Leu Glu Gly Gln Asp Pro Ser Gly Leu Gln
1               5                   10                  15

Leu Gly Leu



<210> 293
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 293
Leu Glu Gly Gln Asp Pro Ser Gly Leu Gln Leu Gly Leu Thr Glu Ala
1               5                   10                  15

Leu His Phe



<210> 294
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

P58975ep

<400> 294
Glu Gly Leu Glu Ala Glu Asp Trp Ala Gln Gly Val Val Glu Ala Gly
1               5                   10                  15

Gly Ser Phe



<210> 295
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 295
Gly Ala Tyr Gly Ala Gln Glu Glu Ala Gln Cys Pro Thr Leu His Phe
1               5                   10                  15

Leu Glu Gly



<210> 296
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 296
Thr Ser Phe Pro Ile Asp Asp Arg Val Gln Ser His Ile Leu His Leu
1               5                   10                  15

Glu His Asp



<210> 297
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 297

P58975ep
His Val Thr Arg Lys Asn His Ala Arg Gln Ala Gly Val Arg Gly Leu
1               5                   10                  15

Gly His Gln



<210> 298
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 298
Asn Phe Ala Leu Arg Val Leu Leu Val Gln Val Asp Val Lys Asp Pro
1               5                   10                  15

Gln Gln Ala



<210> 299
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 299
Leu Val Gln Val Asp Val Lys Asp Pro Gln Gln Ala Leu Lys Glu Leu
1               5                   10                  15

Ala Lys Met



<210> 300
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 300
Tyr Leu Glu Thr Tyr Lys Ala Tyr Glu Gln Lys Pro Ala Asp Leu Leu
1               5                   10                  15

P58975ep

Met Glu Lys

<210> 301
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 301
Ala Asp Leu Leu Met Glu Lys Leu Glu Gln Asp Phe Val Ser Arg Val
  1               5                  10                  15

Thr Glu Cys

<210> 302
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 302
Val Lys Ser Val Asn Lys Thr Asp Ser Gln Thr Leu Leu Thr Thr Phe
  1               5                  10                  15

Gly Ser Leu

<210> 303
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 303
Ile Lys Arg Leu Arg Lys Lys Phe Ala Gln Lys Met Leu Arg Lys Ala
  1               5                  10                  15

Arg Arg Lys

P58975ep

<210> 304
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 304
Ser Phe Glu Pro Phe Ser Asn Lys Gly Gln Thr Leu Val Val Gln Phe
1               5                   10                  15

Thr Val Lys

<210> 305
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 305
Ser Asn Lys Gly Gln Thr Leu Val Val Gln Phe Thr Val Lys His Glu
1               5                   10                  15

Gln Asn Ile

<210> 306
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 306
Val Lys Leu Phe Pro Asn Ser Leu Asp Gln Thr Asp Met His Gly Asp
1               5                   10                  15

Ser Glu Tyr

P58975ep

<210> 307
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 307
Gly Ile Pro Ala Gly Val Pro Met Pro Gln Ala Pro Ala Gly Leu Ala
1               5                   10                  15

Gly Pro Val

<210> 308
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 308
Pro Val Arg Gly Val Gly Gly Pro Ser Gln Gln Val Met Thr Pro Gln
1               5                   10                  15

Gly Arg Gly

<210> 309
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 309
Gly Val Pro Ala Gly Val Pro Ile Pro Gln Ala Pro Ala Gly Leu Ala
1               5                   10                  15

Gly Pro Val

<210> 310

P58975ep
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 310
Pro Val Arg Gly Val Gly Gly Pro Ser Gln Gln Val Met Thr Pro Gln
1               5                   10                  15

Gly Arg Gly



<210> 311
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 311
Gly Ile Pro Ala Gly Val Pro Met Pro Gln Ala Pro Ala Gly Leu Ala
1               5                   10                  15

Gly Pro Val



<210> 312
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 312
Pro Val Arg Gly Val Gly Gly Pro Ser Gln Gln Val Met Thr Pro Gln
1               5                   10                  15

Gly Arg Gly



<210> 313
<211> 19
<212> PRT

P58975ep
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 313
Val Leu Ile Met Cys Glu Ala Cys Ser Gln Ser Pro Glu His Glu Ala
  1               5                  10                  15

His Ser Val



<210> 314
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 314
Trp Lys Ile Gln Val Glu Thr Arg Lys Gln Ser Ile Val Trp Glu Phe
  1               5                  10                  15

Glu Lys Tyr



<210> 315
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 315
Ser Ile Val Trp Glu Phe Glu Lys Tyr Gln Arg Leu Leu Glu Lys Lys
  1               5                  10                  15

Gln Pro Pro



<210> 316
<211> 19
<212> PRT
<213> Artificial Sequence

P58975ep
<220>
<223> Description of Artificial Sequence: peptide
      derived from systemic lupus erythematosus and
      Sjogren syndrome autoantigen

<220>
<221> SITE
<222> (1)..(19)

<400> 316
Glu Leu Asn His Ser Glu Leu Ile Gln Gln Ser Gln Val Leu Trp Arg
1               5                   10                  15

Met Ile Ala



<210> 317
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 317
Asn His Ser Glu Leu Ile Gln Gln Ser Gln Val Leu Trp Arg Met Ile
1               5                   10                  15

Ala Glu Leu



<210> 318
<211> 19
<212> PRT
<213> Artificial Sequence

<220>
<223> Description of Artificial Sequence: peptide
      derived from systemic lupus erythematosus and
      Sjogren syndrome autoantigen

<220>
<221> SITE
<222> (1)..(19)

<400> 318
Met Ile Ala Glu Leu Lys Glu Arg Ser Gln Arg Pro Val Arg Trp Met
1               5                   10                  15

Leu Gln Asp



<210> 319
<211> 19
<212> PRT
<213> Artificial Sequence

<220>
<223> Description of Artificial Sequence: peptide

P58975ep
derived from systemic lupus erythematosus and
Sjogren syndrome autoantigen

<220>
<221> SITE
<222> (1)..(19)

<400> 319
Pro Val Arg Trp Met Leu Gln Asp Ile Gln Glu Val Leu Asn Arg Ser
1               5                   10                  15

Lys Ser Trp



<210> 320
<211> 19
<212> PRT
<213> Artificial Sequence

<220>
<223> Description of Artificial Sequence: peptide
derived from systemic lupus erythematosus and
Sjogren syndrome autoantigen

<220>
<221> SITE
<222> (1)..(19)

<400> 320
Leu Asn Arg Ser Lys Ser Trp Ser Leu Gln Gln Pro Glu Pro Ile Ser
1               5                   10                  15

Leu Glu Leu



<210> 321
<211> 19
<212> PRT
<213> Artificial Sequence

<220>
<223> Description of Artificial Sequence: peptide
derived from miscellaneous/celiac disease
autoantigen

<220>
<221> SITE
<222> (1)..(19)

<400> 321
Thr Leu His Phe Glu Gly Arg Asn Tyr Gln Ala Ser Val Asp Ser Leu
1               5                   10                  15

Thr Phe Ser



<210> 322
<211> 19
<212> PRT
<213> Artificial Sequence

<220>
<223> Description of Artificial Sequence: peptide
derived from miscellaneous/celiac disease
autoantigen

P58975ep

<220>
<221> SITE
<222> (1)..(19)

<400> 322
Val Tyr Leu Asp Ser Glu Glu Glu Arg Gln Glu Tyr Val Leu Thr Gln
1               5                   10                  15

Gln Gly Phe



<210> 323
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

<400> 323
Glu Glu Arg Gln Glu Tyr Val Leu Thr Gln Gln Gly Phe Ile Tyr Gln
1               5                   10                  15

Gly Ser Ala



<210> 324
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

<400> 324
Glu Arg Gln Glu Tyr Val Leu Thr Gln Gln Gly Phe Ile Tyr Gln Gly
1               5                   10                  15

Ser Ala Lys



<210> 325
<211> 19
<212> PRT
<213> Artificial Sequence

<220>
<223> Description of Artificial Sequence: peptide
      derived from miscellaneous/celiac disease
      autoantigen

<220>

P58975ep
<221> SITE
<222> (1)..(19)

<400> 325
Asn Ile Pro Trp Asn Phe Gly Gln Phe Gln Asp Gly Ile Leu Asp Ile
1               5                   10                  15

Cys Leu Ile



<210> 326
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

<400> 326
Ser Gly Met Val Asn Cys Asn Asp Asp Gln Gly Val Leu Leu Gly Arg
1               5                   10                  15

Trp Asp Asn



<210> 327
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

<400> 327
His Gly Cys Gln Arg Val Lys Tyr Gly Gln Cys Trp Val Phe Ala Ala
1               5                   10                  15

Val Ala Cys



<210> 328
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

P58975ep

<400> 328
Glu Ser Trp Met Thr Arg Pro Asp Leu Gln Pro Gly Tyr Glu Gly Trp
1               5                   10                  15

Gln Ala Leu



<210> 329
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

<400> 329
Arg Lys Leu Val Ala Glu Val Ser Leu Gln Asn Pro Leu Pro Val Ala
1               5                   10                  15

Leu Glu Gly



<210> 330
<211> 19
<212> PRT
<213> Artificial Sequence

<220>
<223> Description of Artificial Sequence: peptide
      derived from miscellaneous/celiac disease
      autoantigen

<220>
<221> SITE
<222> (1)..(19)

<400> 330
Val Glu Gly Ala Gly Leu Thr Glu Glu Gln Lys Thr Val Glu Ile Pro
1               5                   10                  15

Asp Pro Val



<210> 331
<211> 9
<212> PRT
<213> Artificial Sequence

<220>
<223> Description of Artificial Sequence: gluten-derived
      peptide

<220>
<221> SITE
<222> (1)..(9)

<400> 331
Xaa Xaa Xaa Gln Xaa Pro Gln Xaa Tyr

P58975ep
1               5

<210> 332
<211> 14
<212> PRT
<213> Artificial Sequence

<220>
<223> Description of Artificial Sequence: gluten-derived
      peptide

<220>
<221> SITE
<222> (1)..(14)

<400> 332
Gln Gln Pro Phe Pro Gln Gln Pro Gln Gln Pro Tyr Pro Gln
1               5                   10

<210> 333
<211> 14
<212> PRT
<213> Artificial Sequence

<220>
<223> Description of Artificial Sequence: gluten-derived
      peptide

<220>
<221> SITE
<222> (1)..(14)

<400> 333
Gln Val Pro Gln Pro Gln Gln Pro Gln Gln Pro Phe Leu Gln
1               5                   10

<210> 334
<211> 14
<212> PRT
<213> Artificial Sequence

<220>
<223> Description of Artificial Sequence: gluten-derived
      peptide

<220>
<221> SITE
<222> (1)..(14)

<400> 334
Arg Gln Pro Phe Pro Gln Gln Pro Gln Gln Pro Tyr Pro Gln
1               5                   10

<210> 335
<211> 14
<212> PRT
<213> Artificial Sequence

<220>
<223> Description of Artificial Sequence: gluten-derived
      peptide

<220>
<221> SITE

P58975ep
<222> (1)..(14)

<400> 335
Gln Gln Thr Phe Pro Gln Gln Pro Gln Leu Pro Phe Pro Gln
1               5                   10


<210> 336
<211> 14
<212> PRT
<213> Artificial Sequence

<220>
<223> Description of Artificial Sequence: gluten-derived
      peptide

<220>
<221> SITE
<222> (1)..(14)

<400> 336
Gln Gln Thr Tyr Pro Gln Arg Pro Gln Gln Pro Phe Pro Gln
1               5                   10


<210> 337
<211> 14
<212> PRT
<213> Artificial Sequence

<220>
<223> Description of Artificial Sequence: gluten-derived
      peptide

<220>
<221> SITE
<222> (1)..(14)

<400> 337
Gln Gln Pro Ile Gln Gln Gln Pro Gln Pro Phe Pro Gln Gln
1               5                   10

## Description

The invention relates to the field of molecular biology and immunology, more specific to immune diseases, especially celiac disease. It further relates to neo-epitopes that can be generated by the enzyme tissue transglutaminase (tTG) which neo-epitopes can play a role in celiac disease and in autoimmune diseases.

### Introduction

Celiac disease (CD) is a permanent intolerance to gluten (Marsh, M.N., Gastroent. 102: 330-354, 1992). Gluten is a complex mixture of proteins found in wheat and several other grains, and is present in many food products. Typical disease symptoms include chronic diarrhoea, fatigue, and failure to thrive. These symptoms are associated with a lesion in the upper small intestine, characterised by a (sub) total villous atrophy, an increased number of intra epithelial lymphocytes, and a chronic inflammatory response of the lamina propria lymphocytes (Marsh, 1992). Diagnosis is based on morphology of a small intestinal biopsy and improvement of the clinical status after a gluten free diet. A strong indication for CD is the presence of antibodies specific for the enzyme tissue transglutaminase (tTG) in patients (Dieterich, W. *et al*., Nat. Med. 3: 797-801, 1997). Moreover, recent studies demonstrated the involvement of this enzyme in the generation of T cell stimulatory peptides (Molberg, O. *et al*., Nat. Med. 4: 713-717, 1998; van de Wal, Y. *et al*., J. Immunol. 161: 1585-1588, 1998a). CD is strongly associated with HLA-DQ2 (Al*0501, B1*0201) and HLA-DQ8 (A1*0301, B1*0302) (Spurkland A. *et al.*, Tissue Antigens 49: 29-34, 1997). In addition, HLA-DQ2 and/or HLA-DQ8 restricted, gluten specific CD4⁺ T lymphocytes can be isolated from small intestinal biopsies of CD patients, and these are thought to cause disease (Lundin, K. *et al.*, J. Exp. Med. 178: 187-196, 1993; van de Wal, Y. *et al.* Proc. Natl. Acad. Sci. USA 95: 10050-10054, 1998b). The peptide binding motif of HLA-DQ2 and -DQ8 predicts a preference for negative charges at anchor positions in the bound peptides (Johansen, B.H. *et al.*, Int. Immunol. 8: 177-182, 1996; van de Wal, *Y. et al.* Immunogenetics 44: 246-253, 1996; Kwok, W.W. *et al.*, J. Immunol. 156: 2171-2177, 1996). Gluten molecules, however, contain few negative charges. This discrepancy was solved by the finding that tTG can convert glutamine residues into glutamic acid (a process termed deamidation), and thus introduces negative charges in gluten peptides. Indeed it was found that tTG is required for, or enhances the gluten specific response of T cell clones and T cell lines derived from the majority of CD patients (van de Wal, 1998a; Molberg, 1998; Arentz-Hansen, H. *et al.*, J. Exp. Med. 191: 603-612, 2000; Anderson, R.P. *et al.*, Nat. Med. 6: 337-342, 2000). Considering the abundance of glutamine residues in gluten (∼30-40%) tTG has many potential target sites in gluten. Mass spectral analysis of the deamidated T cell stimulatory gluten peptides, however, showed a highly restricted pattern of deamidation (van de Wal, 1998a; Molberg, 1998). Usually the pattern of deamidation coincides with the positions where negative charges are preferred in the HLA binding motif, thus favouring the binding of gluten peptides to HLA-DQ2 and/or -DQ8. The specificity of gluten deamidation by tTG, therefore, is a crucial factor in the generation of toxic gluten peptides. It is not known, however, why only particular glutamine residues are targeted by tTG.

Current therapy of CD mainly involves dietary treatment of glutensensitive patients with diets lacking cereal compounds such as flour, which deprives these patients of such typical staple foods as for instance bread. The criteria for glutenfree products are established by the Codex Alimentarius Committe "Nutrition and Food for Special Dietary Uses" that meets every 1.5 year. The current criterion is based on the determination of nitrogen. To be considered glutenfree a product may contain maximally 50 mg N per 100 gram of product. The determination is only useful when there is a certain relationship between the amount of nitrogen and the amount of gluten. This is only true, to a certain extent, for wheat. There is still a need for a more reliable method to detect whether food materials would cause problems in CD patients.

For auto-immune diseases in general, it is not known which combination of factors gives rise to an immunological response. One of the hypotheses is that presumed antigenic compounds are processed in the human body and give rise to so-called neo-epitopes. These neo-epitopes are then recognised by the immune system and disease occurs. It is, however, not yet clear in which way these neo-epitopes can be formed.

### Summary of the invention

The invention now provides a peptide sequence of 14-40 amino acids, which is prone to deamidation by tTG and which is a causative factor of celiac disease, which comprises an amino acid sequence selected from the group consisting essentially of QQPYPQQPQQPFPQ, QQPFPQQPQQPFPQ, PFPQTQQPQQPFPQ, PFPQLQQPQQPFPQ, QFPQTQQPQQPFPQ, QLPFPQQPQQPFPQ, PFPQPQQPQQPFPQ and PFPQSQQPQQPFPQ. Also, the invention provides for epitopes made by deamidation of these peptides, thus resulting in the sequences QQPYPEQPQQPFPQ, QQPFPEQPQQPFPQ, PFPQTEQPQQPFPQ, PFPQLEQPQQPFPQ, QFPQTEQPQQPFPQ, QLPFPEQPQQPFPQ, PFPQPEQPQQPFPQ, PFPQSEQPQQPFPQ, QQPYPEQPEQPFPQ, QQPFPEQPEQPFPQ, PFPQTEQPEQPFPQ, PFPQLEQPEQPFPQ, QFPQTEQPEQPFPQ, QLPFPEQPEQPFPQ, PFPQPEQPEQPFPQ, PFPQSEQPEQPFPQ, QQPYPQQPEQPFPQ, QQPFPQQPEQPFPQ, PFPQTQQPEQPFPQ, PFPQLQQPEQPFPQ, QFPQTQQPEQPFPQ, QLPFPQQPEQPFPQ, PFPQPQQPEQPFPQ and PFPQSQQPEQPFPQ, wherein further Q residues may be deamidated into an E residue.

The invention also provides for peptide sequences, which are prone to deamidation by tTG and which are a causative factor of an autoimmune disease selected from the group consisting essentially of RA (rheumatoid arthritis), MS (multiple sclerosis), SLE (systemic lupus erythomatosus), SS (Sjogren syndrome) and DB (diabetes), which peptides are shown in table 5.

Preferably these peptide sequence are residing in sequences 8 - 40 amino acid residues which comprise at least one 8-mer which is a subsequence of at least one of the sequences listed in claim 3, wherein said 8-mer comprises the central Q residue of the sequence listed in table 5. The invention also harbours the epitope formed by deamidation of one or more of the Q residues of these peptides.

A further part of the invention is an isolated HLA-DQ restricted T-cell capable of recognising a gluten-derived epitope according to the invention and more specifically an isolated or recombinant HLA-DQ restricted T-cell receptor capable of recognising such an epitope.

Also encompassed are antibodies reactive with a gluten-derived peptide or epitope according to the invention.

Further disclosed in the invention is a method to screen foodstuffs or plant material for the occurrence of these peptides or epitopes using such a T cell, a T cell receptor or antibody. Preferably in such a method the foodstuff or plant material is pretreated with a protease such as trypsin, chymotrypsin or pepsin. Most preferably in such a method the foodstuff or plant material is additionally treated with tTG.

Furthermore part of the invention is a diagnostic kit comprising: a T cell, a T cell or an antibody as mentioned above and a suitable means of detection especially for detecting in food, food components, plant material or biological samples the presence of a gluten-derived peptide or epitope.

Further part of the invention is an oligonucleotide primer set suitable for use in an amplification assay which is capable of binding to a nucleotide sequence coding for a gluten derived peptide or epitope or a DNA sequence which codes for a peptide which matches a consensus sequence according to the following formula (1), (2), (3), (4) or (5):
(1) X₁ - X₂ - X₃ - Q₄ - X₅ - P₆ - Q₇ - X₈ - P₉ - X₁₀ in which X₁₀ is F, Y, M, W, I or L and X₁, X₂, X₃, X₅, and X₈ are any amino acid;
(2) X₁ - X₂ - X₃ - Q₄ - X₅ - P₆ - Q₇ - X₈ - X₉ - X₁₀ in which X₉ is F, Y, M, W, I or L and X₁, X₂, X₃, X₅, X₈ and X₁₀ are any amino acid;
(3) Q - Xi - P in which X₁ is any amino acid;
(4) Q - X₁ - P - X₂ in which X₁ is any amino acid and X₂ is F,Y,W,M,L,I, or V;
(5) Q - Xi - X₂ - X₃ in which X₁ and X₂ are any amino acid and X₃ is F,Y,W,M,L,I, or V.

Said primer set is preferentially used in a nucleotide amplification assay wherein food, food components, plant material or biological samples are tested for the presence of proteins or peptides which match any of the consensus sequences.

The hereinbefore mentioned detection methods may be used for the detection of cereals which are better suited to be used in food for celiac disease patients. It is also part of the invention to produce cereals which are better suited to be used in food for celiac disease patients by selecting cereals having a low amount of proteins or peptides matching the consensus sequences and crossbreeding them to produce cereals with even lower amounts.

Alternatively, the cereals can be produced by using genetic engineering techniques to diminish the natural amount of proteins or peptides matching the consensus sequences. Preferably the genetic engineering comprises transformation with recombinant DNA, most preferably homologous recombination or gene silencing, or induced mutation.

The preferred cereal is wheat.

Also part of the invention are cereal plants thus obtained and the use of such a cereal to prepare food for celiac disease patients.

Another part of the invention is a method to identify neo-epitopes from auto-antigens comprising comparing the sequence of an auto-antigen and searching for peptide sequences which match with the consensus sequences as defined before and subsequently screen biological material obtained from auto-immune disease patients for antibodies which bind to the peptide sequences or T cells which bind to the HLA-bound peptide sequences as identified in the previous step or peptides which are formed from said peptide sequences by deamidation of one or more Q residues.

Further, the invention comprises a method to inhibit the binding of a T cell receptor to an HLA-bound gluten-derived epitope or to an HLA-bound auto-antigen derived epitope comprising providing a blocking substance, preferably by blocking the binding of said epitopes to the HLA molecules.

Also comprised in the invention are analogue of the epitopes of the invention which are an antagonist for the activity of T cells recognising said epitope.

A further part of the invention is a pharmaceutical composition comprising a peptide or an epitope according to the invention, which may preferably be used for the induction of tolerance or for the treament of celiac disease or auto-immune disease.

A next part of the invention is a method to decrease sensitivity to auto-antigens by inhibiting the deamidation of said auto-antigen, preferably by inhibiting the function of tTG and most preferably at the site of presence of the auto-antigen.

Also part of the invention is the use of a protease inhibitor for preventing the generation of a peptide according to the invention or a polypeptide comprising such a peptide. For the similar purpose acid neutralising substances can be used. Further, the invention provides for a method to reduce the production of a auto-antigen derived peptide by inhibiting the expression of the auto-antigen from which it is derived.

### Legend to the figures

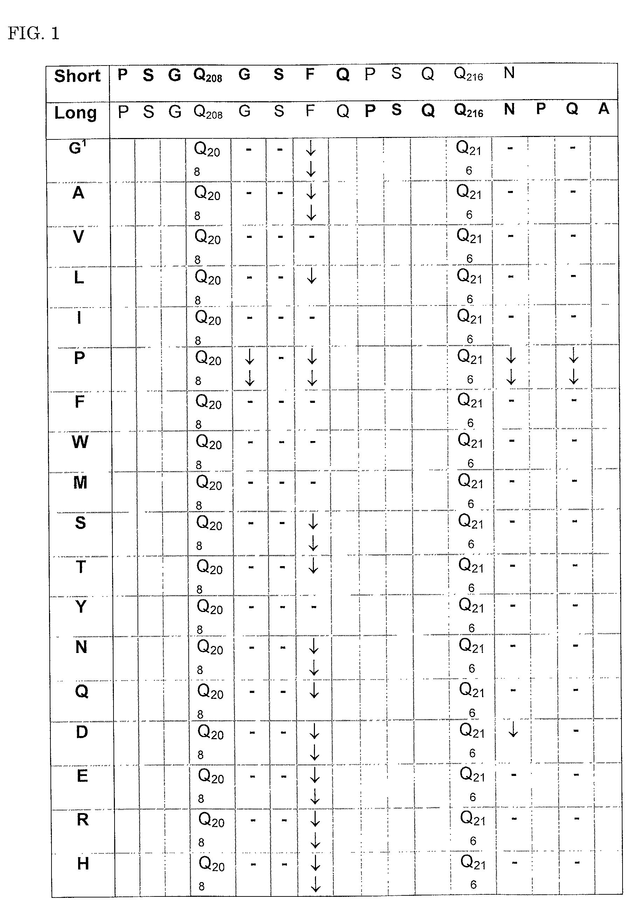

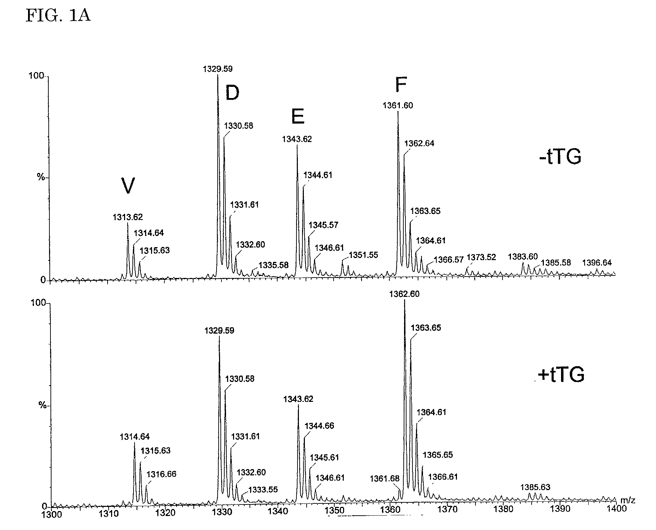

### Figure 1. The influence of amino acid substitutions on deamidation of Q₂₀₈ and Q₂₁₆.

A. Simultaneous mass spectral analysis of four substitution analogs of the gliadin peptide before and after treatment with tTG as indicated. The untreated substitution analogs with a V, D, E, or F at position Q₂₀₈+3 have a mass of 1313, 1329, 1343 and 1361 Da respectively. After treatment with tTG two of those masses increase 1 Da, 1313 to 1314 and 1361 to 1362, indicating the conversion of a glutamine residue (128 Da) to a glutamic acid (129 Da) in the peptides with a V and F at position Q₂₀₈+3.

B. Overview of the influence of C-terminal amino acid substitutions on deamidation of Q₂₀₈ in the short version of the gliadin peptide and Q₂₁₆ in the long version of the peptide. The amino acids G₂₀₉, S₂₁₀, F₂₁₁, N₂₁₇, and Q₂₁₉ were substituted for all amino acids except lysine and cysteine and the effect on deamidation was determined. Substitution at other position in the peptide had only minor effects on deamidation of the Q₂₀₈- and Q₂₁₆-residues (not shown). (-) indicates no effect of the substitution; Substitutions that resulted in major differences in deamidation are designated with arrows: ↓↓ decrease of 70-100%, ↓ decrease of 30-69%.

¹ Substitution analogs were made in which the amino acids indicated were replaced by every natural amino acid except cysteine and lysine, because these residues could affect deamidation by formation of disulphide bridges and tTG driven cross-linking, respectively.

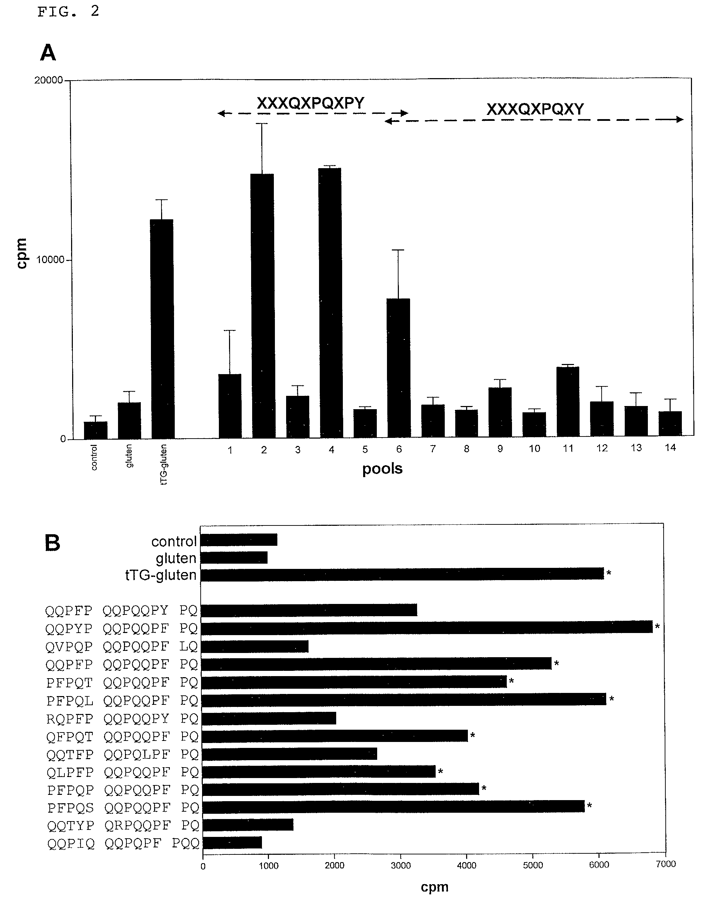

### Figure 2. Stimulation of gluten specific T cell lines by predicted peptides

A. A gluten specific T cell line isolated from a small intestinal biopsy of a celiac disease patient was tested against gluten, tTG-treated gluten and against the predicted peptides that were present in pools of five peptides each (pools 1-14). The odd numbers represent pools that contain the native peptides, whereas the even numbers represent corresponding pools that were treated with tTG. Strong reactivity of the T cell line was only observed with tTG-treated gluten and tTG-treated pools of peptides predicted by the XXXQXPQXPY algorithm.

B. Reactivity of the T cell line against the individual peptides from the T cell stimulatory pools. Bars indicated with an asterisk are considered positive (Stimulation Index > 3).

### Detailed description

In celiac disease both T-cells reactive with (epitopes derived from) gluten and antibodies reactive with the enzyme tTG (tissue transglutaminase) have been found. It is hypothesized that the epitopes from gluten are the causative factor of the disease. The antibodies specific for the endogenous tTg can be explained because the enzyme crosslinks with gluten proteins or parts of those, which complex then will be recognized by a B-cell expressing tTG specific antibodies on its cell surface. The complex, including the gluten will then be processed by the B-cell to present the antigenic determinants to gluten specific T-cells, thereby delivering specific T-cell help, resulting in the secretion of tTG-specific antibodies.

We have now determined the patterns of deamidation in gluten, and find that this is highly dependent on the spacing between the glutamine and proline residues. This knowledge can be used to predict novel T cell stimulatory gluten peptides. Moreover, our results provide an explanation for the toxicity of gluten and the related hordeins in barley and the secalins in rye, while other food proteins, in particular the avenins in oats, are not toxic for CD patients. Also, the finding can explain the formation of neo-epitopes in autoimmune diseases such as RA (rheumatoid arthritis), MS (multiple sclerosis), SLE (systemic lupus erythomatosus), SS (Sjogren syndrome) and DB (diabetes).

It has been known in the prior art that the tTG enzyme affects the deamidation of a glutamine (Gln; IUPAC code: Q) moiety on the target peptide into a glutamic acid moiety (Glu; IUPAC code: E). Gluten proteins, such as gliadin, glutelin, hordein and secalin, however, contain very many Q residues (in gliadin approximately 30-40% of all amino acid residues) and it has been shown that not all Q residues are susceptible to deamidation by tTG in the same manner. Thus, it is clear that the near environment of the Q residue on the target protein (be it in the primary, secondary or tertiary structure of the protein) influences the availability of the Q residue to act as a substrate for the enzyme. It has been shown earlier (van de Wal, 1998a) that in one of the gliadin peptide parts which is able to stimulate T-cells (PSGQ₂₀₈GSFQPSQQ₂₁₆NPQA) the Q residues at position 208 and 216 are deamidated, while the Q residues at positions 212, 215 and 219 are less or not amidated. From detailed studies as are shown in the experimental part it has now been established that the presence of proline (Pro, IUPAC code P) in the neighbourhood of the Q residue is a key determining factor of the susceptibility of said residue to deamidation by tTG. In the sequences QP and QXXP (X is any amino acid) no or very little deamidation of the Q-residue was observed. In contrast, in the sequences QXP, QXXF(Y, W, L, I, M or V) and QXPF(Y,W, L, I, M, or V) efficient deamidation of the Q-residue was observed. Also, a minimum of three amino acids C-terminal of the target Q-residue is required for deamidation. Based on this finding two consensus sequences have been developed which have a very high predictive value for susceptibility to tTG catalysed deamidation.

On the basis of these results two search algorithms were defined, specifically aimed to aid in the search for HLA-DQ2-binding peptides in the gluten databases that would be a potential target for gluten specific T cells isolated from celiac disease patients. In these algorithms the deamidation of Q redidues at position p7 and/ p4 is predicted to mediate bindign to HLA-DQ2. The first algorithm predicts deamidation at p4 and p7 and reads as follows:
(1) X₁ - X₂ - X₃ - Q₄ - X₅ - P₆ - Q₇ - X₈ - P₉ - X₁₀ in which X₁₀ is F, Y, M, W, I or L and X₁, X₂, X₃, X₅, and X₈ are any amino acid.
   The second algorithm predicts deamidation at p4 and reads as follows:
(2) X₁ - X₂ - X₃ - Q₄ - X₅ - P₆ - Q₇ - X₈ - X₉ - X₁₀ in which X₉ is F, Y, M, W, I or L and X₁, X₂, X₃, X₅, X₈ and X₁₀ are any amino acid.

On basis of these algorithms (also called herein consensus sequences) novel peptide parts of gluten proteins have been discovered, which are able to act as substrate for tTg and which, upon deamidation, have antigenic activity as measured by their ability to stimulate gluten specific T cell lines. From hereon these novel peptides will be called gluten-derived peptides. These peptides have the amino acid sequences: QQPYPQQPQQPFPQ, QQPFPQQPQQPFPQ, PFPQTQQPQQPFPQ, PFPQLQQPQQPFPQ, QFPQTQQPQQPFPQ, QLPFPQQPQQPFPQ, PFPQPQQPQQPFPQ and PFPQSQQPQQPFPQ.

It should be clear that also C-terminally and N-terminally extended peptides comprising the herebefore mentioned sequences are part of the invention. Thus, the invention comprises amino acid sequences of 14-40 amino acids which comprise the above mentioned sequences.

Also part of the invention are the epitopes derived from these gluten-derived peptides by deamidation of one or more Q residues. These gluten-derived epitopes thus have the amino acid sequences: QQPYPEQPQQPFPQ, QQPFPEQPQQPFPQ, PFPQTEQPQQPFPQ, PFPQLEQPQQPFPQ, QFPQTEQPQQPFPQ, QLPFPEQPQQPFPQ, PFPQPEQPQQPFPQ, PFPQSEQPQQPFPQ, QQPYPEQPEQPFPQ, QQPFPEQPEQPFPQ, PFPQTEQPEQPFPQ, PFPQLEQPEQPFPQ, QFPQTEQPEQPFPQ, QLPFPEQPEQPFPQ, PFPQPEQPEQPFPQ, PFPQSEQPEQPFPQ, QQPYPQQPEQPFPQ, QQPFPQQPEQPFPQ, PFPQTQQPEQPFPQ, PFPQLQQPEQPFPQ, QFPQTQQPEQPFPQ, QLPFPQQPEQPFPQ, PFPQPQQPEQPFPQ and PFPQSQQPEQPFPQ, wherein further Q residues may be deaminated into an E residue.

Furthermore, it is known from the prior art that neo-epitopes derived from presumed antigenic auto-immunogens can be a mechanism to explain the occurence of major auto-immune diseases such as RA (rheumatoid arthritis), MS (multiple sclerosis),

SLE (systemic lupus erythomatosus), SS (Sjogren syndrome) and DB (diabetes). Thus far, it has not been recognised which factor is responsible for the existence of these neo-epitopes. Surprisingly now, when the amino acid sequences of the antigens which are deemed to play a role in the above mentioned auto-immune diseases are screened with the following consensus sequence(s): QX₁P, QX₁PX₂ or QX₁X₁X₂ (wherein X₁ is any amino acid and X₂ is F, Y, W, M, L, I or V) or the sequences as described above in (1) or (2) in all antigens listed in Table 5 peptide sequences are found which are prone to deamidation by tTG. It is now postulated that in these diseases tTG is able to recognise the antigens or processed antigens and that the peptide part which is recognised has been deamidated and will function as a neo-epitope which will evoke a T-cell response.

Table 5 shows the antigens and the amino acid sequences comprised in these antigens which are thought to be involved in the above mentioned auto-immune diseases. Thus, a further aspect of the invention comprises the auto-antigen derived peptides listed in Table 5, including the peptides in which the Q residue is replaced by an E at position 10 (the central Q residue).

To a person skilled in the art it is known that peptides of variable length can bind to HLA-molecules. The relevant peptides can therefore be longer or shorter versions as those shown and might even extend to N-terminally and/or C-terminally extended sequences that are not shown but present in the proteins from which these peptide sequences are derived. Thus, the invention comprises amino acid sequences of 8 amino acid residues (8-mers) derivable from and comprising the central Q residue of those sequences as listed in Table 5. For example then (taking the first sequence of Table 5: SRFSWGAEG**Q**RPGFGYGGR) the peptides of the invention would include the following 8-mers: FSWGAEG**Q**, SWGAEG**Q**R, WGAEG**Q**RP, GAEG**Q**RPG, AEG**Q**RPGF, EG**Q**RPGFG, G**Q**RPGFGY and **Q**RPGFGYG. Further comprised in the invention are all peptides of 40 amino acid residues or less than 40, which comprise one or more of the above mentioned 8-mers. For ease of reference these peptides further are denominated as auto-antigen derived peptides.

Analagous to the situation with the gluten-derived peptides, also here in case of the auto-antigen derived peptides, neo-epitopes can be defined in which the central Q residue in the peptides listed in Table 5 has been deamidated into an E residue.

Since these consensus sequences now make it possible for the first time to predict amino acid sequences which upon deamidation by tTG act as epitopes in celiac disease or other auto-immune diseases, the information can also be used in various assays and therapies relating to these diseases.

The most promising application in the area of celiac disease is to screen foodstuffs for the presence of sequences matching with the consensus sequence(s).

A first embodiment for such a screening approach which is included in the invention is the use of (HLA-DQ2 and HLA-DQ8) restricted T cells which recognise the gluten-derived epitopes, or the receptor of such T cells. Such T cells are obtainable by methods well known to those skilled in the art. A preferred way to obtain these T cells is to collect T cell biopsies from celiac disease patients, which show typical clinical symptoms and/or are responsive in an anti-endomysium test. These biopsies then are cultured with either a (chymo)trypsin/pepsin digest of gluten optionally additionally treated with tTG. Cultures that show evidence of T cell proliferation are expanded and tested for specificity to the gluten-derived epitopes of the invention. From these gluten specific T cells optionally clones can be generated and the T cell receptor of such T cell clone can be cloned and transfected or transduced into other appropriate cells or cell lines to yield a functional T cell receptor.

These T cells or T cell receptors can be used to screen foodstuffs for the presence of epitopes. They can also be used to screen plant material, especially varieties of cereals such as wheat, to check the occurrence of said epitopes. Preferably in these types of assays the protein material in the food or in the plant varieties is treated with a protease such as trypsin, chymotrypsin or pepsin and further treated with the enzyme tTG to mimic the protein degradation in the human body.

Preferably such a screening is performed with a panel of T cells having specific reactivity to different epitopes. In this way the safety of food for celiac disease patients can be tested reliably. Also in this way it is possible to detect varieties of cereals which would be suitable for incorporation into food products for celiac disease patients.

Next to the use of specific T cells another embodiment comprised in the invention for assaying the safety of foodstuffs or the suitability of cereal varieties for food processing is to use antibodies which are capable to bind the gluten-derived peptides or gluten-derived epitopes of the invention. Such antibodies are for instance obtainable by immunising an immunocompetent animal with the gluten-derived peptides or gluten-derived epitopes according to the invention or an immunogenic fragment thereof, coupled to a suitable carrier protein if necessary, and harvesting polyclonal antibodies from said animal, or obtainable by other methods known in the art such as by producing monoclonal antibodies, or (single chain) antibodies or binding proteins expressed from recombinant nucleic acids derived from a nucleic acid library, for example obtainable though phage display techniques.

With such an antibody, the invention also provides for an immunoassay comprising an antibody according to the invention. Various types of immunoassays are available within the art, for example ELISA (Enzyme Linked Immuno Sorbent Assay) or Western blotting.

Analogous to assays with specific T cells as discussed above, also in the case of antibodies preferably a panel of antibodies is used.

A further example for assaying the safety of food and to identify cereal varieties that lack the predicted T-cell stimulatory sequences is the use of nucleotide primer based amplification technology. In such a method oligonucleotide primers are developed corresponding with DNA coding for the specific gluten-derived peptides or the general amino acid consensus sequence(s). These primers then are brought into contact with DNA or RNA isolated from the food or the plants and the mixture is subjected to a treatment to amplify the nucleotide sequences to which the primers bind. These DNA or RNA amplification techniques, such as PCR and NASBA, are known and readily available to a person skilled in the art. The amplification step will result in a specific multiplication of the nucleotide fragment to which the primers bind and, in the present case, thus is indicative of the occurrence of even a single copy of the nucleotide coding for a gluten-derived peptide or a peptide which matches the consensus sequence.

It is acknowledged that to develop an assay testing for the safety of food for celiac disease patients more than one T cell clone, more than one set of primers or more than one antibody is needed. In yet another embodiment the invention thus comprises assays having an array of antbodies as discussed above, all recognising different sequences, which fall within the consensus sequence(s) of the invention. Similarly, the invention also provides for a multiple of oligonucleotide primer sets which are able to bind with different nucleic acids coding for amino acid sequences which fall within the consensus sequence(s) of the invention.

In yet another embodiment the invention provides a diagnostic kit comprising an antibody according to the invention or a set of oligonucleotide primers according to the invention and a suitable means of detection. Such a diagnostic kit is, for example, very useful for detecting in food, food components or samples from (suspected) patients the presence of a gluten-derived peptide according to the invention or a peptide sequence involved in food-related immune enteropathy (for example: celiac sprue, tropical sprue, giardiasis or food allergies of childhood) matching the consensus sequence(s) of the invention. At present such a quantitative and qualitative diagnostic kit determining the presence and/or amount of gluten-derived peptide is not available. Currently two different assays are used for gluten detection. One assay determines the nitrogen content of food (components) as a measure for the presence of gluten. The other assay utilises gluten specific antibodies in ELISA systems. However, both assay systems do not test for the toxic gluten-derived peptides involved in food-related immune enteropathy. A diagnostic kit comprises, for example, an antibody according to the invention specifically recognising a toxic gluten-derived peptide involved in food-related immune enteropathy. Another advantage of the diagnostic kit as described in the present application is the capability of testing food (components) which cannot be tested or cannot be tested reliably by the currently used gluten assays. The existing assays are hardly informative when food (components) contain significant amounts of other nitrogen containing compounds (e.g. other proteins) or when food (components) contain partially hydrolysed gluten proteins that are not recognised by antibodies currently used in ELISA-kits. Examples of food (components) for which the existing assays are troublesome are beer, melassis and soy sauce. In addition, the existing assays lack the level of sensitivity required for many applications.

Preferably a diagnostic kit according to the invention uses different kinds of T cells, antibodies or oligonucleotide primer sets according to the invention or different antibodies, each capable of recognizing another gluten-derived peptide involved in food-related immune enteropathy. Thereby multiple gluten-derived peptides involved in food-related immune enteropathy are detected. In the art different kinds of means of detection are available and the skilled person knows how to select a proper means of detection. Examples are chromogenic or fluorigenic substances. The invention thus provides methods and means for the monitoring of a T-cell reactive component in food, food component or samples from (suspected) patients.

Another aspect of the invention is the use of the consensus sequence(s) of the invention to search for peptide sequences which could invoke immune responses. One of the possible ways to achieve this is to screen existing protein or nucleotide databases and to identify amino acid sequences or nucleotide sequences coding for amino acid sequences which match with the consensus sequence of the invention. Methods for identification of matching sequences in databases, such as the BLASTN computer program (which is publicly available, for instance through the National Centre for Biotechnological Information, accessible via the internet on http://www.ncbi.nlm.nih.gov/) are well known to persons skilled in the art. Furthermore the invention provides a method to select and/or breed a cereal which has a decreased amount of or is substantially free of peptide sequences which are toxic for celiac disease patients. Cereals are selected for the absence of peptide sequences matching the consensus sequence(s) of the invention according to any of the above described methods. Such selected cereals are than included in breeding schemes using normal agricultural breeding methods to develop cereal varieties useful for food production which exhibit a better tolerance to celiac disease patients. Cereal, in this application, relates to grain or related grasses or plants that produce it and to the (prepared) foodstuff. In particular wheat gluten, but also rye, and to a lesser extent barley and oat are preferred cereals.

Because the consensus sequence(s) for determining peptides which cause immunological effects are disclosed herein, one is now also able to genetically modify the genome of cereals to generate new cereals with a decreased source of toxic peptides. Modifications are, for example, generated by point-mutations in the nucleic acid sequence coding for the gluten proteins or are generated by replacing, e.g. through homologous recombination, a sequence coding for an amino acid sequence which matches the consensus sequence(s) of the invention by another sequence not giving rise to amino acid sequences matching the consensus sequence(s). Further, expression of a protein which contains toxic peptide sequences may be prohibited by gene silencing. Numerous methods for gene silencing in plants, such as antisense expression, sense co-suppression and RNAi are known in the art and readily applicable by a person skilled in the art.

Since, according to this invention, the presence of proline residues determine for a large part the susceptibility of a peptide for deamidation by tTG, it is preferred to change the nucleotide sequences coding for the amino acid sequences in such a way that the proline residues of peptide fragments which match the consensus sequence are changed into other residues. Such conservative substitutions of the proline residues are possible without substantially affecting the chemical and physical properties that are so desired in gluten. Cereal plants, thus genetically modified, can again be used in cross-breeding programmes in order to come to a variety which can be used in agriculture.

A cereal selected and/or bred according to a method of the invention is used to prepare food low or preferably free of toxic peptides involved in food-related immune enteropathy.

Further, the invention also comprises methods to identify possible toxic peptides from auto-antigens. According to any of the above-mentioned methods peptide sequences from antigens which are deemed to play a role in auto-immune diseases can be detected. Table 5 lists a number of predicted toxic peptide sequences comprised in proteins which are believed to play a role in diseases such as diabetes, rheumatoid arthritis, multiple sclerosis, systemic lupus erythomatosus, Sjogren syndrome and the like. It is now possible, using the information from the consensus sequence(s) of the invention to identify toxic peptides from yet other auto-antigens. Neo-epitopes can be formed from these peptide sequences by deamidation with tTG. It is now possible for a person skilled in the art to investigate whether these neo-epitopes indeed play a role in auto-immune diseases. A simple way to do this is to screen serum, lymph or any other biological material from auto-immune disease patients for the presence of T cells and/or antibodies against such neo-epitopes (which do not, or to a lesser extent, occur in non-autoimmune diseased control patients).

This would further allow for a diagnostic method for assessing disease or predisposition to disease on basis of the occurrence of said antibodies.

Furthermore the invention provides a method to decrease or more preferably completely inhibit the binding of a T-cell receptor to a gluten-derived epitope or neo-epitope involved in auto-immune disease comprising providing a blocking substance of said T-cell receptor. By decreasing and more preferably completely inhibiting the binding of the T-cell receptor to an HLA-bound gluten-derived epitope or neo-epitope, effects of the immune disease are decreased or preferably completely diminished.

Such a blocking substance prevents the association of the T-cell receptor with the HLA-bound gluten peptide and prevents the T-cell receptor in its activity. For example such a blocking substance is a natural or synthetic variant of a gluten-derived epitope or neo-epitope according to the invention, for example an altered peptide ligand, and is especially well suited for use in a therapy against gluten-derived peptide or auto-antigen derived peptide sensitivity. It is clear that the binding of said blocking substance to a T-cell receptor does not allow functional signalling of a T-cell comprising said T-cell receptor.

Furthermore gluten-derived peptides or auto-antigen derived peptides according to the invention are used to prepare therapeutic agents capable of eliminating a subset of cells, directly or indirectly, especially gluten-sensitive or auto-antigen sensitive T-cells. This means that an agent, which typically comprises a gluten-derived peptide or an auto-antigen derived peptide according to the invention as recognised selectively by T-cells, which agent induces elimination of the cells recognising said peptide, is administered to the patient. Such an agent most typically also comprises a toxic moiety to mediate the elimination of the specific T cells.

In yet another embodiment the invention provides a method to decrease (or more preferably completely inhibit) the binding of gluten-derived peptides involved in food-related immune enteropathy (for example celiac sprue, tropical sprue, giardiasis or food allergies of childhood) or auto-antigen derived peptides involved in auto-immune diseases to T cell receptor molecules comprising providing substances that block the binding of said peptides to said receptor molecules. By decreasing and more preferably completely inhibiting the binding of a gluten-derived epitope to an HLA-DQ molecule or the binding of an neo-epitope to its receptor at the T cell, effects of the immune related disease are decreased or more preferably, completely diminished.

A blocking substance associates with, for example, a T cell receptor molecule and prevents the epitope to associate with said molecule. Such a blocking substance is, for example, a natural or synthetic variant of a gluten-derived epitope or neo-epitope. It is clear that the binding of said blocking substance to said T cell receptor is such that it decreases or more preferably completely diminishes the binding of a gluten-derived epitope or neo-epitope to said receptor molecule. Another way to decrease or more preferably to completely inhibit the binding of a gluten-derived epitope to an HLA-DQ molecule or a neo-epitope to its T cell receptor, is by providing an antibody which associates with said gluten-derived epitope or neo-epitope.

In yet another embodiment the invention provides a method to detect and/or enumerate T-cells bearing a T-cell receptor according to the invention comprising tetrameric complexes of HLA molecules and a gluten-derived epitope or neo-epitope according to the invention. Methods to arrive at such a tetrameric complex are known in the art. In another embodiment the invention provides a method to detect and/or enumerate T-cells bearing a T-cell receptor according to the invention comprising (synthetic) liposomes comprising complexes of HLA molecules and a gluten-derived epitope or neo-epitope according to the invention. Methods to arrive at such complexes are known by the person skilled in the art. These data provide a novel tool for detection and enumeration of T-cells comprising a T-cell receptor according to the invention.

In another embodiment the invention provides a pharmaceutical composition comprising a gluten-derived peptide or auto-antigen derived peptide according to the present invention. Such a pharmaceutical composition is used for the induction of tolerance against said gluten-derived or auto-antigen derived peptide. For tolerance induction doses of this peptide according to the invention are given repeatedly, for instance intravenously, but other routes of administration are suitable too. Another possibility is the repeated oral or nasal administration of such a peptide. Such a peptide according to the present invention is given alone, or in combination with other (toxic) gluten-derived peptides and/or auto-antigen derived peptides, or as part of larger molecules, or coupled to carrier materials/molecules. A pharmaceutical composition comprising a peptide according to the present invention can also be used for elimination of a certain subset of T-cells or for the treatment of celiac disease or auto-immune diseases. Preferably such a pharmaceutical composition according to the present invention contains various, different kinds of, gluten-derived or auto-antigen derived peptides.

In yet another embodiment use is made of a protease inhibitor or of acid neutralizing substances for preventing the generation of a gluten-derived or auto-antigen derived peptide according to the invention or a polypeptide comprising such a peptide. The proteins which comprise the gluten-derived or the auto-antigen derived peptides are not capable of binding to an HLA molecule directly and must first be processed by proteases to provide a peptide or peptides capable of binding to an HLA molecule. Gluten-derived peptides and polypeptides comprising a gluten-derived peptide according to the invention are bound to HLA-DQ molecules and are thereby recognized by a T-cell receptor. By preventing the formation of gluten-derived peptides, binding to HLA-DQ molecules and recognition by T-cell receptors is prevented. One way to prevent a gluten-derived peptide from being generated is by inhibiting the enzyme (for example by protease inhibitors) which is capable of processing the proteins from which the gluten-derived peptides are derived (for example glutenins and/or gliadins). Another way to prevent the gluten-derived peptides from being generated is inactivating the enzyme, which is capable of processing the proteins from which the gluten-derived peptides are derived by providing neutralizing substances. Pepsin and trypsin are examples of enzymes that work under acidic conditions and by providing neutralizing substances the effects of these enzymes are diminished or more preferably completely inhibited.

In the case of auto-antigen derived peptide or polypeptides comprising those, inhibition of the reaction which deamidates said peptides to form neo-epitopes would yield a decrease of the antigenic effect of the auto-antigen. Preferably said inhibition is accomplished by local inhibition of the enzyme tTG at the site(s) where normally these neo-epitopes are formed. For celiac disease, site specific administration in the gut would be the method of choice. In RA, the specific site to inhibit the formation of neo-epitopes would be the joints, while for Sjogren syndrome the site would be the tear and salivary glands. Inhibition of the enzyme can be accomplished by specific inhibitory compounds, e.g. by antibodies binding to the enzyme in such a way that the enzymatic function is decreased. Another approach is to design specific compounds on the basis of the defined specificity of tTG for particular gluten sequences like QLPF and QXXF. Site-specific action of such compounds can be achieved by local application of the inhibitor, e.g. by injection.

The invention will be explained in more detail in the following detailed description which is not limiting the invention.

### Materials and Methods

*Peptides and tTG treatment*. Peptides were synthesised by standard Fmoc chemistry on a multiple peptide synthesizer (SyroII, MultiSynTech GmbH, Witten, Germany). Integrity of synthetic peptides was checked by rpHPLC and mass spectrometry. Tissue transglutaminase (tTG) treatment was performed by incubating the peptides (500 µg/ml) with the enzyme (100 µg/ml, Sigma, Zwijndrecht, NL; T-5398) at 37°C for 4 h minimum, in 50 mM triethylamine-acetate pH 6.5, 2 mM CaCl₂.

*Mass spectrometry.* Electrospray ionisation mass spectrometry was performed on the synthetic gluten peptides before and after tTG treatment, using a Q-TOF hybrid mass spectrometer (Micromass, Manchester, UK). Precursor ions were selected with the quadrupole window set to 3 Da and fragments were collected with high efficiency with the orthogonal time of flight mass spectrometer. The collision gas applied was argon (pressure 4x10⁻⁵ mbar) and the collision voltage approximately 30 V. Deamidation of glutamine residues in synthetic gluten peptides was determined by mass spectrometric analysis as described previously (van de Wal, 1998a). Deamidation of a glutamine residue results in an increase of 1 Da. These conversions were assigned to particular glutamine residues by comparison of the fragmentation spectra of tTG treated and non-treated peptides.

*Database searching*. The program PeptideSearch was used for pattern searches Mann, M. and Wilm, M., Anal. Chem. 66: 4390-4399, 1994). The patterns were composed on the basis of the newly identified specificity of tissue transglutaminase in combination with the previously identified HLA-DQ2 peptide binding motif (Johansen 1996; van de Wal, 1996). For database searching a selected subset of wheat proteins from a compilation of the Swiss Prot databank, SPTREMBL and PIR was used. This compilation contained 982 proteins.

*T cell clones and T cell lines.* Gluten specific T cell lines were generated from small intestinal biopsies of celiac disease patients as described before (van de Wal, 1998a).

*T cell proliferation assays*. Proliferation assays were performed in duplicate in 150 µl RPMI1640 (Gibco, Breda, NL) supplemented with 10% human serum in 96-well flat-bottomed plates (Falcon) using 10⁴ T cells stimulated with 10⁵ irradiated PBMCs (3000 RAD) in the presence or absence of antigen (1-10 µg/ml). After 48 hours at 37°C, cultures were pulsed with 0.5 µCi of ³H-thymidine and harvested 18 hours thereafter as described previously (van de Wal, 1998b).

### Results

*The specificity of tTG.* Previously we have characterized an HLA-DQ8 restricted, T cell stimulatory gliadin peptide (van de wal, 1998a; 1998b). In the core of this gliadin peptide (sequence PSGQ₂₀₈GSFQPSQQ₂₁₆NPQA) the Q₂₀₈ and Q₂₁₆ are deamidated by tTG, whereas positions Q₂₁₂, Q₂₁₅, and Q₂₁₉ are not (van de Wal, 1998a). Preliminary studies indicated that the nature of C-terminal flanking amino acids influenced deamidation of glutamine residues (not shown). We have now investigated this systematically by analyzing the deamidation in amino acid substitution analogs of a shorter version of the gliadin peptide (sequence: PSGQ₂₀₈GSFQPSQQ₂₁₆N). In this peptide only the Q₂₀₈ is deamidated by tTG treatment (Fig. 1). An example of this type of analysis shows the simultaneous mass spectrometric analysis of 4 of these substitution analogs, that have either the amino acid V, D, E, or F at position Q₂₀₈+3, and reveals the distinct masses corresponding to these peptides (Fig. 1a). Upon treatment of the peptides with tTG two of the masses increase by 1 Da, indicating conversion of a glutamine into a glutamic acid (Fig. 1a). Subsequent analysis of collision induced fragments of the individual peptides (not shown) was used to verify that deamidation occurred at Q₂₀₈ in these peptides. These results demonstrate that in the substitution analogs with the amino acids V and F at position Q₂₀₈+3, complete deamidation of Q₂₀₈ is found while in the peptides that carry a D or E at Q₂₀₈+3, no deamidation of Q₂₀₈ takes place. The complete analysis demonstrates that at only two positions in the peptide substitutions had effect on deamidation of Q₂₀₈ (not shown and Fig. 1b). First, the replacement of Q₂₀₉ by a P abolished deamidation of Q₂₀₈.

Second, several amino acid substitutions at F₂₁₁ had a strong negative effect on deamidation of Q₂₀₈, in particular replacements of F₂₁₁ for amino acids with significantly smaller or charged side chains or replacement by proline (Fig. 1a, b). Next, we carried out a similar analysis to determine the influence of amino acid substitutions on the deamidation of Q₂₁₆ in a longer version of the peptide (Fig.1b). The results demonstrate that the substitution of N₂₁₇ with a P abolishes deamidation of Q₂₁₆, a finding that underscores the observation that in a QP sequence the Q is not a target for tTG. Moreover, the substitution of Q₂₁₉ with a P had a negative effect on deamidation of Q₂₁₆, confirming the negative influence of the P in the sequence QXXP (X stands for any amino acid). Amino acid substitutions (with the exception of the P substitution) at Q₂₁₆+3, however, had no effect on deamidation of Q₂₁₆, a result that is in contrast to the strong influence of amino acid substitutions at position Q₂₀₈+3 on deamidation of Q₂₀₈. A marked difference between the sequence Q₂₀₈GSF and Q₂₁₆NPQ is the presence of a P in the latter sequence at Q+2 suggesting that in the sequence QXP the Q is a good target for deamidation and the nature of the amino acid at Q+3 is much less important (see also below).

Together the results indicate that in the sequences QP and QXXP the Q is not a target for tTG. In contrast the sequences QXP, QXX(F, Y, W, M, L, I, or V) and QXP(F, Y, W, M, L, I, or V) favor deamidation of the Q (Table 1). To verify these "rules" for deamidation by tTG, a large panel of gluten peptides was synthesized, treated with tTG and analyzed by mass spectrometry (Table 2). Without exception we found that a Q preceding a P is not deamidated by tTG while in the majority of cases a P at Q+3 also inhibited deamidation. In contrast, a bulky/hydrophobic residue at Q+3, and P at Q+2 allowed good deamidation. In the case that two opposite rules coincide, as is found in peptide 20 (LQQPQQ₆PQFQPQQQF), the negative effect of QP↓ overrules the positive effect of QXXF↑ at position 6. Together, these results confirm the defined patterns. Proline thus plays a crucial role in the deamidation of gluten peptides.

*Prediction of novel gluten epitopes* The peptide binding motifs of the disease associated molecules HLA-DQ2 and HLA-DQ8 display preferential binding of negative charges at anchor positions (Johansen, 1996; van de Wal, 1996, Kwok, 1996). HLA-DQ2 has a strong preference for a negative charge at p4 and p7. Strikingly, at p6 a proline is one of the preferred amino acids in the HLA-DQ2 peptide binding motif that could thus target specific deamidation at p4 (Table 3). Therefore we combined the HLA-DQ2 peptide binding motif with the specificity of tTG to construct an algorithm that could be used to predict novel T cell stimulatory gluten peptides from the gluten database (Table 3). In this algorithm glutamine residues were introduced at the putative DQ2 binding positions p4 and p7 that serve as potential target sites for tTG to allow introduction of a negative charge at these positions. Next, proline was selected for p6 to promote deamidation at Q₄, which is also consistent with the requirements for peptide binding at this position (Table 3). For the p9 position two options were designed. In the first option bulky/hydrophobic residues were selected for p9, according to the peptide binding motif. In the second option, a proline residue was placed at p9, followed by bulky/hydrophobic at p10. This option does not agree with the peptide binding motif but should allow optimal deamidation at the Q₇ according to our QXPF rule. The remaining positions p1, p2, p3, p5, and p8 were not defined (Table 3). These two algorithms were used to search in the gluten database. A total of 98 matches were obtained, which corresponded to 27 unique gluten sequences. These 27 unique gluten peptides were synthesized, and deamidated by tTG. Mass spectral analysis confirmed deamidation of the predicted Q₄- and Q₇-residues (not shown).

The native and deamidated peptides were pooled in pools of five peptides each, and tested in T cell proliferation assays with three gluten specific T cell lines isolated from small intestinal biopsies of celiac disease patients. Two of these T cell lines responded to stimulation by several of the deamidated peptide pools (Fig 2a, results shown for one of the T cell lines). The responses were exclusively found against pools that contained peptides predicted by the second algorithm, XXXQ₄XP₆Q₇XP₉ (Y,F,W,I,L)₁₀ (Fig. 2a, b). Subsequently, the T cell line was tested against the individual peptides of the positive pools, and found to respond to 8 out of 13 peptides tested (Fig. 2b).

In addition we performed searches with a less strict variant of algorithm 2, XXXX₄X P₆ Q₇ X P₉ (Y,F,W,I,L)₁₀, which predicts the deamidation of only one Q-residue. This search yielded 261 matches in the gluten database (Table 4). Eighteen of these matches corresponded to an alpha-gliadin peptide that has previously been shown to be immunodominant in adult celiac disease patients, QLQPFPQPQLPY (Arentz-Hansen, 2000; Anderson, 2000).

Next we used the three algorithms to perform additional database searches in gluten-like storage molecules, in particular the hordeins from barley, the secalins from rye and the avenins from oats. While the first two cereals are toxic for CD patients, the latter is not (Janatuinen, E.K. *et al.*, N. Engl. J. Med. 333: 1033-1037, 1995; Janatuinen, E.K. *et al.*, Gut 46: 327-331, 2000). Strikingly, all three search algorithms yielded many matches in the available hordein and secalin sequences but none in the avenin sequences (Table 4). Consequently, the predicted T cell stimulatory gluten peptide QQPFPQQPQQPFPQ (Fig 2) is present in hordeins and secalins but not in avenins (Table 4). Additional database searches with less strict algorithms revealed a general lack of matches in the avenin database as compared to the gluten, hordein and secalin databases (Table 4). Notably, no matches were found in the avenins with three variants of the predictive algorithm 2.

The enzyme pepsin is known to preferentially cleave proteins after phenylalanine and to a lesser extent after tyrosine, leucine, and isoleucine. Such a cleavage in the stomach will thus generate many gluten peptides with the tTG substrate sequence QXPF(Y) at the C-terminus. The subsequent activity of pepsin and tTG, therefore, favors the generation of many gluten peptides with an appropriate p7 anchor for binding to HLA-DQ2. We also demonstrate that by combining the HLA-DQ2 peptide binding motif with two deamidation patterns an algorithm is obtained that predicts novel T cell stimulatory peptides in gluten. Strikingly, in this predictive algorithm [X XXQ₄XP₆Q₇XP₉(Y,F,W,I,L)₁₀] we had incorporated a deamidation pattern that results in peptides that do not optimally fit the HLA-DQ2 peptide binding motif, e.g. they lack an amino acid with a large hydrophobic side chain at position 9 in the peptide. The lack of this anchor is probably compensated by the introduction of two negative charges in the peptide at p4 and p7 due to deamidation that serve as strong anchors. Thus, the T cell stimulatory activity of these peptides appears to depend more on optimal deamidation than on adherence to the exact HLA-DQ2 peptide binding motif. A less stringent search algorithm, combining only one deamidation rule with the HLA-DQ2 peptide binding motif proved equally successful since it predicted the previously identified T cell stimulatory alpha-gliadin peptide. Importantly, the first predictive algorithm identified 13 peptides, 8 out of which stimulated T cells. The second, less stringent algorithm predicted 261 peptides. Eighteen of those (1 in 15) matched a known T cell stimulatory gliadin peptide. These algorithms, therefore, have a high predictive value. It is striking, therefore, that these algorithms identified very similar and sometimes identical peptides in the barley and rye derived hordeins and secalins but not in the oats derived avenins. These results appear not related to the size of the avenin database since the secalin database is the smallest of the three investigated. Oats is considered safe for CD patients (Janatuinen, 1995; 2000). A striking difference between the amino acid content of gliadins, hordeins, and secalins on one side and the avenins on the other side is the relative lack of proline in the latter (Wieser, H. *et al.* Z. Lebensm. Unters. Forsch. 177: 457-460, 1983). While gliadins, hordeins and secalins contain approximately 36% glutamine and 20% proline, avenins contain a similar percentage of glutamine (34%) but half the amount of proline (10%). The lack of proline residues in Q-rich regions leads to non-selective deamidation of the glutamine residues in such regions. In the gluten sequence PFSQQQQPV, for example, the underlined Q-residues are specifically and efficiently deamidated by tTG while in the naturally occurring homologous sequence PFSQQQQLVL, in which the proline at p8 is replaced by a leucine, all Q-residues are deamidated. The deamidated peptide does not match the HLA-DQ2 peptide binding motif. Our results therefore offer a simple explanation for the clinical observation that oats is not toxic for CD patients. Moreover, our results indicate that the replacement of particular proline residues in gluten by other amino acids would yield gluten molecules with considerably less toxicity for CD patients but that would retain the chemical and physical properties that are so desired in gluten. Potentially this knowledge may be used to construct gluten molecules with lower toxicity or design oligonucleotide primers to identify wheat varieties that lack (some of) these relevant proline residues.

**Table 1.**

| Deamidation patterns in the HLA-DQ8 restricted gliadin peptide. | |
|---|---|
| **Pattern** | **Effect on deamidation** |
| **Q**P ↓ | no deamidation |
| **Q**XX(F,Y,W,M,L,I,V) ↑ | good deamidation |
| **Q**XXP ↓ | no deamidation |
| **Q**XP ↑ | good deamidation |
| **Q**XP(F,Y,W,M,L,I,V) ↑ | good deamidation |

### Table 2. Validation of the deamidation patterns.

The observed deamidation patterns in a set of twenty-one gluten peptides were compared with those predicted according to the deamidation patterns given in Table 1. The deamidation patterns are represented in the four columns. The percentages shown indicate whether the pattern is fully or partially applicable to the specific deamidation of Q residues in the listed gluten peptides. A bar (-) indicates the absence of that sequence in the peptide. Deamidated glutamine residues are shown in bold and underlined.

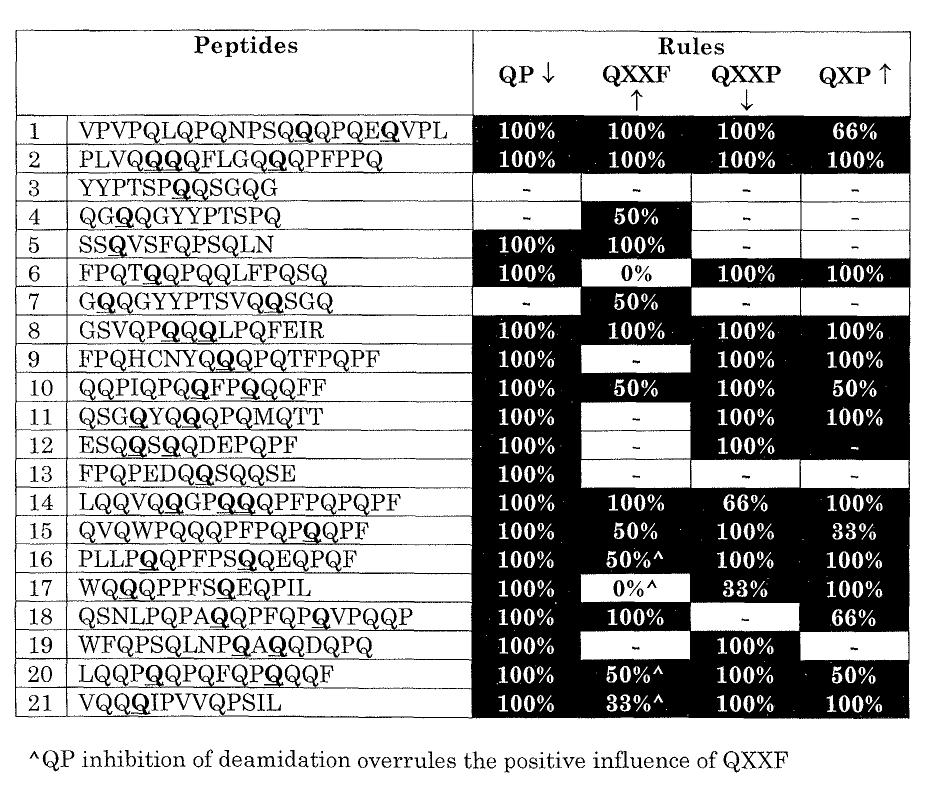

**Table 3.**

| Design of search algorithms based on the HLA-DQ2 peptide binding motif and the tTG specificity. | | | | | | | | | | | | | | |
|---|---|---|---|---|---|---|---|---|---|---|---|---|---|---|
| **DQ2 peptide binding motif** | | | | | | | | | | | | | | |
| Residue | -2 | -1 | 1 | 2 | 3 | 4 | 5 | 6 | 7 | 8 | 9 | 10 | 11 | 12 |
| Preferred | - | - | F,W, YI,L, | | | D,E V,L, | | P,A E | D, E | | F,W,Y I,L,V, V I M | - | - | - |

| **DQ2 epitope selection algorithms** | | | | | | | | | | | | | | |
|---|---|---|---|---|---|---|---|---|---|---|---|---|---|---|
| Algonth m 1 | X | X | X | X | X | Q | X | P | Q | X | F,Y,W I,L | X X X | | |
| Algorith m 2 | X | X | X | X | X | Q | X | P | Q | X | P | F,Y, WI,L | X | X |

| **Gluten Database search** | | | | | | | | | | | | | | |
|---|---|---|---|---|---|---|---|---|---|---|---|---|---|---|
| Algorith m 1 | Matches: 52 Unique: 14 | | | | | | Source: Gliadin (6); Glutenin (8) | | | | | | | |
| Algorith m 2 | Matches: 46 Unique: 13 | | | | | | Source: Gliadin (all) | | | | | | | |

### Table 4. Database searches with the search algorithms.

The search algorithms reveal many matches in the gluten, hordeins and secalin sequences but very few in the avenin sequences. The predictive algorithm 2 [QXPQXP(YFWIL)] and the less strict variants of that algorithm have no matches in the avenin database. The novel T cells stimulatory gluten peptide (QQPFPQQPQQPFPQ) is also found in the hordeins and secalin but not in the avenins. Other search algorithms identify only a limited number of sequences in the avenins.

| **Search algorithm** | | **# of matches in databases gluten hordein secalin avenin** | | | |
|---|---|---|---|---|---|
| Algorithm 1 | Q₄XPQ₇X(YFWIL) | 52 | 48 | 8 | - |
| Algorithm 1 - Q₄ | XXPQ₇X(YFWIL) | 334 | 91 | 14 | 6 |
| Algorithm 1 - Q₇ | Q₄XPXX(YFWIL) | 286 | 142 | 60 | 11 |
| Algorithm 2 | Q₄XPQ₇XP(YFWIL) | 46 | 60 | 33 | - |
| Algorithm 2 - Q₄* | XXPQ₇XP(YFWIL) | 261 | 196 | 89 | - |
| Algorithm 2 - Q₇ | Q₄XPXXP(YFWIL) | 51 | 68 | 33 | - |
| Short algorithm | QXP(YFWIL) X≠ P** | >500 | 276 | 105 | 7 |
| Predicted epitope | QQPFPQQPQQPFPQ | 6 | 12 | 2 | - |

| | | | | | |
|---|---|---|---|---|---|
| * Algorithm 2 without the specification of a Q at position 4 predicts the known gliadin α9 epitope | | | | | |
| ** A proline at p2 in this algorithm inhibits deamidation of the glutamine | | | | | |

**Table 5**

| **List of protein autoantigens in multiple sclerosis (MS), rheumatoid arthritis (RA), diabetes (DB), systemic lupus erythematosus (SLE), Sjogren syndrome (SS) and** **Coeliac disease (CD) and peptides derived from these proteins that contain the tTG-substrate motif disclosed herein**^{**1**} | | | | |
|---|---|---|---|---|
| Autoautigen^{2,5} | Code used³ | Database | Accession number | Amino acids⁴ |
| **MS** | | | | |
| Myelin basic protein | MBP | Swiss Prot | P02686 | 304 |
| Myelin-oligodendrocyte glycoprotein | MOG | Swiss Prot | Q16653 | 247 |
| Myelin proteolipid protein | PLP | Swiss Prot | P06905 | 276 |
| **RA** | | | | |
| Complement C1q subcomponent, A chain | C1QA | Swiss Prot | P02745 | 245 |
| Complement C1q subcomponent, B chain | C1QB | Swiss Prot | P02746 | 251 |
| Complement C1q subcomponent, C chain | C1QC | Swiss Prot | P02747 | 245 |
| Carbonic anhydrase II | CA2 | Swiss Prot | P00918 | 259 |
| Calpain inhibitor | CALP | Swiss Prot | P20810 | 708 |
| Alpha-1 type II collagen | COL2A1 | TrEMBL | Q14047 | 1487 |
| Filaggrin | FILA | Swiss Prot | P20930 | 416 |
| 78 kDa glucose-regulated protein | GR78 | Swiss Prot | P11021 | 654 |
| Matrix metalloprotease-1 | MMP1 | Swiss Prot | P03956 | 469 |
| Matrix metalloprotease-3 | MMP3 | Swiss Prot | P08254 | 477 |
| Matrix metalloprotease-19 | MMP19 | Swiss Prot | Q99542 | 508 |
| Heterogenous nuclear ribonucleoproteins A2/B1 | ROA2 | Swiss Prot | P22626 | 353 |
| Pulmonary surfactant-associated protein A | SPA | Swiss Prot | P07714 | 248 |
| Vinculin | VINC | Swiss Prot | P18206 | 1065 |
| Chitinase 3-like protein 2 | YKL39 | Swiss Prot | Q15782 | 390 |
| Chitinase 3-like protein 1 | YKL40 | Swiss Prot | P36222 | 383 |
| **DB** | | | | |
| Carboxypeptidase H | CPH | Swiss Prot | P16870 | 476 |
| Delta-like protein | DLK | Swiss Prot | P80370 | 383 |
| Glutamate decarboxylase, 65 kDa isoform | GAD | Swiss Prot | Q05329 | 585 |
| Glucose transporter type 4 | GLUT4 | Swiss Prot | P14672 | 509 |
| 60 kDa Heat shock protein | HSP60 | Swiss Prot | P10809 | 573 |
| Protein-tyrosine phosphatase-like N | ICA | Swiss Prot | Q16849 | 979 |
| 69 kDa islet cell autoantigen | ICA69 | Swiss Prot | Q05084 | 480 |
| Imogen 44 | IM44 | TrEMBL | Q61733 | 384 |
| Insulin precursor | INS | Swiss Prot | P01308 | 110 |
| Insulin receptor | INSR | Swiss Prot | P06213 | 1382 |
| Insulin receptor substrate-1 | IRS1 | Swiss Prot | P35568 | 1242 |
| SOX-13 protein | SOX13 | Swiss Prot | Q9UN79 | 889 |
| **SLE/SS** | | | | |
| Ceritromere protein A | CENPA | Swiss Prot | P49450 | 140 |
| Centromere protein B | CENPB | Swiss Prot | P07199 | 599 |
| DNA excision repair protein ERCC-1 | ERCC1 | Swiss Prot | P07992 | 297 |
| 60S ribosomal protein L7 | RL7 | Swiss Prot | P18124 | 248 |
| 52 kDa ribonucleoprotein autoaritigen RO/SS-A | ROSS | Swiss Prot | P27797 | 417 |
| Small nuclear ribonucleo-protein associated proteins B and B' | SMPB | Swiss Prot | P14678 | 240 |
| Small nuclear ribonucleo-protein associated protein N | SMPN | Swiss Prot | P14648 | 240 |
| SNRPB protein | SNRPB | TrEMBL | Q15182 | 285 |
| SSA protein SS-56 | SS56 | TrEMBL | Q96PF7 | 485 |
| **Miscellaneous/CD** | | | | |
| Tiisue transglutaminase | TTG | Swiss Prot | P21980 | 687 |

| | | | | |
|---|---|---|---|---|
| ¹ This table reflects the consensus amino acid sequences of the proteins mentioned. It is known that for some proteins mentioned variants exsist, like splice variants and variants having one or more amino acid mutations. These variants should be regarded as being comprised in the table. Also the peptides that are derived from these variant and that contain the tTG-substrate motif disclosed herein should be regarded as being comprised in this table. | | | | |
| ² Some of the proteins listed are also known by one or more other names | | | | |
| ³ For some of the proteins listed one or more other codes are used in databases | | | | |
| ⁴ Number of amino acids given here reflect the amino acid sequence indicated, numbers might be different for variants | | | | |
| ⁵ Some antigens are known to possibly play a role in more then one of the diseases mentioned. The way the antigens are grouped is only meant for clarity and is by no means intended to limit the claims ⁶ Peptides are named by the sequence number in the protein that corresponds to the aminoterminal amino acid of the peptide ⁷ Only 19-mer peptides having the target Q at position 10 are shown. It is known that the length of HLA-bound peptides can vary from 8 to up to 40 amino acids. Longer peptides containing a 19-mer sequence or part of a 19-mer sequence from the table and shorter peptides containing part of a 19-mer sequence from the table are considered to be comprised in this table | | | | |

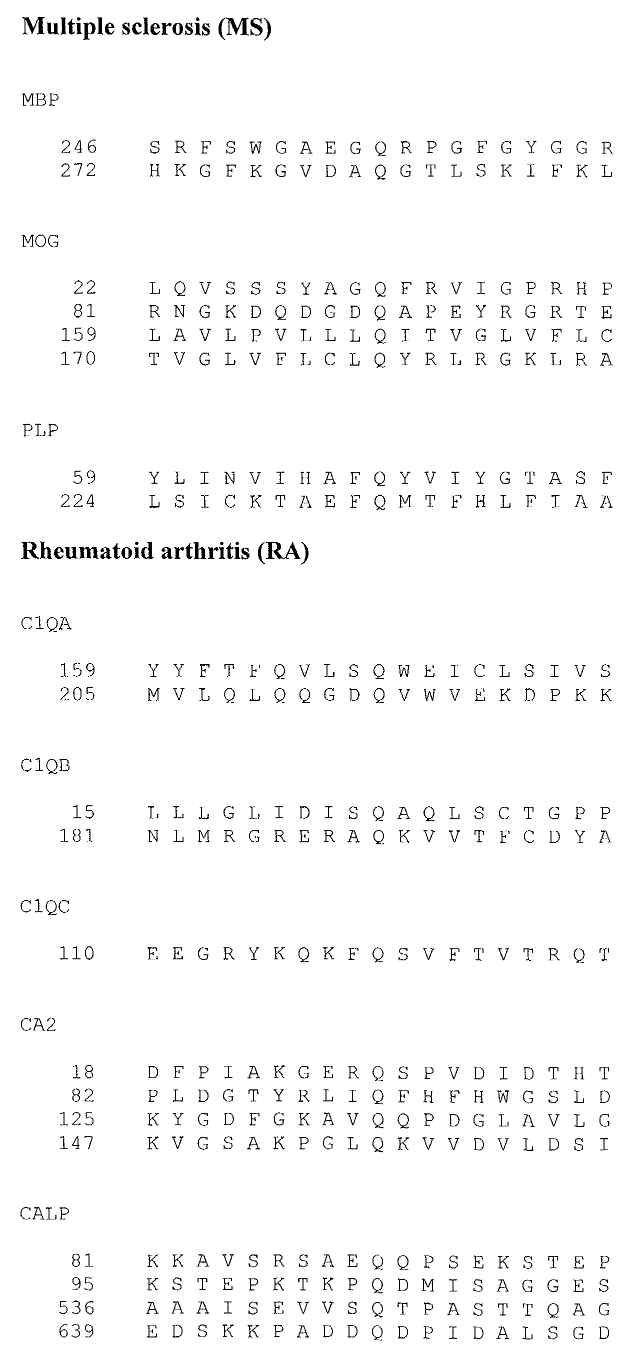

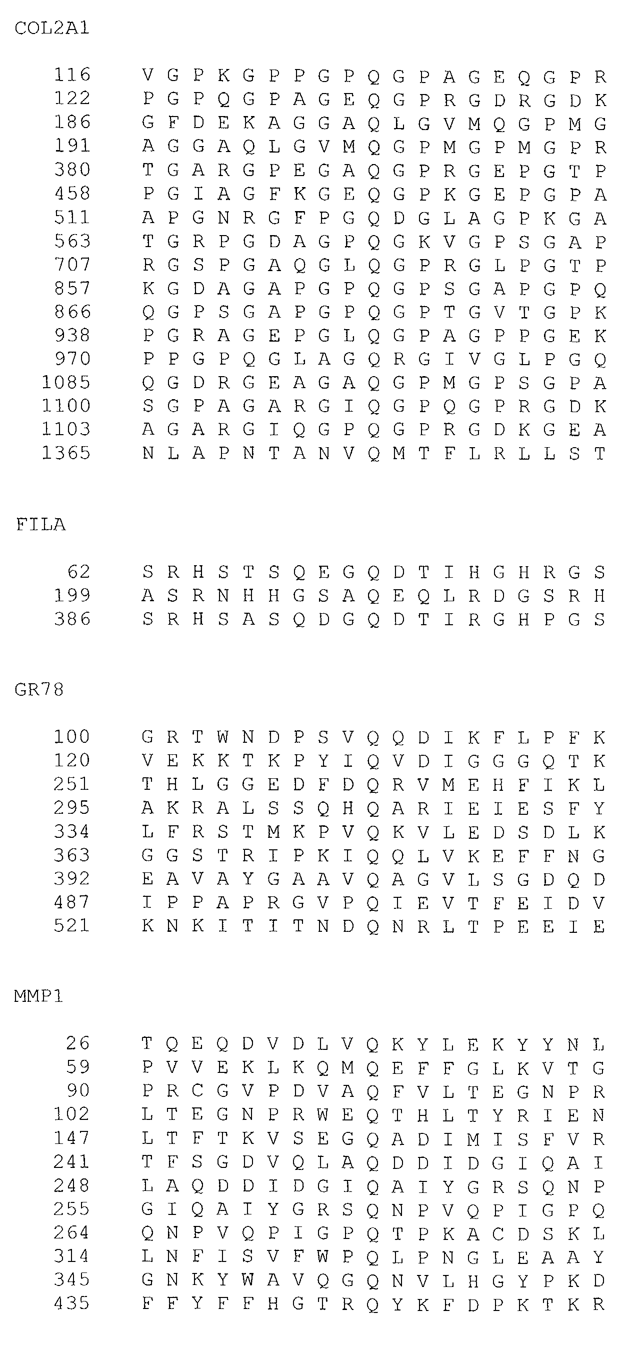

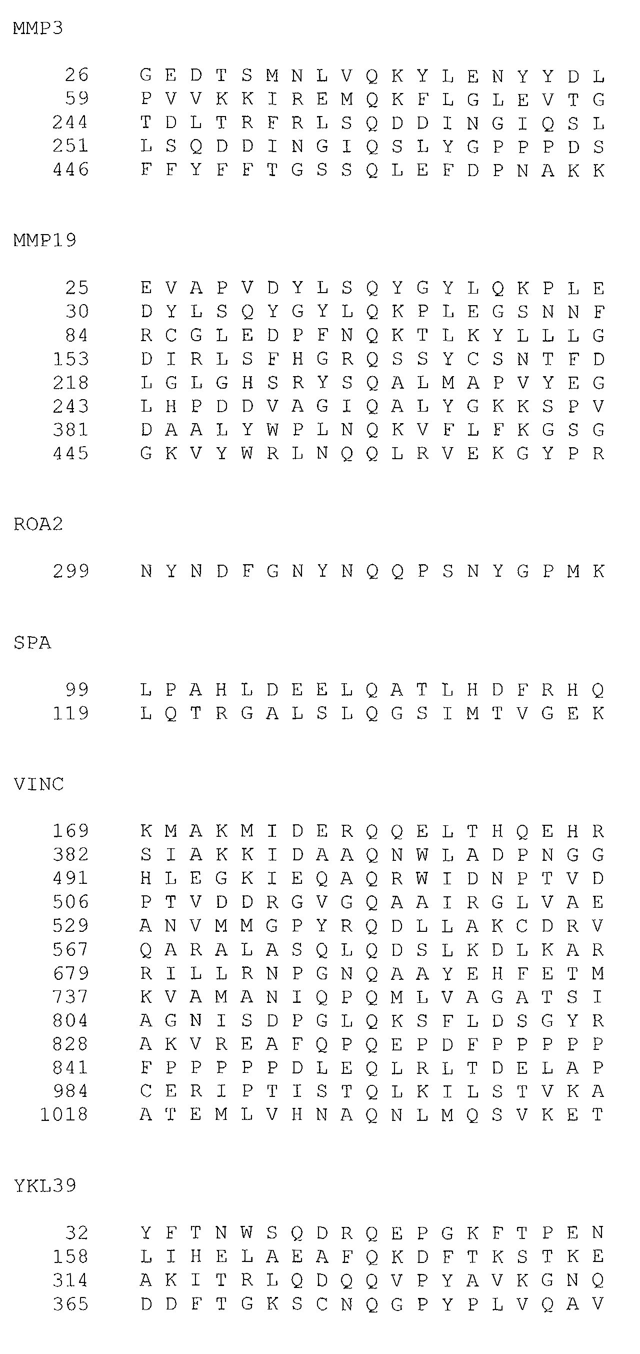

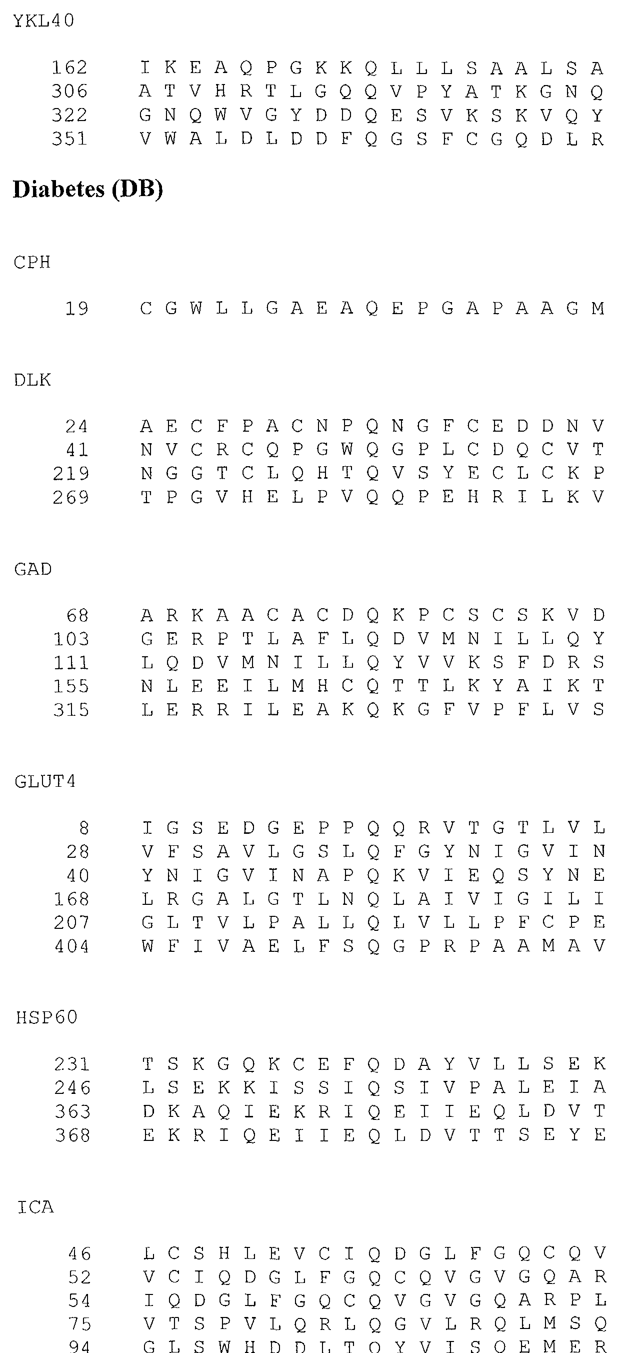

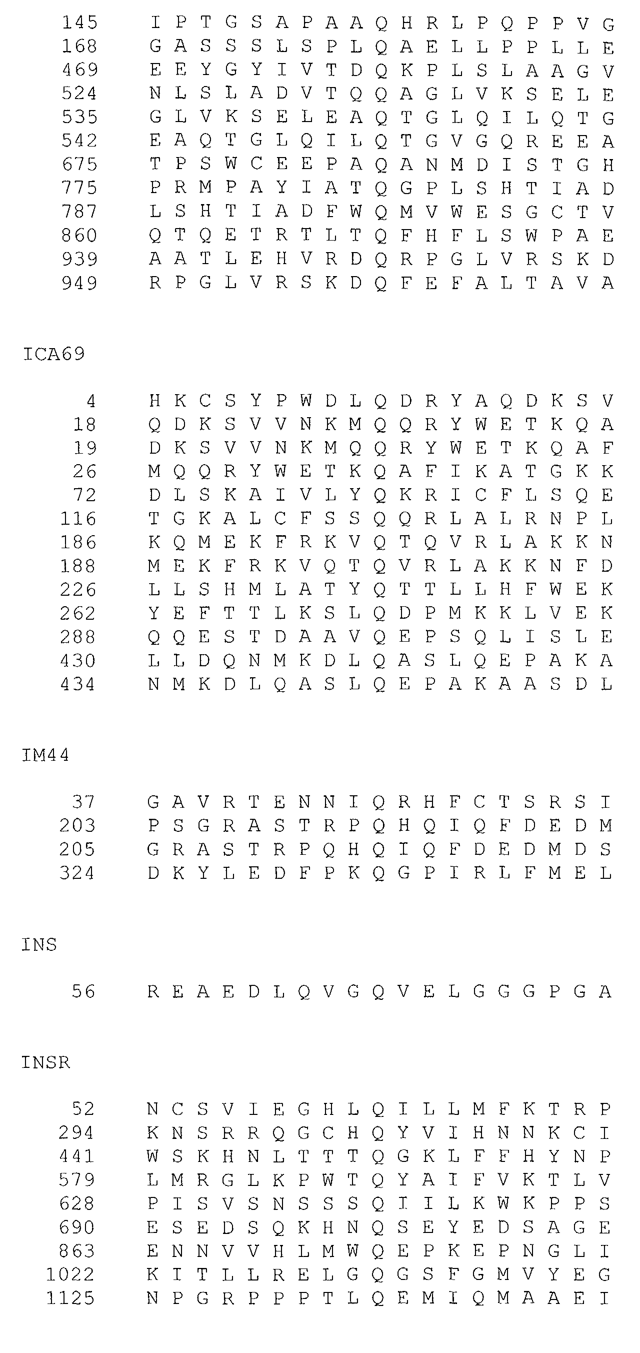

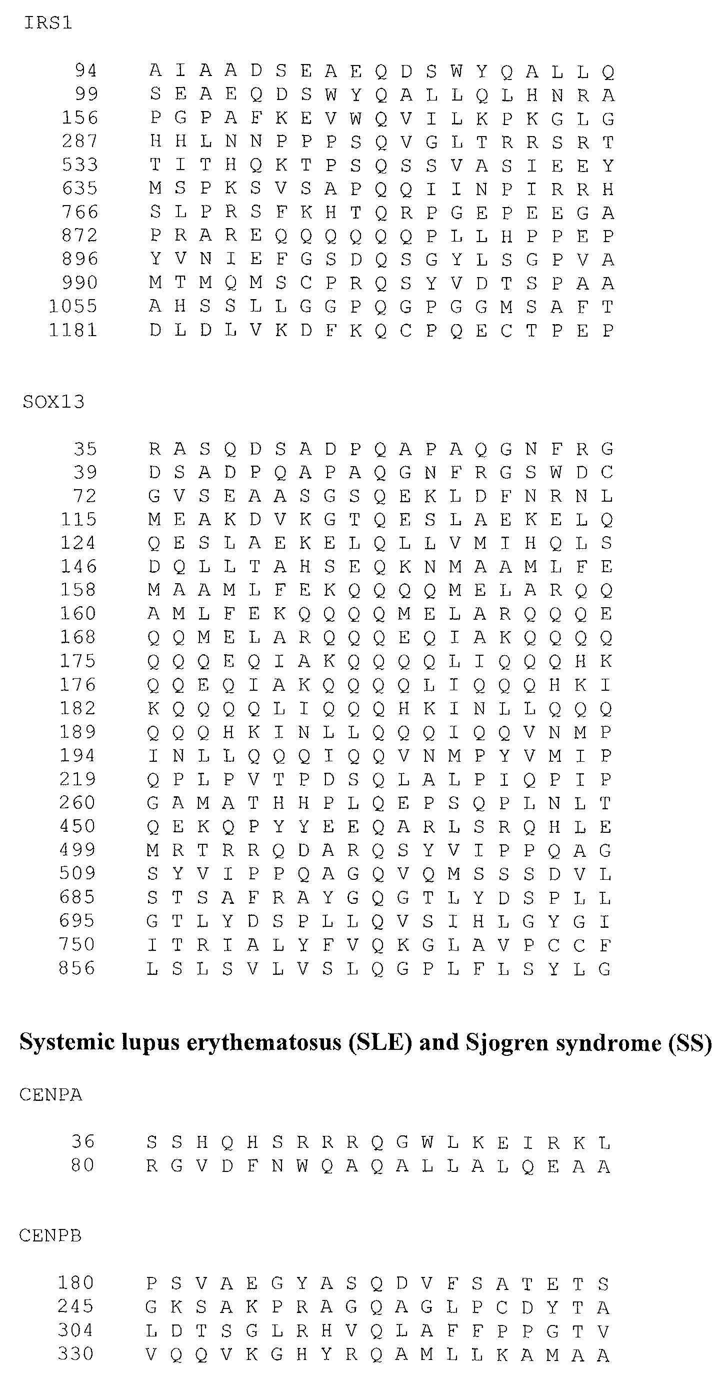

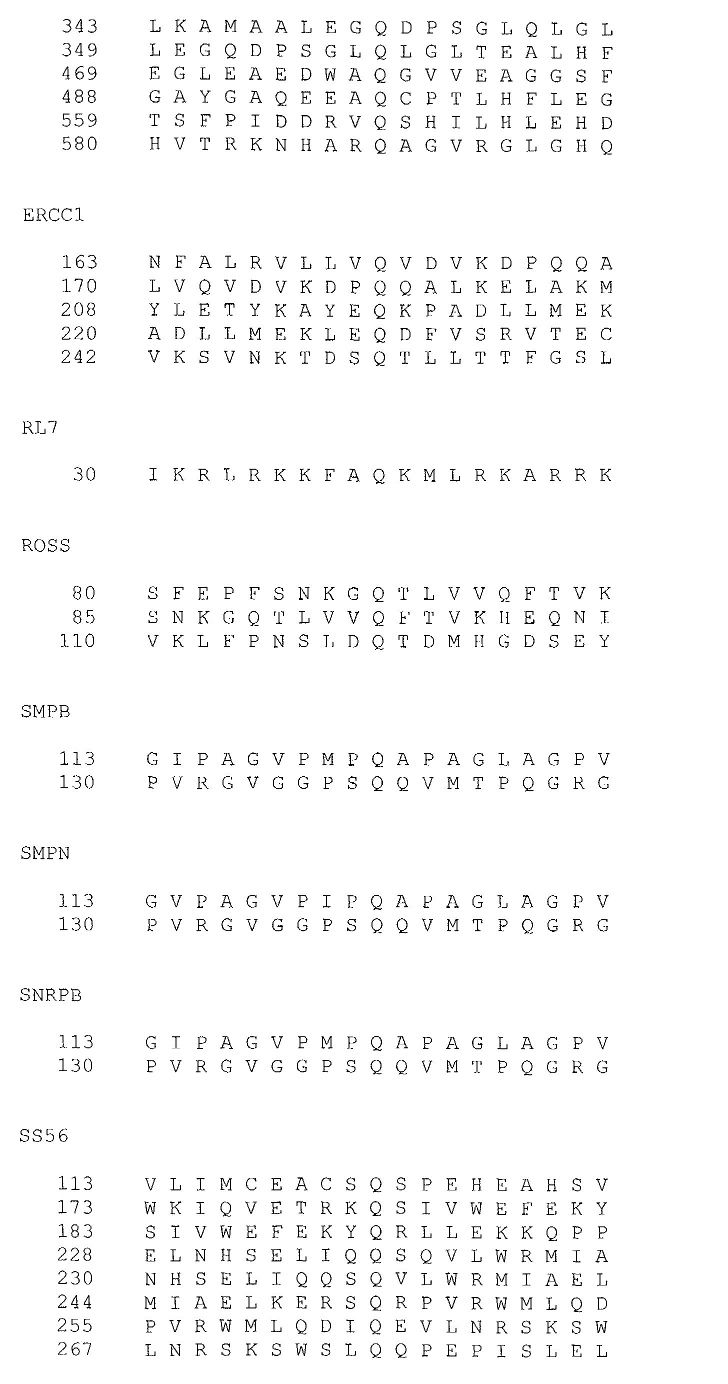

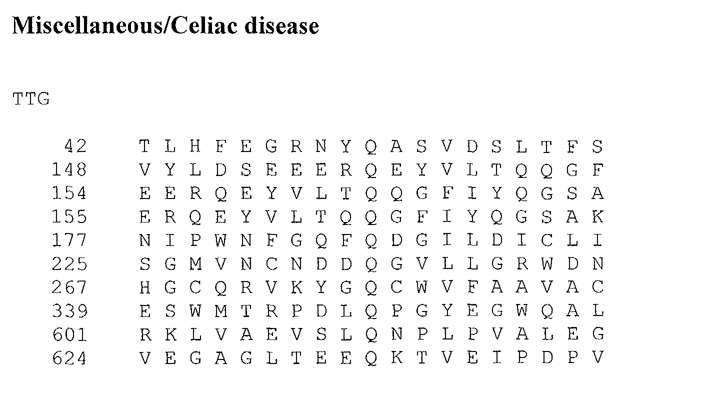

P58975ep
## Claims

1. Peptide sequence of 14-40 amino acids, which is prone to deamidation by tTG and which is a causative factor of celiac disease, which comprises an amino acid sequence selected from the group consisting essentially of QQPYPQQPQQPFPQ, QQPFPQQPQQPFPQ, PFPQTQQPQQPFPQ, PFPQLQQPQQPFPQ, QFPQTQQPQQPFPQ, QLPFPQQPQQPFPQ, PFPQPQQPQQPFPQ and PFPQSQQPQQPFPQ.

2. Epitope which is the causative factor of celiac disease, selected from the group consisting essentially of QQPYPEQPQQPFPQ, QQPFPEQPQQPFPQ, PFPQTEQPQQPFPQ, PFPQLEQPQQPFPQ, QFPQTEQPQQPFPQ, QLPFPEQPQQPFPQ, PFPQPEQPQQPFPQ, PFPQSEQPQQPFPQ, QQPYPEQPEQPFPQ, QQPFPEQPEQPFPQ, PFPQTEQPEQPFPQ, PFPQLEQPEQPFPQ, QFPQTEQPEQPFPQ, QLPFPEQPEQPFPQ, PFPQPEQPEQPFPQ, PFPQSEQPEQPFPQ, QQPYPQQPEQPFPQ, QQPFPQQPEQPFPQ, PFPQTQQPEQPFPQ, PFPQLQQPEQPFPQ, QFPQTQQPEQPFPQ, QLPFPQQPEQPFPQ, PFPQPQQPEQPFPQ and PFPQSQQPEQPFPQ, wherein further Q residues may be deamidated into an E residue.

3. Peptide sequence, which is prone to deamidation by tTG and which is a causative factor of an autoimmune disease selected from the group consisting essentially of RA (rheumatoid arthritis), MS (multiple sclerosis), SLE (systemic lupus erythomatosus), SS (Sjogren syndrome) and DB (diabetes), selected from the group consisting essentially of:

S R F S W G A E G Q R P G F G Y G G R,
H K G F K G V D A Q G T L S K I F K L,
L Q V S S S Y A G Q F R V I G P R H P,
R N G K D Q D G D Q A P E Y R G R T E,
L A V L P V L L L Q I T V G L V F L C,
T V G L V F L C L Q Y R L R G K L R A,
Y L I N V I H A F Q Y V I Y G T A S F,
L S I C K T A E F Q M T F H L F I A A,
Y Y F T F Q V L S Q W E I C L S I V S,
M V L Q L Q Q G D Q V W V E K D P K K,

L L L G L I D I S Q A Q L S C T G P P,
N L M R G R E R A Q K V V T F C D Y A,
E E G R Y K Q K F Q S V F T V T R Q T,
D F P I A K G E R Q S P V D I D T H T,
P L D G T Y R L I Q F H F H W G S L D,
K Y G D F G K A V Q Q P D G L A V L G,
K V G S A K P G L Q K V V D V L D S I,
K K A V S R S A E Q Q P S E K S T E P,
K S T E P K T K P Q D M I S A G G E S,
A A A I S E V V S Q T P A S T T Q A G,
E D S K K P A D D Q D P I D A L S G D,
V G P K G P P G P Q G P A G E Q G P R,
P G P Q G P A G E Q G P R G D R G D K,
G F D E K A G G A Q L G V M Q G P M G,
A G G A Q L G V M Q G P M G P M G P R,
T G A R G P E G A Q G P R G E P G T P,
P G I A G F K G E Q G P K G E P G P A,
A P G N R G F P G Q D G L A G P K G A,
T G R P G D A G P Q G K V G P S G A P,
R G S P G A Q G L Q G P R G L P G T P,
K G D A G A P G P Q G P S G A P G P Q,
Q G P S G A P G P Q G P T G V T G P K,
P G R A G E P G L Q G P A G P P G E K,
P P G P Q G L A G Q R G I V G L P G Q,
Q G D R G E A G A Q G P M G P S G P A,
S G P A G A R G I Q G P Q G P R G D K,
A G A R G I Q G P Q G P R G D K G E A,
N L A P N T A N V Q M T F L R L L S T,
S R H S T S Q E G Q D T I H G H R G S,
A S R N H H G S A Q E Q L R D G S R H,
S R H S A S Q D G Q D T I R G H P G S,
G R T W N D P S V Q Q D I K F L P F K,
V E K K T K P Y I Q V D I G G G Q T K,
T H L G G E D F D Q R V M E H F I K L,
A K R A L S S Q H Q A R I E I E S F Y,
L F R S T M K P V Q K V L E D S D L K,
G G S T R I P K I Q Q L V K E F F N G,
E A V A Y G A A V Q A G V L S G D Q D,
I P P A P R G V P Q I E V T F E I D V,
K N K I T I T N D Q N R L T P E E I E,
T Q E Q D V D L V Q K Y L E K Y Y N L,
P V V E K L K Q M Q E F F G L K V T G,
P R C G V P D V A Q F V L T E G N P R,
L T E G N P R W E Q T H L T Y R I E N,
L T F T K V S E G Q A D I M I S F V R,
T F S G D V Q L A Q D D I D G I Q A I,
L A Q D D I D G I Q A I Y G R S Q N P,
G I Q A I Y G R S Q N P V Q P I G P Q,
Q N P V Q P I G P Q T P K A C D S K L,
L N F I S V F W P Q L P N G L E A A Y,
G N K Y W A V Q G Q N V L H G Y P K D,
F F Y F F H G T R Q Y K F D P K T K R,

G E D T S M N L V Q K Y L E N Y Y D L,
P V V K K I R E M Q K F L G L E V T G,
T D L T R F R L S Q D D I N G I Q S L,
L S Q D D I N G I Q S L Y G P P P D S,
F F Y F F T G S S Q L E F D P N A K K,
E V A P V D Y L S Q Y G Y L Q K P L E,
D Y L S Q Y G Y L Q K P L E G S N N F,
R C G L E D P F N Q K T L K Y L L L G,
D I R L S F H G R Q S S Y C S N T F D,
L G L G H S R Y S Q A L M A P V Y E G,
L H P D D V A G I Q A L Y G K K S P V,
D A A L Y W P L N Q K V F L F K G S G,
G K V Y W R L N Q Q L R V E K G Y P R,
N Y N D F G N Y N Q Q P S N Y G P M K,
L P A H L D E E L Q A T L H D F R H Q,
L Q T R G A L S L Q G S I M T V G E K,
K M A K M I D E R Q Q E L T H Q E H R,
S I A K K I D A A Q N W L A D P N G G,
H L E G K I E Q A Q R W I D N P T V D,
P T V D D R G V G Q A A I R G L V A E,
A N V M M G P Y R Q D L L A K C D R V,
Q A R A L A S Q L Q D S L K D L K A R,
R I L L R N P G N Q A A Y E H F E T M,
K V A M A N I Q P Q M L V A G A T S I,
A G N I S D P G L Q K S F L D S G Y R,
A K V R E A F Q P Q E P D F P P P P P,
F P P P P P D L E Q L R L T D E L A P,
C E R I P T I S T Q L K I L S T V K A,
A T E M L V H N A Q N L M Q S V K E T,
Y F T N W S Q D R Q E P G K F T P E N,
L I H E L A E A F Q K D F T K S T K E,
A K I T R L Q D Q Q V P Y A V K G N Q,
D D F T G K S C N Q G P Y P L V Q A V,
I K E A Q P G K K Q L L L S A A L S A,
A T V H R T L G Q Q V P Y A T K G N Q,
G N Q W V G Y D D Q E S V K S K V Q Y,
V W A L D L D D F Q G S F C G Q D L R,
C G W L L G A E A Q E P G A P A A G M,
A E C F P A C N P Q N G F C E D D N V,
N V C R C Q P G W Q G P L C D Q C V T,
N G G T C L Q H T Q V S Y E C L C K P,
T P G V H E L P V Q Q P E H R I L K V,
A R K A A C A C D Q K P C S C S K V D,
G E R P T L A F L Q D V M N I L L Q Y,
L Q D V M N I L L Q Y V V K S F D R S,
N L E E I L M H C Q T T L K Y A I K T,
L E R R I L E A K Q K G F V P F L V S,
I G S E D G E P P Q Q R V T G T L V L,
V F S A V L G S L Q F G Y N I G V I N,
Y N I G V I N A P Q K V I E Q S Y N E,
L R G A L G T L N Q L A I V I G I L I,
G L T V L P A L L Q L V L L P F C P E,

W F I V A E L F S Q G P R P A A M A V,
T S K G Q K C E F Q D A Y V L L S E K,
L S E K K I S S I Q S I V P A L E I A,
D K A Q I E K R I Q E I I E Q L D V T,
E K R I Q E I I E Q L D V T T S E Y E,
L C S H L E V C I Q D G L F G Q C Q V,
V C I Q D G L F G Q C Q V G V G Q A R,
I Q D G L F G Q C Q V G V G Q A R P L,
V T S P V L Q R L Q G V L R Q L M S Q,
G L S W H D D L T Q Y V I S Q E M E R,
I P T G S A P A A Q H R L P Q P P V G,
G A S S S L S P L Q A E L L P P L L E,
E E Y G Y I V T D Q K P L S L A A G V,
N L S L A D V T Q Q A G L V K S E L E,
G L V K S E L E A Q T G L Q I L Q T G,
E A Q T G L Q I L Q T G V G Q R E E A,
T P S W C E E P A Q A N M D I S T G H,
P R M P A Y I A T Q G P L S H T I A D,
L S H T I A D F W Q M V W E S G C T V,
Q T Q E T R T L T Q F H F L S W P A E,
A A T L E H V R D Q R P G L V R S K D,
R P G L V R S K D Q F E F A L T A V A,
H K C S Y P W D L Q D R Y A Q D K S V,
Q D K S V V N K M Q Q R Y W E T K Q A,
D K S V V N K M Q Q R Y W E T K Q A F,
M Q Q R Y W E T K Q A F I K A T G K K,
D L S K A I V L Y Q K R I C F L S Q E,
T G K A L C F S S Q Q R L A L R N P L,
K Q M E K F R K V Q T Q V R L A K K N,
M E K F R K V Q T Q V R L A K K N F D,
L L S H M L A T Y Q T T L L H F W E K,
Y E F T T L K S L Q D P M K K L V E K,
Q Q E S T D A A V Q E P S Q L I S L E,
L L D Q N M K D L Q A S L Q E P A K A,
N M K D L Q A S L Q E P A K A A S D L,
G A V R T E N N I Q R H F C T S R S I,
P S G R A S T R P Q H Q I Q F D E D M,
G R A S T R P Q H Q I Q F D E D M D S,
D K Y L E D F P K Q G P I R L F M E L,
R E A E D L Q V G Q V E L G G G P G A,
N C S V I E G H L Q I L L M F K T R P,
K N S R R Q G C H Q Y V I H N N K C I,
W S K H N L T T T Q G K L F F H Y N P,
L M R G L K P W T Q Y A I F V K T L V,
P I S V S N S S S Q I I L K W K P P S,
E S E D S Q K H N Q S E Y E D S A G E,
E N N V V H L M W Q E P K E P N G L I,
K I T L L R E L G Q G S F G M V Y E G,
N P G R P P P T L Q E M I Q M A A E I,
A I A A D S E A E Q D S W Y Q A L L Q,
S E A E Q D S W Y Q A L L Q L H N R A,
P G P A F K E V W Q V I L K P K G L G,

H H L N N P P P S Q V G L T R R S R T,
T I T H Q K T P S Q S S V A S I E E Y,
M S P K S V S A P Q Q I I N P I R R H,
S L P R S F K H T Q R P G E P E E G A,
P R A R E Q Q Q Q Q Q P L L H P P E P,
Y V N I E F G S D Q S G Y L S G P V A,
M T M Q M S C P R Q S Y V D T S P A A,
A H S S L L G G P Q G P G G M S A F T,
D L D L V K D F K Q C P Q E C T P E P,
R A S Q D S A D P Q A P A Q G N F R G,
D S A D P Q A P A Q G N F R G S W D C,
G V S E A A S G S Q E K L D F N R N L,
M E A K D V K G T Q E S L A E K E L Q,
Q E S L A E K E L Q L L V M I H Q L S,
D Q L L T A H S E Q K N M A A M L F E,
M A A M L F E K Q Q Q Q M E L A R Q Q,
A M L F E K Q Q Q Q M E L A R Q Q Q E,
Q Q M E L A R Q Q Q E Q I A K Q Q Q Q,
Q Q Q E Q I A K Q Q Q Q L I Q Q Q H K,
Q Q E Q I A K Q Q Q Q L I Q Q Q H K I,
K Q Q Q Q L I Q Q Q H K I N L L Q Q Q,
Q Q Q H K I N L L Q Q Q I Q Q V N M P,
I N L L Q Q Q I Q Q V N M P Y V M I P,
Q P L P V T P D S Q L A L P I Q P I P,
G A M A T H H P L Q E P S Q P L N L T,
Q E K Q P Y Y E E Q A R L S R Q H L E,
M R T R R Q D A R Q S Y V I P P Q A G,
S Y V I P P Q A G Q V Q M S S S D V L,
S T S A F R A Y G Q G T L Y D S P L L,
G T L Y D S P L L Q V S I H L G Y G I,
I T R I A L Y F V Q K G L A V P C C F,
L S L S V L V S L Q G P L F L S Y L G,
S S H Q H S R R R Q G W L K E I R K L,
R G V D F N W Q A Q A L L A L Q E A A,
P S V A E G Y A S Q D V F S A T E T S,
G K S A K P R A G Q A G L P C D Y T A,
L D T S G L R H V Q L A F F P P G T V,
V Q Q V K G H Y R Q A M L L K A M A A,
L K A M A A L E G Q D P S G L Q L G L,
L E G Q D P S G L Q L G L T E A L H F,
E G L E A E D W A Q G V V E A G G S F,
G A Y G A Q E E A Q C P T L H F L E G,
T S F P I D D R V Q S H I L H L E H D,
H V T R K N H A R Q A G V R G L G H Q,
N F A L R V L L V Q V D V K D P Q Q A,
L V Q V D V K D P Q Q A L K E L A K M,
Y L E T Y K A Y E Q K P A D L L M E K,
A D L L M E K L E Q D F V S R V T E C,
V K S V N K T D S Q T L L T T F G S L,
I K R L R K K F A Q K M L R K A R R K,
S F E P F S N K G Q T L V V Q F T V K,
S N K G Q T L V V Q F T V K H E Q N I,

V K L F P N S L D Q T D M H G D S E Y,
G I P A G V P M P Q A P A G L A G P V,
P V R G V G G P S Q Q V M T P Q G R G,
G V P A G V P I P Q A P A G L A G P V,
P V R G V G G P S Q Q V M T P Q G R G,
G I P A G V P M P Q A P A G L A G P V,
P V R G V G G P S Q Q V M T P Q G R G,
V L I M C E A C S Q S P E H E A H S V,
W K I Q V E T R K Q S I V W E F E K Y,
S I V W E F E K Y Q R L L E K K Q P P,
E L N H S E L I Q Q S Q V L W R M I A,
N H S E L I Q Q S Q V L W R M I A E L,
M I A E L K E R S Q R P V R W M L Q D,
P V R W M L Q D I Q E V L N R S K S W,
L N R S K S W S L Q Q P E P I S L E L,
T L H F E G R N Y Q A S V D S L T F S,
V Y L D S E E E R Q E Y V L T Q Q G F,
E E R Q E Y V L T Q Q G F I Y Q G S A,
E R Q E Y V L T Q Q G F I Y Q G S A K,
N I P W N F G Q F Q D G I L D I C L I,
S G M V N C N D D Q G V L L G R W D N,
H G C Q R V K Y G Q C W V F A A V A C,
E S W M T R P D L Q P G Y E G W Q A L,
R K L V A E V S L Q N P L P V A L E G, and
V E G A G L T E E Q K T V E I P D P V.

4. Peptide sequence of 8 - 40 amino acid residues which comprises at least one 8-mer which is a subsequence of at least one of the sequences listed in claim 3, wherein said 8-mer comprises the central Q residue of the sequence listed in claim 3.

5. Epitope formed by deamidation of one or more of the Q residues of the peptide according to claim 3 or 4.

6. An isolated HLA-DQ restricted T-cell capable of recognising an epitope according to claim 2.

7. An isolated or recombinant HLA-DQ restricted T-cell receptor capable of recognising an epitope according to claim 2.

8. Antibody reactive with a peptide or an epitope according to claim 1 or 2.

9. Method to screen foodstuffs or plant material for the occurrence of peptides according to claim 1 or epitopes according to claim 2 using a T cell according to claim 6, a T cell receptor according to claim 7 or an antibody according to claim 8.

10. Method according to claim 9, wherein the foodstuff or plant material is pretreated with a protease.

11. Method according to claim 10, wherein the protease is trypsin, chymotrypsin or pepsin.

12. Method according to any of claims 10 or 11, wherein the foodstuff or plant material is additionally treated with tTG.

13. A diagnostic kit comprising:
- a T cell according to claim 6 or a T cell receptor according to claim 7 or an antibody according to claim 8; and
- a suitable means of detection.

14. A diagnostic kit according to claim 13 for detecting in food, food components, plant material or biological samples the presence of a peptide or epitope according to claim 1 or 2.

15. An oligonucleotide primer set suitable for use in an amplification assay which is capable of binding to a nucleotide sequence coding for a peptide according to claim 1, an epitope according to claim 2 or a DNA sequence which codes for a peptide which matches a consensus sequence according to the following formula (1), (2), (3), (4) or (5):
(1) X₁ - X₂ - X₃ - Q₄ - X₅ - P₆ - Q₇ - X₈ - P₉ - X₁₀ in which X₁₀ is F, Y, M, W, I or L and X₁, X₂, X₃, X₅, and X₈ are any amino acid;
(2) X₁ - X₂ - X₃ - Q₄ - X₅ - P₆ - Q₇ - X₈ - X₉ - X₁₀ in which X₉ is F, Y, M, W, I or L and X₁, X₂, X₃, X₅, X₈ and X₁₀ are any amino acid;
(3) Q - Xi - P in which X₁ is any amino acid;
(4) Q - Xi - P - X₂ in which X₁ is any amino acid and X₂ is F,Y,W,M,L,I, or V;
(5) Q - X₁ - X₂ - X₃ in which X₁ and X₂ are any amino acid and X₃ is F,Y,W,M,L,I, or V.

16. A nucleotide amplification assay wherein food, food components, plant material or biological samples are tested for the presence of proteins or peptides which match any of the consensus sequences: (1) or (2) as defined in claim 15.

17. Method for the detection of proteins or peptides which match any of the consensus sequences: (1) or (2) as defined in claim 15 in a nucleotide amplification assay in which the primer set of claim 15 is used.

18. Method for the detection of cereals which are better suited to be used in food for celiac disease patients by using a method according to claim 9, 10, 11, 12, 16 or 17.

19. Method for producing cereals which are better suited to be used in food for celiac disease patients by selecting cereals having a low amount of proteins or peptides matching the consensus sequences as defined in claim 15 and crossbreeding them to produce cereals with even lower amounts.

20. Method for producing cereals which are better suited to be used in food for celiac disease patients by using genetic engineering techniques to diminish the natural amount of proteins or peptides matching the consensus sequences as defined in claim 15.

21. Method according to claim 20, wherein the genetic engineering comprises transformation with recombinant DNA or induced mutation.

22. Method according to claim 21, wherein the transformation with recombinant DNA comprises homologous recombination or gene silencing.

23. Method according to any of claims 19-22 wherein the cereal is wheat.

24. Cereal plant obtained by any of the methods 19-23.

25. Use of a cereal according to claim 24 to prepare food for celiac disease patients.

26. A method to identify neo-epitopes from auto-antigens comprising:
- comparing the sequence of an auto-antigen and searching for peptide sequences which match with the consensus sequences as defined in claim 15;
- screen biological material obtained from auto-immune disease patients for antibodies which bind to the peptide sequences or T cells which bind to the HLA-bound peptide sequences as identified in the previous step or peptides which are formed from said peptide sequences by deamidation of one or more Q residues.

27. A method to inhibit the binding of a T cell receptor to an HLA-bound epitope according to claim 2 or to an HLA-bound epitope according to claim 5 comprising providing a blocking substance.

28. A method to inhibit the binding of the epitopes of claim 2 to HLA-DQ molecules comprising providing substances that block the binding of said epitopes to said HLA-DQ molecules.

29. A method to inhibit the binding of the epitopes of claim 5 to HLA molecules comprising providing substances that block the binding of said epitopes to said HLA molecules.

30. Analogue of an epitope according to claim 2 or claim 5, **characterised in that** said analogue is an antagonist for the activity of T cells recognising said epitope.

31. A pharmaceutical composition comprising a peptide according to claims 1 or 4 or an epitope according to claims 2 or 5.

32. A pharmaceutical composition according to claim 31 for the induction of tolerance.

33. A pharmaceutical composition according to claim 31 for the treament of celiac disease or auto-immune disease.

34. A method to decrease sensitivity to auto-antigens by inhibiting the deamidation of said auto-antigen.

35. Method according to claim 34 wherein the inhibition is by inhibiting the function of tTG.

36. Method according to claim 35, wherein the inhibition takes place at the site of presence of the auto-antigen.

37. Use of a protease inhibitor for preventing the generation of a peptide according to claim 1 or claim 4 or a polypeptide comprising a peptide according to claim 1 or claim 4.

38. Use of acid neutralising substances for preventing the generation of a peptide according to claim 1 or claim 4 or a polypeptide comprising a peptide according to claim 1 or claim 4.

39. Method to reduce the production of a peptide according to claim 3, 4 or 5 by inhibiting the expression of the auto-antigen from which it is derived.
